# EUROPEAN PATENT SPECIFICATION

(11) **EP 3 337 786 B1**
(45) Date of publication and mention of the grant of the patent: **11.12.2019**
(21) Application number: 16753399.1
(22) Date of filing: 18.08.2016
(51) Int. Cl.: C07D 215/233, C07B 53/00, C07C 233/00

(54) **ASYMMETRIC BISAMIDATION OF MALONIC ESTER DERIVATIVES**
ASYMMETRISCHE BISAMIDIERUNG VON MALONESTERDERIVATEN
BISAMIDATION ASYMÉTRIQUE DE DÉRIVÉS D'ESTER MALONIQUE

(30) Priority: 19.08.2015 EP 15002462
(43) Date of publication of application: 27.06.2018
(73) Proprietor: Sandoz AG, 4056 Basel (CH)
(72) Inventor: SCHREINER, Erwin, 6250 Kundl (AT); NERDINGER, Sven, 6250 Kundl (AT); LAUS, Gerhard, 6020 Innsbruck (AT)
(74) Representative: Altmann Stößel Dick Patentanwälte PartG mbB
(86) International application number: PCT/EP2016/069621
(87) International publication number: WO 2017/029362

(56) References cited:
- WO-A1-2010/083414
- WO-A2-2005/030140
- US-A- 5 334 747
- MANSON CHATTAWAY: "Halogen Derivatives of Malonanilide, Ethyl Malonanilate, and Malonanilic Acid", JOURNAL OF THE CHEMICAL SOCIETY, CHEMICAL SOCIETY, LETCHWORTH; GB, vol. 97, no. Part 1, 1 January 1910 (1910-01-01), pages 339-345, XP008157203, ISSN: 0368-1769 cited in the application
- FUMITERU TERAOKA ET AL: "Asymmetric Carbonyl Migration of [alpha]-Amino Acid Derivatives via Memory of Chirality", SYNLETT, vol. 2011, no. 04, 1 March 2011 (2011-03-01), pages 543-546, XP055235295, DE ISSN: 0936-5214, DOI: 10.1055/s-0030-1259324

## Description

### FIELD OF THE INVENTION

The present invention relates to processes, process steps and intermediates useful in the asymmetric bisamidation of malonic ester derivatives. The new processes, process steps and intermediates are, for example, useful in the preparation of asymmetric malonic acid bisanilides, such as cabozantinib.

### BACKGROUND OF THE INVENTION

Asymmetric bisanilides of disubstituted malonic acids are a structural motif present in a novel class of tyrosine kinase inhibitors in clinical investigation such as altiratinib, cabozantinib, foretinib, golvatinib, and sitravatinib (MG-516). One of these compounds, N-{4-[(6,7-dimethoxy-4-quinolinyl)oxy]phenyl}-N'-(4-fluorophenyl)-1,1-cyclopropanedicarboxamide (international non-proprietary name (INN): cabozantinib) represented by chemical structure 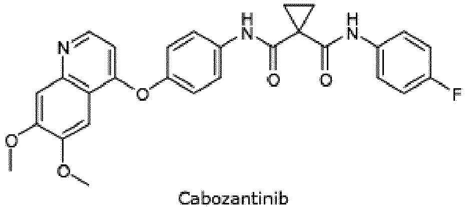 is a RET, MET, KIT and VEGFR2 receptor tyrosine kinase inhibitor. It is currently marked for the treatment of thyroid cancer under the brand name Cometriq. Cabozantinib is classified as a BCS class II compound having a low solubility and high permeability.

The above mentioned compound will be referred to in the present application by its international non-proprietary name, i.e. cabozantinib.

International patent application WO 2005/030140 discloses a multi-step process for the preparation of cabozantinib and analogues. Starting from 1,1-cyclopropanedicarboxylic acid, which is obtained from the corresponding ester by saponification, the monosubstituted fluorophenylcarbamoylcyclopropanecarboxylic acid was prepared via an acid chloride intermediate in 65 % yield. In the next step the second amide bond is formed by condensation with 4-benzyloxyaniline in the presence of EDCI in dichloromethane (94%). Next, palladium-catalyzed hydrogenolytic removal of the benzyl group provides a phenol intermediate (94 %). Finally, cabozantinib is obtained by forming the ether bond by means of treatment with trifluoromethanesulfonic acid 6,7-dimethoxy-quinolin-4-yl ester in 2,6-lutidine at 165 °C in 88 % yield.

WO 2010/083414 discloses another multi-step process for the preparation of cabozantinib wherein the monosubstituted fluorophenylcarbamoylcyclopropanecarboxylic acid, prepared similarly as described in WO 2005/030140 in a three step sequence from a 1,1-cyclopropanedicarboxylic acid ester, is transformed into the corresponding acid chloride by treatment with oxalyl chloride and subsequently with 4-((6,7-dimethoxyquinolin-4-yl)oxy)aniline.

Both processes suffer from involving multiple steps and corrosive acid chlorides as intermediates. Furthermore, the use of thionyl chloride as a corrosive reagent negatively affects process handling and safety.

F. D. Chattaway et al. (J.Chem. Soc. 97, 339 1910) discloses conversion of unsubstituted malonic acid diethyl ester with various halogenated anilines into mono- and bisanilides under reflux conditions (e.g. 199 °C for DEM). Low or no yields are reported.

Furthermore, the preparation of malonic monoester monoanilides and symmetric bisanilides, by reaction of substituted anilines with diethyl malonate in the presence of sodium methanolate in a suitable organic solvent has been disclosed in US 5,334,747.

Finally, the preparation of symmetric bisanilides is described by Sechi et al. (Molecules 2008, 13, 2442-2461), wherein dimethyl malonate or diethyl malonate have been reacted with aromatic amines at a temperature 185 °C.

However, no process for the formation of asymmetric bisanilides by direct conversion of the ester functionalities into the amides without saponification and subsequent activation of the acid has been disclosed so far.

Consequently, it would thus be beneficial to develop a short, scalable process starting from a dialkyl malonic acid ester for the production of asymmetric malonic acid bisanilides, such as cabozantinib, altiratinib, cabozantinib, foretinib, golvatinib, and sitravatinib (MG-516) or derivatives thereof. The envisaged process should not suffer from some or all of aformentioned drawbacks.

### SUMMARY OF THE INVENTION

The present invention relates to a two-step process starting from a 1,1-disubstituted dicarboxylic acid ester, preferably a 1,1-cyclopropanedicarboxylic acid ester, wherein both amide bonds are formed by sequential reaction with the alkali metal salts or alkaline earth metal salts of the desired amines, preferably anilines.

Optionally, the intermediate monoamide can be purified by separation of the precipitated alkali metal salt or alkaline earth metal salt or crystallization of the free form. Another option is to react the 1,1-cyclopropanedicarboxylic acid ester sequentially with both amines as their alkali metal salts or alkaline earth metal salts without isolation of the intermediate in a one-pot reaction.

The process of the invention provides pure asymmetric bisamide without the need for chromatographic purification in good to excellent yield.

### (1)

According to the inventive concept, it is preferred that in a first step, a 1,1-disubstituted dicarboxylic acid ester is reacted with the alkali metal salt or alkaline earth metal salt of a first amine and to convert, in a second step, the alkali metal salt or alkaline earth metal salt respectively obtained to the asymmetric bisamide.

Therefore, the present invention relates to a process for preparing an asymmetric malonic acid diamide of formula (IV) 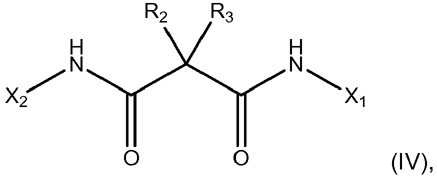 or a pharmaceutically acceptable salt thereof, the process comprising
(i) reacting a compound of formula (I) 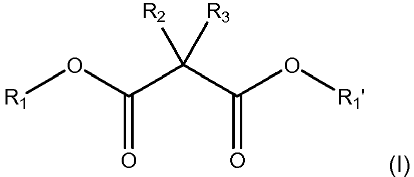 with an Mₐ salt of a compound H₂N-X₁, obtaining a reaction mixture comprising an Mₐ salt of formula (II) 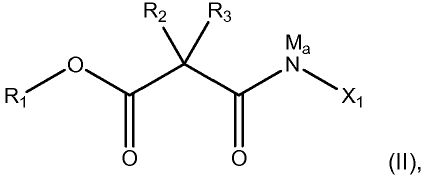 or an Mₐ salt of formula (II') 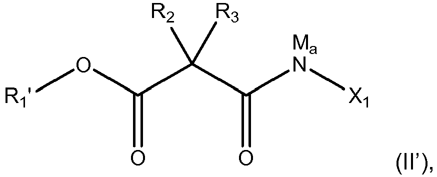 or a mixture of the Mₐ salt of formula (II) and the Mₐ salt of formula (II');
(ii) converting the Mₐ salt of formula (II) and/or of formula (II') to the compound of formula (IV) comprising forming an amide bond between the carbon atom of the ester moiety comprised in the compound of formula (II) and/or formula (II'), preferably of formula (II), and the nitrogen atom comprised in a compound H₂N-X₂;
   wherein
   R₁ and R₁' are alkyl or aryl, and R₁ and R₁' are the same or different;
   R₂ and R₃ are alkyl, alkoxy, aryl or heteroaryl and R₂ and R₃ are the same or different, or R₂ and R₃, together with the C atom at which R₂ and R₃ are attached, form a cycle;
   X₁ is a substituted aromatic or heteroaromatic residue;
   X₂ is a substituted or unsubstituted alkyl, alkenyl, cycloalkyl, aromatic or heteroaromatic residue wherein X₁ is different from X₂;
   Mₐ is an alkali or alkaline earth metal.

With regard to the residue R₁, no specific limitations exist provided that the process of the present invention can be carried out. For example, R₁ may be an optionally substituted alkyl residue or an optionally substituted aryl residue. Preferably, R₁ is C1-C6 alkyl, i.e. an alkyl residue having 1, or 2, or 3, or 4, or 5, or 6 carbon atoms. More preferably, R₁ is C1-C3 alkyl, more preferably C1 or C2 alkyl. With regard to the residue R₁', no specific limitations exist provided that the process of the present invention can be carried out. For example, R₁' may be an optionally substituted alkyl residue or an optionally substituted aryl residue. Preferably, R₁' is C1-C6 alkyl, i.e. an alkyl residue having 1, or 2, or 3, or 4, or 5, or 6 carbon atoms. More preferably, R₁' is C1-C3 alkyl, more preferably C1 or C2 alkyl. Preferably, R₁ and R₁' are the same. More preferably, both R₁ and R₁' are methyl.

With regard to the residues R₂ and R₃, no specific restrictions exist provided that the process of the present invention can be carried out. For example, R₂ and R₃, independently from each other, may be optionally suitably substituted alkyl, optionally suitably substituted alkoxy, optionally suitably substituted alkylaryl, optionally suitably substituted arylalkyl, optionally suitably substituted aryl, or optionally suitably substituted heteroaryl residues. Preferably, R₂ is C1-C6 alkyl, i.e. alkyl having 1 or 2 or 3 or 4 or 5 or 6 carbon atoms, C1-C6 alkoxy, i.e. alkoxy having 1 or 2 or 3 or 4 or 5 or 6 carbon atoms, aryl or heteroaryl having from 5 to 10 atoms, i.e. 5 or 6 or 7 or 8 or 9 or 10 atoms constituting the aryl or heteroaryl structure. Preferably, R₃ is C1-C6 alkyl, i.e. alkyl having 1 or 2 or 3 or 4 or 5 or 6 carbon atoms, C1-C6 alkoxy, i.e. alkoxy having 1 or 2 or 3 or 4 or 5 or 6 carbon atoms, aryl or heteroaryl having from 5 to 10 atoms, i.e. 5 or 6 or 7 or 8 or 9 or 10 atoms constituting the aryl or heteroaryl structure. Also preferably, R₂ and R₃, together with the C atom at which R₂ and R₃ are attached, form a cycle having from 3 to 6, i.e. 3 or 4 or 5 or 6 atoms, preferably from 3 to 5, i.e. 3 or 4 or 5 atoms, more preferably 3 or 4 atoms. Therefore, the compound of formula (I) is preferably 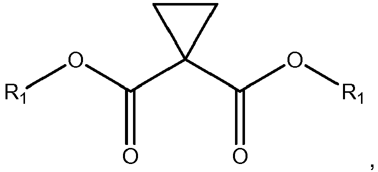 more preferably

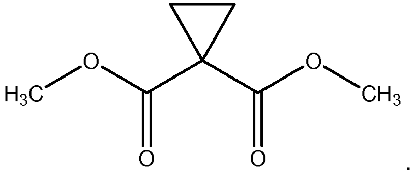

With regard to the Mₐ salt of the compound H₂N-X₁, it is preferred that Mₐ comprises, preferably is, an alkali metal, an alkaline earth metal, or a combination of two or more therefore. Preferably, Mₐ comprises an alkali metal. More preferably, Mₐ is an alkali metal. While it is generally conceivable that the Mₐ salt of the compound H₂N-X₁ is (Mₐ)₂N-X₁., it is preferred that the Mₐ salt of the compound H₂N-X₁ is MₐHN-X₁. Preferably, Mₐ comprises sodium or potassium. More preferably, Mₐ is sodium or potassium. More preferably, Mₐ comprises sodium. More preferably, Mₐ is sodium. More preferably, the Mₐ salt of the compound H₂N-X₁ is MₐHN-X₁.

As mentioned above, X₁ is a substituted aromatic or substituted heteroaromatic residue preferably having from 5 to 10 atoms, i.e. 5 or 6 or 7 or 8 or 9 or 10 atoms constituting the aromatic or heteroaromatic structure. If X1 is a heteroaromatic residue, it may contain one or more heteroatoms wherein the heteroatoms may include N, O, or S. With regard to the substitution pattern of X₁, no specific limitations exist provided that the process of the present invention can be carried out. For example, X₁ may have 1 or 2 or 3 or 4 or 5 or more substituents which may be the same or different. Preferably, X₁ is substituted with one or more of halogen, preferably one or more of F and Cl, and Br, more preferably one or more of F and CI; alkyl, preferably C1-C6 alkyl, i.e. alkyl having 1 or 2 or 3 or 4 or 5 or 6 carbon atoms; alkoxy, preferably C1-C6 alkoxy, i.e. alkoxy having 1 or 2 or 3 or 4 or 5 or 6 carbon atoms; aryloxy; and heteroaryloxy. Preferably, X₁ is monosubstituted. More preferably, X₁ is a monosubstituted phenyl residue, i.e. a para- or meta- or ortho-monosubstituted phenyl residue, more preferably a para- or ortho-monosubstituted phenyl residue. More preferably, X₁ is a monosubstituted phenyl residue, i.e. a para- or meta- or ortho-monosubstituted phenyl residue, more preferably a para- or ortho-monosubstituted phenyl residue, more preferably a para-monosubstituted phenyl residue wherein the substituent comprises, preferably is, F or CI, more preferably F. Therefore, it is preferred that the compound H₂N-X₁ is 4-haloaniline, preferably 4-fluoroaniline.

Therefore, according to a preferred embodiment of the present invention, the compound of formula (I) is 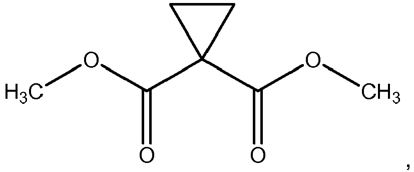 the compound H₂N-X₁ is 4-fluoroaniline and the Mₐ salt of formula (II) is 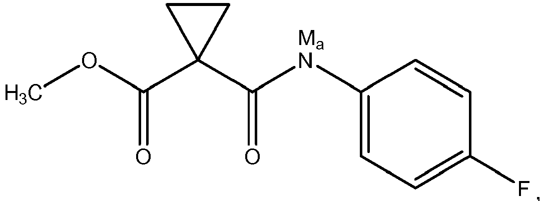 preferably

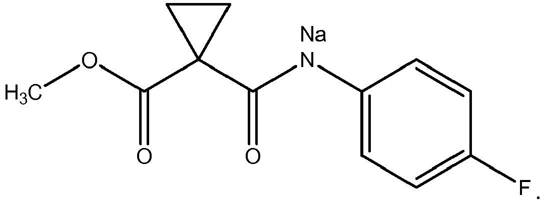

Thus, the present invention also relates to a compound of formula (II) 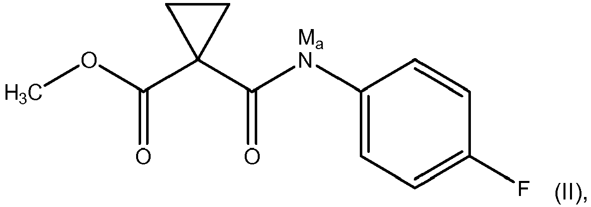 wherein Mₐ is as defined above, preferably magnesium, sodium or potassium, more preferably sodium. Further, the present invention relates to the use of this compound for the preparation, preferably for a two-pot process preparation, of an asymmetric malonic acid diamide of formula (IV) 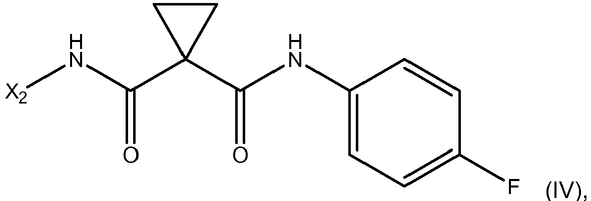 wherein X₂ is as defined above. Preferably X₂ is as defined in embodiments 60 to 75.

Generally, it may be possible to carry out the reaction according to (i) in the absence of a solvent. Preferably, however, (i) comprises reacting the compound of formula (I) with the Mₐ salt of a compound H₂N-X₁ in a reaction solvent. With regard to the chemical nature of the reaction solvent, no specific restrictions exist provided that the reacting according to (i) can be carried out. However, solvents and solvent systems are preferably to be chosen for (i) such that the Mₐ salt of the compound H₂N-X₁ is not quickly protonated.

Using a mixture of two or more suitable solvents is conceivable. Preferably, no or only low levels of protic solvents, such that the reacting according to (i) can be carried out, are present when the compound of formula (I) is reacted with the Mₐ salt of a compound H₂N-X₁. Preferably, the reaction solvent comprises, and preferably consists of, one or more aprotic solvents, more preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran. More preferably, the reaction solvent comprises toluene. More preferably, the reaction solvent is toluene.

Preferably, with regard to (i), the compound of formula (I) is provided in a solvent for the compound of formula (I). With regard to the chemical nature of the solvent for the compound of formula (I), no specific restrictions exist provided that the reacting according to (i) can be carried out. However, solvents and solvent systems are preferably to be chosen for (i) such that the Mₐ salt of the compound H₂N-X₁ is not quickly protonated. Using a mixture of two or more suitable solvents is conceivable. Preferably, no or only low levels of protic solvents, such that the reacting according to (i) can be carried out, are present when the compound of formula (I) is reacted with the Mₐ salt of a compound H₂N-X₁. Preferably, the solvent for the compound of formula (I) comprises one or more aprotic solvents, more preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran. More preferably, the solvent for the compound of formula (I) comprises toluene. More preferably, the solvent for the compound of formula (I) is toluene. Preferably, the solvent for the compound of formula (I) is the reaction solvent as defined above.

According to (i), the compound of formula (I) is reacted with an Mₐ salt of a compound H₂N-X₁. The Mₐ salt of a compound H₂N-X₁ can be provided or prepared according to all conceivable methods. The Mₐ salt of a compound H₂N-X₁ can be provided in solid form and added to the reaction with the compound of formula (I). Alternatively, the Mₐ salt of a compound H₂N-X₁ can prepared by reacting the compound H₂N-X₁ with a suitable alcoholate MₐORₐ and by removing the alcohol formed by the reaction of the alcoholate with the amine, thus driving equilibrium to the direction of the Mₐ salt of a compound H₂N-X₁. Preferably, Rₐ is an optionally suitably substituted alkyl residue, more preferably an alkyl residue. More preferably, Rₐ is C1-C6 alkyl, more preferably C1-C5 alkyl, more preferably C1-C4 alkyl, more preferably methyl, ethyl, or tert-butyl, more preferably methyl or ethyl, more preferably methyl. Therefore, it is preferred that the residue Rₐ is R₁ or R₁', preferably R₁. In particular, it is preferred that the compound of formula (I) is 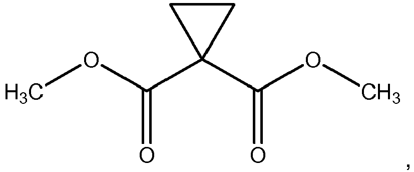 the compound H₂N-X₁ is 4-fluoroaniline and the Mₐ salt of formula (II) is 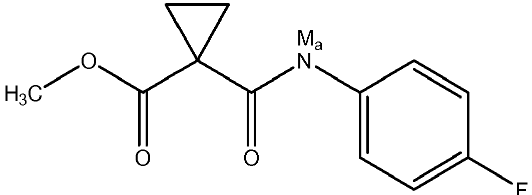 preferably 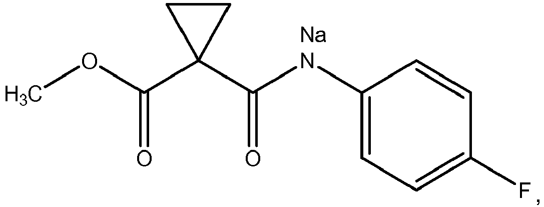 and the compound MₐORₐ is NaOCH₃.

In this regard, it is preferred that the compound H₂N-X₁ is provided in a suitable solvent for the compound H₂N-X₁. With regard to the chemical nature of the solvent for the compound H₂N-X₁, no specific restrictions exist provided that the reacting according to (i) can be carried out. However, solvents and solvent systems are preferably to be chosen for (i) such that the resulting Mₐ salt of the compound H₂N-X₁ is not quickly protonated. Using a mixture of two or more suitable solvents is conceivable. Preferably, no or only low levels of protic solvents, such that the reacting according to (i) can be carried out, are present when the compound of formula (I) is reacted with the Mₐ salt of a compound H₂N-X₁. Preferably, the solvent for the compound H₂N-X₁ comprises one or more aprotic solvents, more preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran. More preferably, the solvent for the compound H₂N-X₁ comprises toluene. More preferably, the solvent for the compound H₂N-X₁ is toluene. Preferably, the solvent for the compound H₂N-X₁ is the reaction solvent as defined above. Therefore, it is preferred that solvent for the compound H₂N-X₁ is the solvent for the compound of formula (I), and this solvent is the reaction solvent.

For reacting the compound H₂N-X₁ with a suitable alcoholate MₐORₐ, it is preferred that the compound MₐORₐ is provided in a suitable solvent. With regard to the chemical nature of this solvent, no specific restrictions exist provided that the reacting can be carried out. Preferably, the solvent for the compound MₐORₐ comprises, more preferably is, an alcohol HORₐ, wherein Rₐ is as defined above. More preferably, HORₐ is methanol.

According to the present invention, it is preferred that when reacting the compound H₂N-X₁ with the compound MₐORₐ, thus obtaining the Mₐ salt of the compound H₂N-X₁, the solvent for the compound H₂N-X₁ as described above and a compound HORₐ which is formed from reacting the compound H₂N-X₁ with the compound MₐORₐ together form an azeotropic mixture such that the alcohol formed by the reaction of the alcoholate with the amine can be removed, thus driving equilibrium to the direction of the Mₐ salt of a compound H₂N-X_{1.}. If the compound MₐORₐ is provided, as described above, in a suitable solvent, and if this suitable solvent is HORₐ, the compound HORₐ which is formed from reacting the compound H₂N-X₁ with the compound MₐORₐ is identical with the suitable solvent is HORₐ. Preferably, the reacting of the compound H₂N-X₁ with the compound MₐORₐ, is carried out at a temperature which allows for distilling off this azeotropic mixture. If, for example, HORₐ is HOCH₃ and the solvent for the compound H₂N-X₁ is toluene, the temperature allowing for distilling off the azeotropic mixture comprising, preferably essentially consisting of methanol and toluene is preferably at least 65 °C, more preferably at least 70 °C, more preferably at least 80 °C, more preferably in the range of from 80 to 100 °C, more preferably in the range of from 90 to 100 °C.

When reacting the compound of formula (I) with the Mₐ salt of a compound H₂N-X₁ according to (i), it is preferred that the reaction solvent described above forms an azeotropic mixture with the compound HORₐ. Preferably, the reacting of the compound of formula (I) with the Mₐ salt of a compound H₂N-X₁ in the presence of the reaction solvent is carried out at a temperature which allows for distilling off this azeotropic mixture. Further, when reacting the compound of formula (I) with the Mₐ salt of a compound H₂N-X₁ according to (i), it is preferred that the reaction solvent described above forms an azeotropic mixture with the compound HOR₁ or HOR₁, , preferably HOR₁. Preferably, the reacting of the compound of formula (I) with the Mₐ salt of a compound H₂N-X₁ in the presence of the reaction solvent is carried out at a temperature which allows for distilling off this azeotropic mixture. If, for example, HORₐ is HOCH₃, HOR₁ or HOR₁', preferably HOR₁ is HOCH₃, and the solvent for the compound H₂N-X₁ is toluene and, the temperature allowing for distilling off the azeotropic mixture comprising, preferably essentially consisting of methanol and toluene is preferably at least 65 °C, more preferably at least 70 °C, more preferably at least 80 °C, more preferably in the range of from 80 to 100 °C, more preferably in the range of from 90 to 100 °C. By removing the alcohol formed by the reaction of the alcoholate with the amine by way of azeotropic distillation, the equilibrium of the alcoholate / amin system will be driven to the direction of the Mₐ salt of a compound H₂N-X₁, which can then react with an ester functionality of the compound of formula (I).

Generally, no specific restrictions exist with regard to the amounts of the compounds to be reacted with each other according to (i). Preferably, prior to the reacting according to (i), the molar ratio of the compound of formula (I) provided according to (i) relative to the compound H₂N-X₁ provided according to (i) is in the range of from 2:1 to 1:1, preferably in the range of from 1.5:1 to 1:1, more preferably in the range of from 1.1:1 to 1:1. Preferably, prior to reacting according to (i), the molar ratio of the compound MₐORₐ provided according to (i) relative to the compound H₂N-X₁ provided according to (i) is in the range of from 2:1 to 1:1, preferably in the range of from 1.5:1 to 1:1, more preferably in the range of from 1.1:1 to 1:1.

According to a very preferred aspect of the present invention, the Mₐ salt of formula (II) and/or of formula (II'), preferably the Mₐ salt of formula (II), which is obtained from (i), is obtained as a solid compound which can be separated from the respective reaction mixture.

The composition which is obtained directly from (i), i.e. directly from reacting the compound of formula (I) with the Mₐ salt of a compound H₂N-X₁, is a composition comprising an Mₐ salt of formula (II) 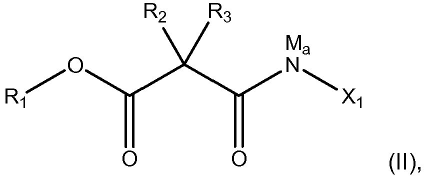 or an Mₐ salt of formula (II') 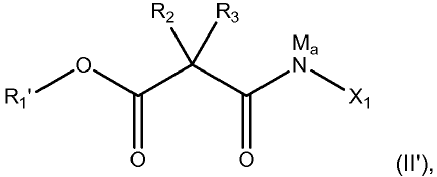 or a mixture of the Mₐ salt of formula (II) and the Mₐ salt of formula (II'), with a degree of purity in the range of from 75:25 to 95:5, preferably from 85:15 to 95:1, more preferably from 92:8 to 95.5, more preferably from 93:7 to 95:5, wherein the degree of purity is defined as the molar amount of the Mₐ salt of formula (II) or of the Mₐ salt of formula (II') or of the mixture of the Mₐ salt of formula (II) and the Mₐ salt of formula (II'), comprised in the composition, relative to the molar amount of an Mₐ salt of the symmetric malonic acid diamide of formula (S) 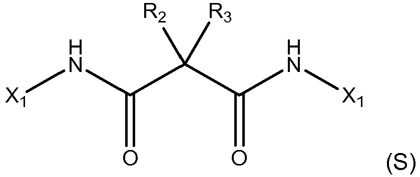 comprised in the composition.

According to (ii), the Mₐ salt of formula (II) and/or of formula (II') is converted to the compound of formula (IV) comprising forming an amide bond between the carbon atom of the ester moiety comprised in the compound of formula (II) and/or formula (II'), preferably of formula (II), and the nitrogen atom comprised in a compound H₂N-X₁.

### (a)

According to a first preferred embodiment of the present invention, said converting according to (ii) comprises
(ii.a.1) converting the Mₐ salt of formula (II) to a compound of formula (III) 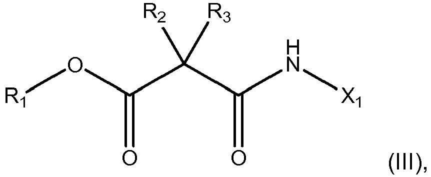 or the Mₐ salt of formula (II') to a compound of formula (III') 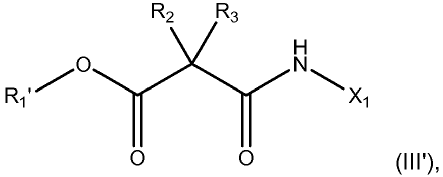 or the mixture of the Mₐ salt of formula (II) and the Mₐ salt of formula (II') to a mixture of the compounds of formula (III) and formula (III').

Generally, no specific restrictions exist how step (ii.a.1) is carried out. Preferably, the amide nitrogen is suitably protonated, and the converting according to (ii.a.1) comprises
(ii.a.1.1) protonating the amide nitrogen in the compound of formula (II) and/or formula (II'), preferably of formula (II), preferably by bringing the compound of formula (II) and/or formula (II'), preferably of formula (II), in contact with an acid.

The acid used according to (ii.a.1.1) is not subject to any specific restrictions and can be one or more inorganic acids, one or more organic acids, or a mixture of one or more inorganic acids and one or more organic acids. Preferably, the acid comprises, preferably is, one or more of hydrochloric acid, sulphuric acid, formic acid, and acetic acid, preferably hydrochloric acid. Said protonating is preferably carried out at a temperature in the range of from -10 °C to 50 °C, preferably from 0 to 40 °C, more preferably from 10 to 30 °C wherein prior to said protonating, the molar ratio of the one or more acids, relative to the compound of formula (II) and/or formula (II'), preferably of formula (II), is preferably in the range of from 1.0:1 to 1.5:1, more preferably of from 1.0:1 to 1.1:1.

According to an especially preferred embodiment of the present invention, said protonating is carried out comparatively fast, i.e. the total amount of acid is brought into contact with the total amount of the compound of formula (II) and/or formula (II'), preferably of formula (II), in a very short period of time. Without wanting to be bound by any theory, it is believed that this fast protonating leads to the effect that the time during which the compound of formula (II) and/or formula (II'), preferably of formula (II), is exposed to an aqueous, basic system is minimized and thus saponification of the ester (II) and/or (II') is avoided. Therefore, it is preferred that the amount of the acid, brought into contact with 1 mol of the compound of formula (II) and/or formula (II'), preferably of formula (II), is in the range of from 0.01 to 0.2 mol per second, preferably of from 0.05 to 0.1 mol per second.

Preferably, the acid used in this step is comprised in an aqueous system wherein, further preferably, at least 5 weight-%, more preferably at least 50 weight-%, more preferably at least 90 weight-%, more preferably at least 99 weight-% of the aqueous system consist of water, wherein more preferably, the aqueous system is water. Further preferably, the obtained compound of formula (III) and/or of formula (III'), preferably of formula (III), is not dissolved in the aqueous system. According to this preferred embodiment according to which the reaction mixture comprises an aqueous system, it is preferred that the process further comprises
(ii.a.1.2) separating the obtained compound of formula (III) and/or of formula (III'), preferably of formula (III), from the aqueous system.

Generally, the compound of formula (III) and/or of formula (III'), preferably of formula (III), can be separated from the aqueous system according to any conceivable method. Preferably, the separating comprises extracting the compound of formula (III) and/or of formula (III'), preferably of formula (III), preferably with an extracting agent including one or more of dichloromethane, isopentane, cyclohexane, heptane, and toluene, more preferably one or more of dichloromethane and isopentane, wherein, as extracting agent, isopentane is especially preferred. In a subsequent step, it is possible to separate, preferably by evaporating, the extracting agent from the compound of formula (III) and/or of formula (III'), preferably of formula (III), to obtain the compound of formula (III) and/or of formula (III'), preferably of formula (III), as a solid, preferably as a crystalline solid.

Therefore, it is disclosed a composition comprising a compound of formula (III) 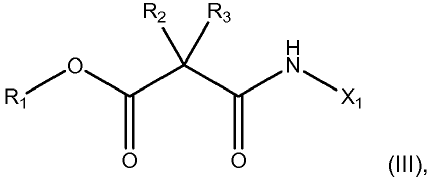 or a compound of formula (III') 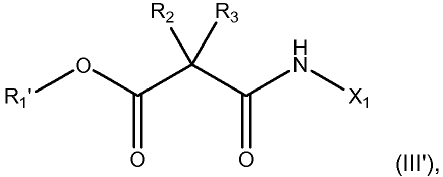 or a mixture of the compounds of formula (III) and formula (III'), wherein the compound of formula (III) or the compound of formula (III') or the mixture of the compounds of formula (III) and formula (III') is dissolved in a solvent system, said solvent system for example comprising, for example consisting of, one or more of dichloromethane, isopentane, cyclohexane, heptane, and toluene, for example one or more of dichloromethane and isopentane, for example isopentane.

In particular with regard to, but not necessarily restricted to, the compound of formula (III) 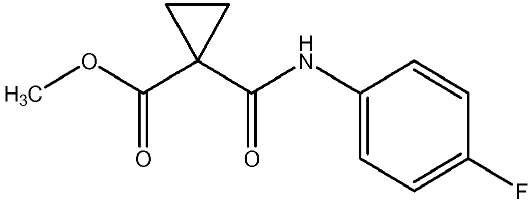 the use of isopentane as the extracting agent has the major advantage that the compound of formula (III) is completely soluble in isopentane whereas the major impurity formed by the reaction, the compound of formula (S) 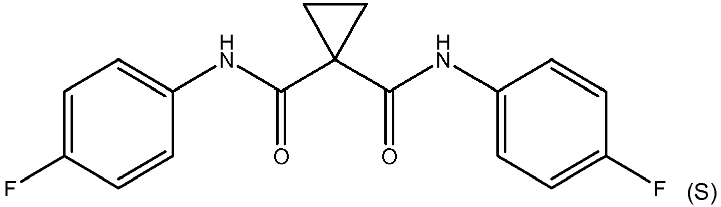 is completely insoluble in isopentane. Therefore, the use of a specific extracting agent, in particular isopentane, allows for a very simple and straight-forward separation of the valuable product, the compound of formula (III), from the major impurity of the reaction, the compound of formula (S).

Therefore, it is preferred that after (ii.a.1.1) and prior to (ii.a.1.2), the compound of formula (III) and/or of formula (III'), preferably of formula (III), has a degree of purity in the range of from 75:25 to 95:5, preferably from 85:15 to 95:1, more preferably from 92:8 to 95:5, more preferably from 93:7 to 95:5, wherein the degree of purity is defined as the molar ratio of the compound of formula (III) and/or of formula (III'), preferably of formula (III), relative to the respective symmetric malonic acid diamide of formula (S) 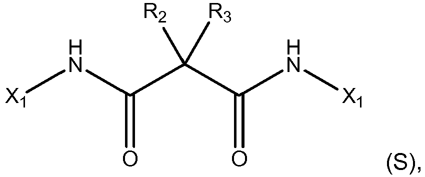 and that after (ii.a.2), the compound of formula (III) and/or of formula (III'), preferably of formula (III), has a degree of purity in the range of from 98:2 to 100:0, preferably from 99:1 to 100:0, more preferably from 99.5:0.5 to 100:0, more preferably from 99.9:0.1 to 100:0, wherein the degree of purity is defined as the molar ratio of the compound of formula (III) and/or of formula (III'), preferably of formula (III), relative to the respective symmetric malonic acid diamide of formula (S).

After step (ii.a.1), the amide bond is formed. No specific restrictions exist therefor. Preferably, the amide bond is formed by
(ii.a.2) reacting the compound of formula (III) and/or of formula (III'), preferably of formula (III), with an M_{b} salt of the compound H₂N-X₂;
wherein M_{b} is an alkali or alkaline earth metal, preferably Mg, Na or K, more preferably Na.

Preferably, X₂ is a substituted aromatic or heteroaromatic residue having from 5 to 10 atoms constituting the aromatic or heteroaromatic structure, preferably having from 6 to 10 atoms constituting the aromatic or heteroaromatic structure, wherein more preferably, X₂ is a substituted, preferably a mono-, di- or tri-substituted, aromatic or heteroaromatic residue having from 6 to 10 atoms, preferably 6 atoms, constituting the aromatic or heteroaromatic structure. For example, X₂ is mono-, di- or tri-substituted phenyl. As a further example, X₂ is an aromatic or heteroaromatic residue having from 5 to 10 atoms constituting the aromatic or heteroaromatic structure, preferably having from 6 to 10 atoms constituting the aromatic or heteroaromatic structure, substituted with one or more of halogen, alkyl, preferably C1-C6 alkyl, alkoxy, preferably C1-C6 alkoxy, alkylthioyl, preferably C1-C6 alkylthioyl, and optionally substituted aryloxy or heteroaryloxy preferably having from 5 to 10 atoms constituting the aromatic or heteroaromatic structure. As yet a further example, X₂ is an aromatic or heteroaromatic residue having from 5 to 10 atoms constituting the aromatic or heteroaromatic structure, preferably having from 6 to 10 atoms constituting the aromatic or heteroaromatic structure, mono-substituted with substituted aryloxy or heteroaryloxy preferably having from 5 to 10 atoms, more preferably having from 6 to 10 atoms, constituting the aromatic or heteroaromatic structure, wherein, more preferably, the substituted aryloxy or heteroaryloxy is substituted with one or more of halogen, preferably F and CI, alkyl, preferably C1-C6 alkyl, alkoxy, preferably C1-C6 alkoxy, alkylthioyl, preferably C1-C6 alkylthioyl, and optionally substituted aryloxy or heteroaryloxy preferably having from 5 to 10 atoms constituting the aromatic or heteroaromatic structure, wherein, for example, the the substituted aryloxy or heteroaryloxy is 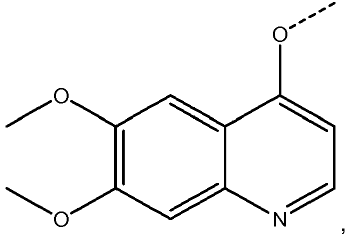 the compound H₂N-X₂ is 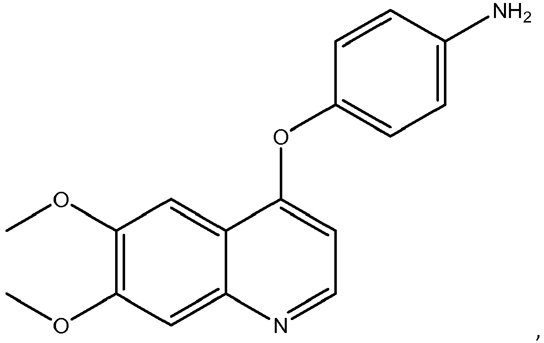 the compound of formula (I) is 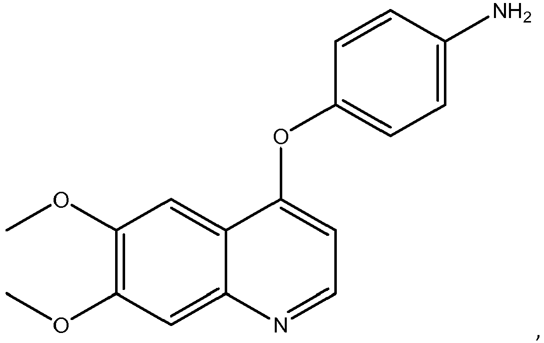 the compound H₂N-X₁ is 4-fluoroaniline, the Mₐ salt of formula (II) is 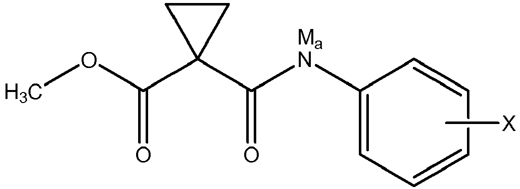 wherein X is halogen, preferably F and CI, alkyl, preferably C1-C6 alkyl, alkoxy, preferably C1-C6 alkoxy, aryloxy, or heteroaryloxy, and the compound of formula (IV) is 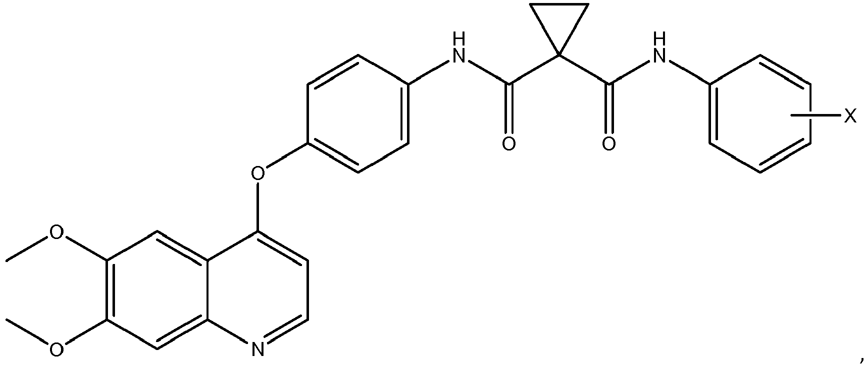 preferably

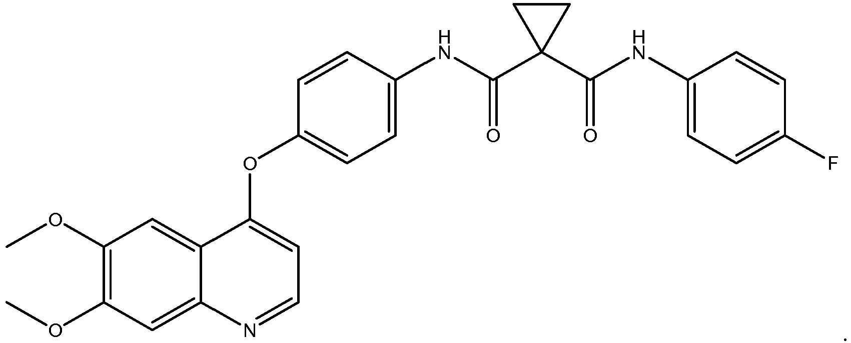

Also preferably, X₂ is 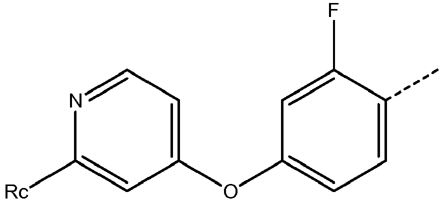 wherein Rc is halogen, azide, nitro, phtalimido, hydroxycarbonyl, alkoxycarbonyl, cyano, carbonylamino, substituted carbamoyl-N-alkyl-amino, substituted carbamoyl-N-alkyl-amino, or N-acylamino, and the compound of formula (IV) is more preferably

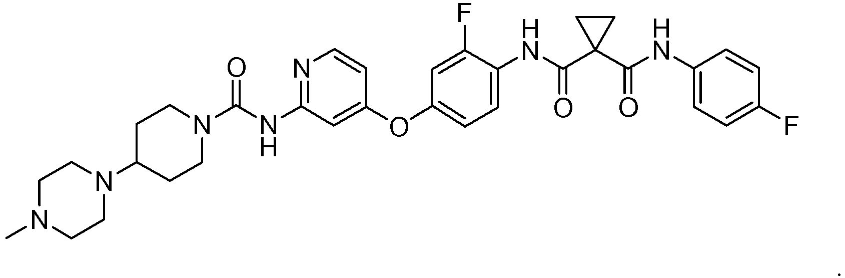

Also preferably, X₂ is 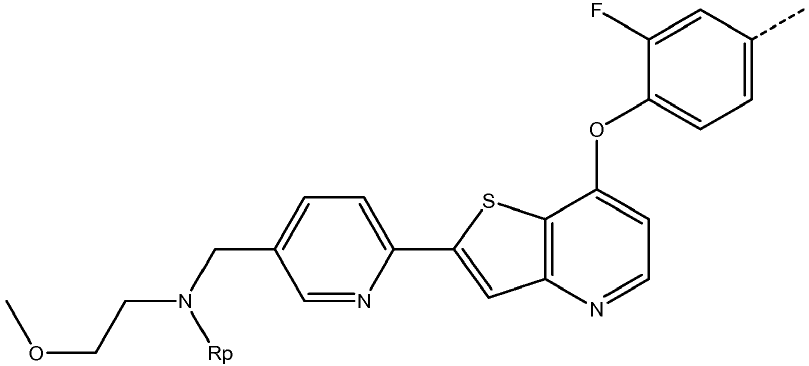 wherein Rp is hydroxyl, alkoxy, aryloxy or a nitrogen protecting group, preferably allyl, Boc, Troc, or Trityl, and the compound of formula (IV) is more preferably

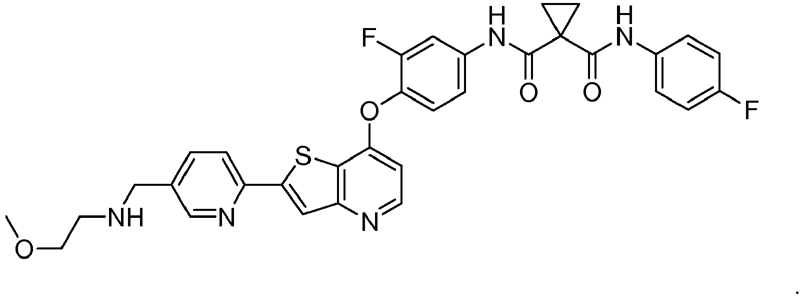

Also preferably, the compound of formula (IV) is

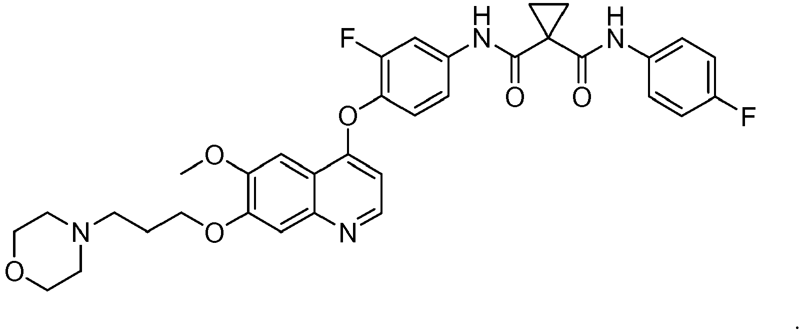

Also preferably, X₂ is an aromatic or heteroaromatic residue having from 5 to 10 atoms constituting the aromatic or heteroaromatic structure, preferably having from 6 to 10 atoms constituting the aromatic or heteroaromatic structure, tri-substituted with one or more of halogen, and substituted aryloxy or heteroaryloxy preferably having from 5 to 10 atoms constituting the aromatic or heteroaromatic structure, more preferably having from 6 to 10 atoms constituting the aromatic or heteroaromatic structure, and the compound of formula (IV) is more preferably

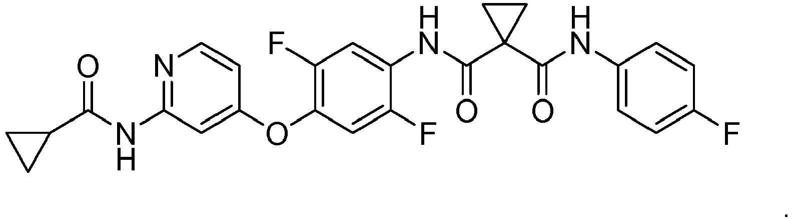

Generally, it may be possible to carry out the reaction according to (ii.a.2) in the absence of a solvent. Preferably, however, (ii.a.2) comprises the reacting in a reaction solvent. With regard to the chemical nature of the reaction solvent, no specific restrictions exist provided that the reacting according to (ii.a.2) can be carried out. However, solvents and solvent systems are preferably to be chosen for (ii.a.2) such that the M_{b} salt of the compound H₂N-Xₛ is not quickly protonated. Using a mixture of two or more suitable solvents is conceivable. Preferably, no or only low levels of protic solvents, such that the reaction according to (ii.a.2) can be carried out, are present when the compound of formula (III) or (III') is reacted with the M_{b} salt of a compound H₂N-X₂. Preferably, the reaction solvent comprises, and preferably consists of, one or more aprotic solvents, more preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran. More preferably, the reaction solvent comprises toluene. More preferably, the reaction solvent is toluene.

Preferably, with regard to (ii.a.2), the compound of formula (III) and/or of formula (III'), preferably of formula (III) is provided in a solvent for the compound of formula (III) and/or of formula (III'), preferably of formula (III). With regard to the chemical nature of the solvent for the compound of formula (III) and/or of formula (III'), preferably of formula (III), no specific restrictions exist provided that the reacting according to (ii.a.2) can be carried out. However, solvents and solvent systems are preferably to be chosen for (ii.a.2) such that the M_{b} salt of the compound H₂N-Xₛ is not quickly protonated. Using a mixture of two or more suitable solvents is conceivable. Preferably, no or only low levels of protic solvents, such that the reaction according to (ii.a.2) can be carried out, are present when the compound of formula (III) or (III') is reacted with the M_{b} salt of a compound H₂N-X₂. Preferably, the reaction solvent comprises, and preferably consists of, one or more aprotic solvents, more preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran. More preferably, the solvent for the compound of formula (III) and/or of formula (III'), preferably of formula (III) comprises toluene. More preferably, the solvent for the compound of formula (III) and/or of formula (III'), preferably of formula (III) is toluene. Preferably, the solvent for the compound of formula (III) and/or of formula (III'), preferably of formula (III) is the reaction solvent as defined above.

According to (ii.a.2), the compound of formula (III) and/or of formula (III'), preferably of formula (III) is reacted with an M_{b} salt of a compound H₂N-X₂. The M_{b} salt of a compound H₂N-X₂ can be provided or prepared according to all conceivable methods. Preferably, the M_{b} salt of a compound H₂N-X₂ is prepared by reacting the compound H₂N-X₂ with a suitable alcoholate M_{b}OR_{b}. Preferably, R_{b} is an optionally suitably substituted alkyl residue, more preferably an alkyl residue. More preferably, R_{b} is C1-C6 alkyl, more preferably C1-C5 alkyl, more preferably C1-C4 alkyl, more preferably methyl, ethyl, or tert-butyl, more preferably methyl or ethyl, more preferably methyl. Therefore, it is preferred that the residue R_{b} is R₁ or R₁', preferably R₁.

In this regard, it is preferred that the compound H₂N-X₂ is provided in a suitable solvent for the compound H₂N-X₂. With regard to the chemical nature of the solvent for the compound H₂N-X₂, no specific restrictions exist provided that the reacting according to (ii.a.2) can be carried out. Using a mixture of two or more suitable solvents is conceivable. Preferably, the solvent for the compound H₂N-X₂ comprises one or more aprotic solvents, more preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran. More preferably, the solvent for the compound H₂N-X₂ comprises toluene. More preferably, the solvent for the compound H₂N-X₂ is toluene. Preferably, the solvent for the compound H₂N-X₂ is the reaction solvent as defined above. Therefore, it is preferred that solvent for the compound H₂N-X₂ is the solvent for the compound of formula (III) and/or of formula (III'), preferably of formula (III), and this solvent is the reaction solvent.

For reacting the compound H₂N-X₂ with a suitable alcoholate M_{b}OR_{b}, it is preferred that the compound M_{b}OR_{b} is provided in a suitable solvent. With regard to the chemical nature of this solvent, no specific restrictions exist provided that the reacting can be carried out. Preferably, the solvent for the compound M_{b}OR_{b} comprises, more preferably is, an alcohol HOR_{b}, wherein R_{b} is as defined above. More preferably, HOR_{b} is methanol.

According to a first preferred alternative, (ii.a.2) comprises reacting the compound M_{b}OR_{b} with the compound H₂N-X₂, obtaining the M_{b} salt of the compound H₂N-X₂; and adding the M_{b} salt of H₂N-X₂ to the compound of formula (III) and/or of formula (III'), preferably of formula (III). According to a second preferred alternative, (ii.a.2) comprises reacting the compound M_{b}OR_{b} with a mixture of the compound H₂N-X₂ and the compound of formula (III) and/or of formula (III'), preferably of formula (III). Preferably, (ii.a.2) further comprises separating the compound HORₐ, at least partially formed during reacting the compound M_{b}OR_{b} with the compound H₂N-X₂, from the Mₐ salt of the compound H₂N-X₁, preferably by distillation or co-distillation.

Generally, no specific restrictions exist with regard to the amounts of the compounds to be reacted with each other according to (ii.a.2). Preferably, the molar ratio of the compound of formula (III) and/or of formula (III'), preferably of formula (III), provided according to (ii.a.2), relative to the compound H₂N-X₂ provided according to (ii.a.2) is in the range of from 1.5:1 to 1:1, preferably from 1.2:1 to 1:1, more preferably from 1.1:1 to 1:1, and the molar ratio of the compound M_{b}OR_{b} provided according to (ii.a.2) relative to the compound H₂N-X₂ provided according to (ii.a.2) is in the range of from 2.5:1 to 2:1, preferably from 2.2:1 to 2:1, more preferably from 2.1:1 to 2:1.

When reacting the compound of formula (III) and/or of formula (III'), preferably of formula (III) with the M_{b} salt of a compound H₂N-X₂ according to (ii.a.2), it is preferred that the reaction solvent described above forms an azeotropic mixture with the compound HOR_{b}. Preferably, the reacting of the compound of formula (III) and/or of formula (III'), preferably of formula (III) with the M_{b} salt of a compound H₂N-X₂ in the presence of the reaction solvent is carried out at a temperature which allows for distilling off this azeotropic mixture such that the alcohol formed by the reaction of the alcoholate with the amine can be removed, thus driving equilibrium to the direction of the M_{b} salt of a compound H₂N-X₂. Further, when reacting the compound of formula (III) and/or of formula (III'), preferably of formula (III) with the M_{b} salt of a compound H₂N-X₂ according to (ii.a.2), it is preferred that the reaction solvent described above forms an azeotropic mixture with the compound HOR₁ or HOR₁, , preferably HOR₁. Preferably, the reacting of the compound of formula (III) and/or of formula (III'), preferably of formula (III) with the M_{b} salt of a compound H₂N-X₂ in the presence of the reaction solvent is carried out at a temperature which allows for distilling off this azeotropic mixture. If, for example, HOR_{b} is HOCH₃, HOR₁ or HOR₁', preferably HOR₁ is HOCH₃, and the solvent for the compound H₂N-X₂ is toluene and, the temperature allowing for distilling off the azeotropic mixture comprising, preferably essentially consisting of methanol and toluene is preferably at least 65 °C, more preferably at least 70 °C, more preferably at least 80 °C, more preferably in the range of from 80 to 100 °C, more preferably in the range of from 90 to 100 °C. By removing the alcohol formed by the reaction of the alcoholate with the amine by way of azeotropic distillation, the equilibrium of the alcoholate / amin system will be driven to the direction of the M_{b} salt of a compound H₂N-X₂, which can then react with an ester functionality of the compound of formula (III) or (III').

From the mixture obtained from (ii.a.2), the compound of formula (IV) can be separated according any suitable method, if so desired. Preferably, such separating comprises
(ii.a.3.1) adding an organic solvent to the reaction mixture obtained from (ii.a.2);
(ii.a.3.2) adding an acid to the mixture obtained from (ii.a.3.1), obtaining the compound of formula (IV) dissolved in the organic solvent according to (ii.a.3.1)
(ii.a.3.3) separating the organic solvent from the compound of formula (IV);
(ii.a.3.4) extracting the compound of formula (IV) with an extracting agent.

Preferably, the organic solvent according to (ii.a.3.1) comprises one or more of ethyl acetate, ispropyl acetate, butyl acetate, methyl tert-butyl ether, and dichloromethane, preferably ethyl acetate. Preferably, the acid according to (ii.a.3.2) comprises ammonium chloride, preferably aqueous ammonium chloride, more preferably saturated aqueous ammonium chloride, wherein more preferably the acid is saturated aqueous ammonium chloride. Preferably, the separating the organic solvent according to (ii.a.3.3) comprises evaporating the organic solvent. Preferably, the extracting agent according to (ii.a.3.4) comprises, preferably is, methyl tert-butyl ether.

It is disclosed a composition comprising the compound of formula (IV), obtainable or obtained from (ii.a.3.4), and to the compound of formula (IV), obtainable or obtained from (ii.a.3.4).

### (b)

According to a second preferred embodiment of the present invention, said converting according to (ii) comprises
(ii.b.1) reacting the compound of formula (II) and/or of formula (II') with an M_{b} salt of a compound H₂N-X₂;
wherein M_{b} is an alkali or alkaline earth metal, preferably Mg, Na or K, more preferably Na.

Therefore, compared to the first preferred embodiment described above, the Mₐ salt, compound of formula (II) and/or of formula (II'), is directly reacted with the M_{b} salt of a compound H₂N-X₂, without converting the Mₐ salt to the respective amide.

Preferably, X₂ is a substituted aromatic or heteroaromatic residue having from 5 to 10 atoms constituting the aromatic or heteroaromatic structure, preferably having from 6 to 10 atoms constituting the aromatic or heteroaromatic structure, wherein more preferably, X₂ is a substituted, preferably a mono-, di- or tri-substituted, aromatic or heteroaromatic residue having from 6 to 10 atoms, preferably 6 atoms, constituting the aromatic or heteroaromatic structure. For example, X₂ is mono-, di- or tri-substituted phenyl. As a further example, X₂ is an aromatic or heteroaromatic residue having from 5 to 10 atoms constituting the aromatic or heteroaromatic structure, preferably having from 6 to 10 atoms constituting the aromatic or heteroaromatic structure, substituted with one or more of halogen, alkyl, preferably C1-C6 alkyl, alkoxy, preferably C1-C6 alkoxy, alkylthioyl, preferably C1-C6 alkylthioyl, and optionally substituted aryloxy or heteroaryloxy preferably having from 5 to 10 atoms constituting the aromatic or heteroaromatic structure. As yet a further example, X₂ is an aromatic or heteroaromatic residue having from 5 to 10 atoms constituting the aromatic or heteroaromatic structure, preferably having from 6 to 10 atoms constituting the aromatic or heteroaromatic structure, mono-substituted with substituted aryloxy or heteroaryloxy preferably having from 5 to 10 atoms, more preferably having from 6 to 10 atoms, constituting the aromatic or heteroaromatic structure, wherein, more preferably, the substituted aryloxy or heteroaryloxy is substituted with one or more of halogen, preferably F and CI, alkyl, preferably C1-C6 alkyl, alkoxy, preferably C1-C6 alkoxy, alkylthioyl, preferably C1-C6 alkylthioyl, and optionally substituted aryloxy or heteroaryloxy preferably having from 5 to 10 atoms constituting the aromatic or heteroaromatic structure, wherein, for example, the the substituted aryloxy or heteroaryloxy is 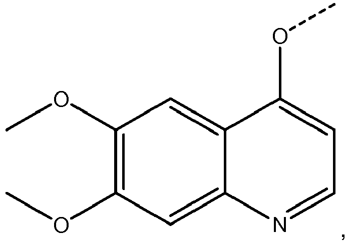 the compound H₂N-X₂ is 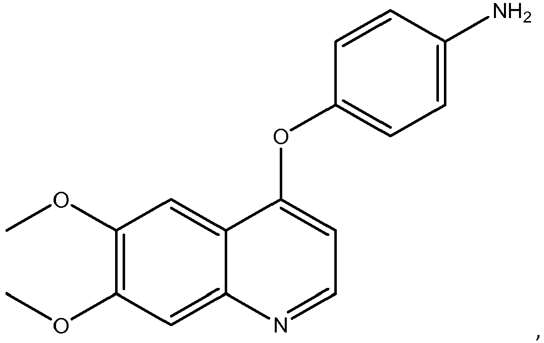 the compound of formula (I) is 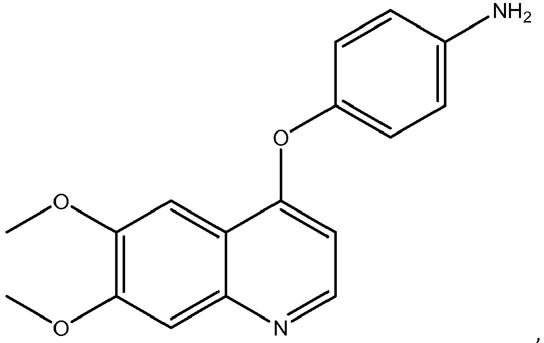 the compound H₂N-X₁ is 4-fluoroaniline, the Mₐ salt of formula (II) is 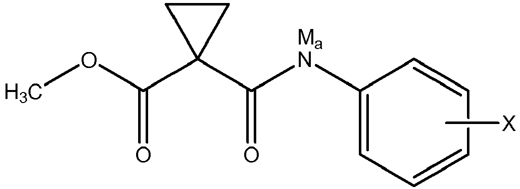 wherein X is halogen, preferably F and CI, alkyl, preferably C1-C6 alkyl, alkoxy, preferably C1-C6 alkoxy, aryloxy, or heteroaryloxy, and the compound of formula (IV) is 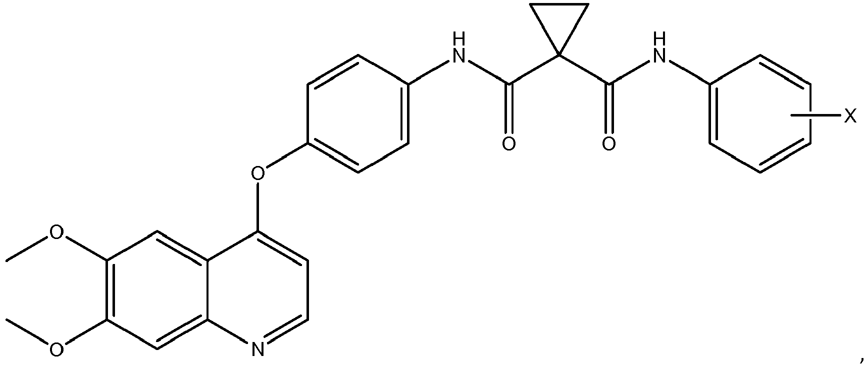 preferably

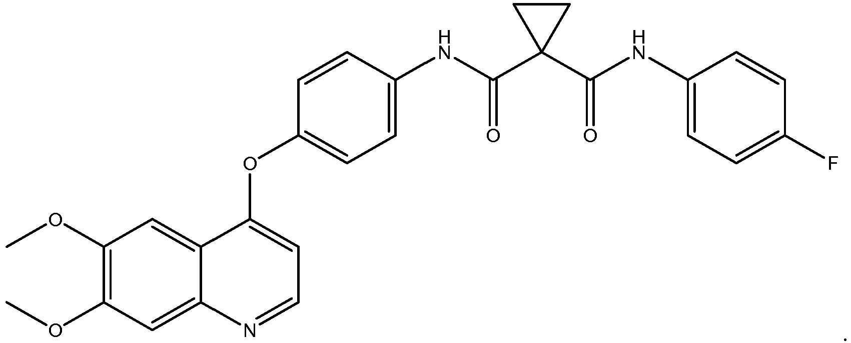

Also preferably, X₂ is 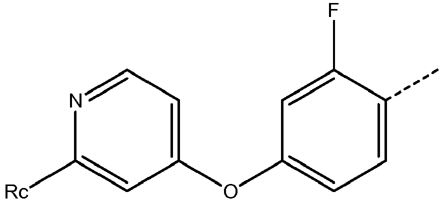 wherein Rc is halogen, azide, nitro, phtalimido, hydroxycarbonyl, alkoxycarbonyl, cyano, carbonylamino, substituted carbamoyl-N-alkyl-amino, substituted carbamoyl-N-alkyl-amino, or N-acylamino, and the compound of formula (IV) is more preferably

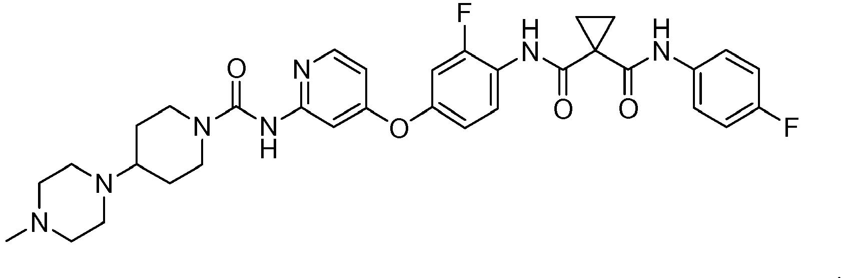

Also preferably, X₂ is 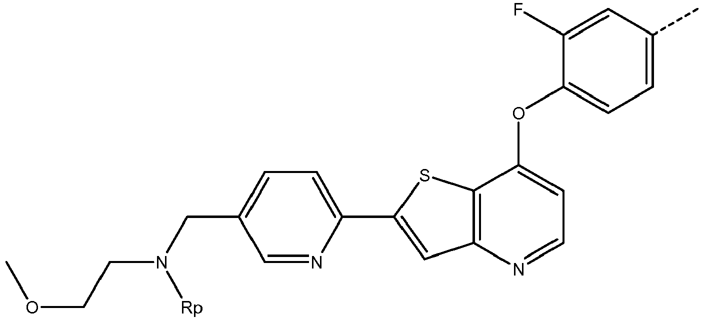 wherein Rp is hydroxyl, alkoxy, aryloxy or a nitrogen protecting group, preferably allyl, Boc, Troc, or Trityl, and the compound of formula (IV) is more preferably

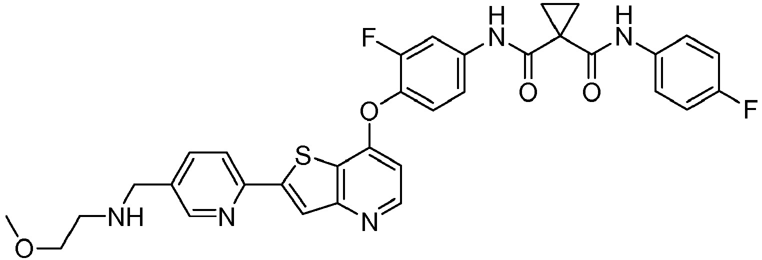

Also preferably, the compound of formula (IV) is

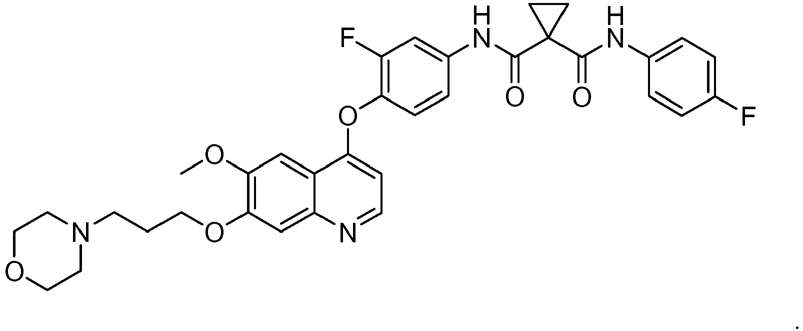

Also preferably, X₂ is an aromatic or heteroaromatic residue having from 5 to 10 atoms constituting the aromatic or heteroaromatic structure, preferably having from 6 to 10 atoms constituting the aromatic or heteroaromatic structure, tri-substituted with one or more of halogen, and substituted aryloxy or heteroaryloxy preferably having from 5 to 10 atoms constituting the aromatic or heteroaromatic structure, more preferably having from 6 to 10 atoms constituting the aromatic or heteroaromatic structure, and the compound of formula (IV) is more preferably

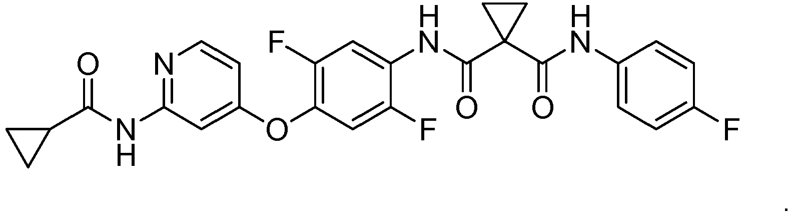

Generally, it may be possible to carry out the reaction according to (ii.b.1) in the absence of a solvent. Preferably, however, (ii.b.1) comprises the reacting in a reaction solvent. With regard to the chemical nature of the reaction solvent, no specific restrictions exist provided that the reacting according to (ii.b.1) can be carried out. However, solvents and solvent systems are preferably to be chosen for (ii.b.1) such that the M_{b} salt of the compound H₂N-Xₛ is not quickly protonated. Using a mixture of two or more suitable solvents is conceivable. Preferably, no or only low levels of protic solvents, such that the reaction according to (ii.b.1) can be carried out, are present when the compound of formula (II) or (II') is reacted with the M_{b} salt of a compound H₂N-X₂. Preferably, the reaction solvent comprises, and preferably consists of, one or more aprotic solvents, more preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran. More preferably, the reaction solvent comprises toluene. More preferably, the reaction solvent is toluene.

Preferably, in (ii.b.1), the compound of formula (II) and/or of formula (II'), preferably of formula (II), the compound H₂N-X_{b}, and a compound M_{b}OR_{b} wherein R_{b} is alkyl are provided. Preferably, R_{b} is C1-C6 alkyl, more preferably C1-C5 alkyl, more preferably C1-C4 alkyl, more preferably methyl, ethyl, or tert-butyl, more preferably methyl or ethyl, more preferably methyl. Therefore, it is preferred that the residue R_{b} is R₁ or R₁', preferably R₁.

Preferably, with regard to (ii.b.1), the compound of formula (II) and/or of formula (II'), preferably of formula (II) is provided in a solvent for the compound of formula (II) and/or of formula (II'), preferably of formula (II). With regard to the chemical nature of the solvent for the compound of formula (II) and/or of formula (II'), preferably of formula (II), no specific restrictions exist provided that the reacting according to (ii.b.1) can be carried out. Using a mixture of two or more suitable solvents is conceivable. Preferably, the solvent for the compound of formula (II) and/or of formula (II'), preferably of formula (II) comprises one or more aprotic solvents, more preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran. More preferably, the solvent for the compound of formula (II) and/or of formula (II'), preferably of formula (II) comprises toluene. More preferably, the solvent for the compound of formula (II) and/or of formula (II'), preferably of formula (II) is toluene. Preferably, the solvent for the compound of formula (II) and/or of formula (II'), preferably of formula (III) is the reaction solvent as defined above.

In this regard, it is preferred that the compound H₂N-X₂ is provided in a suitable solvent for the compound H₂N-X₂. With regard to the chemical nature of the solvent for the compound H₂N-X₂, no specific restrictions exist provided that the reacting according to (ii.b.1) can be carried out. Using a mixture of two or more suitable solvents is conceivable. Preferably, the solvent for the compound H₂N-X₂ comprises one or more aprotic solvents, more preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran. More preferably, the solvent for the compound H₂N-X₂ comprises toluene. More preferably, the solvent for the compound H₂N-X₂ is toluene. Preferably, the solvent for the compound H₂N-X₂ is the reaction solvent as defined above. Therefore, it is preferred that solvent for the compound H₂N-X₂ is the solvent for the compound of formula (II) and/or of formula (II'), preferably of formula (II), and this solvent is the reaction solvent.

For reacting the compound H₂N-X₂ with a suitable alcoholate M_{b}OR_{b}, it is preferred that the compound M_{b}OR_{b} is provided in a suitable solvent. With regard to the chemical nature of this solvent, no specific restrictions exist provided that the reacting can be carried out. Preferably, the solvent for the compound M_{b}OR_{b} comprises one or more aprotic solvents, preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran. More preferably, the solvent for the compound M_{b}OR_{b} comprises, preferably is, toluene.

According to a first preferred alternative, (ii.b.1) comprises reacting the compound M_{b}OR_{b} with the compound H₂N-X₂, obtaining the M_{b} salt of the compound H₂N-X₂; and adding the M_{b} salt of H₂N-X₂ to the compound of formula (II) and/or of formula (II'), preferably of formula (II). According to a second preferred alternative, (ii.b.1) comprises adding the compound H₂N-X₂ to the Mₐ salt of compound of formula (II) and/or of formula (II'), or the mixture of the Mₐ salt of formula (II) and the Mₐ salt of formula (II'), and then adding the compound M_{b}OR_{b} to the reaction mixture.

Generally, no specific restrictions exist with regard to the amounts of the compounds to be reacted with each other according to (ii.b.1). Preferably, the molar ratio of the compound of formula (II) and/or of formula (II'), preferably of formula (II), provided according to (ii.b.1), relative to the compound H₂N-X₂ provided according to (ii.b.1) is in the range of from 1.5:1 to 1:1, preferably from 1.2:1 to 1:1, more preferably from 1.1:1 to 1:1, and the molar ratio of the compound M_{b}OR_{b} provided according to (ii.b.1) relative to the compound H₂N-X₂ provided according to (ii.b.1) is in the range of from 1.5:1 to 1:1, preferably from 1.2:1 to 1:1, more preferably from 1.1:1 to 1:1.

With regard to this preferred embodiment, it is preferred that (i) and (ii.b.1) are carried out in the same solvent, preferably in toluene, wherein it is more preferred that (i) and (ii.b.1) are carried out as a one-pot process, which one-pot process avoids the need to separate the compound of formula (II) or (II'), preferably (II'), from the reaction mixture obtained from (i).

When reacting the compound of formula (II) and/or of formula (II'), preferably of formula (II) with the M_{b} salt of a compound H₂N-X₂ according to (ii.b.1), it is preferred that the reaction solvent described above forms an azeotropic mixture with the compound HOR_{b}. Preferably, the reacting of the compound of formula (II) and/or of formula (II'), preferably of formula (II) with the M_{b} salt of a compound H₂N-X₂ in the presence of the reaction solvent is carried out at a temperature which allows for distilling off this azeotropic mixture such that the alcohol formed by the reaction of the alcoholate with the amine can be removed, thus driving equilibrium to the direction of the M_{b} salt of a compound H₂N-X₂.. Further, when reacting the compound of formula (II) and/or of formula (II'), preferably of formula (II) with the M_{b} salt of a compound H₂N-X₂ according to (ii.b.1), it is preferred that the reaction solvent described above forms an azeotropic mixture with the compound HOR₁ or HOR₁, , preferably HOR₁. Preferably, the reacting of the compound of formula (II) and/or of formula (II'), preferably of formula (II) with the M_{b} salt of a compound H₂N-X₂ in the presence of the reaction solvent is carried out at a temperature which allows for distilling off this azeotropic mixture. If, for example, HOR_{b} is HOCH₃, HOR₁ or HOR₁', preferably HOR₁ is HOCH₃, and the solvent for the compound H₂N-X₂ is toluene and, the temperature allowing for distilling off the azeotropic mixture comprising, preferably essentially consisting of methanol and toluene is preferably at least 65 °C, more preferably at least 70 °C, more preferably at least 80 °C, more preferably in the range of from 80 to 100 °C, more preferably in the range of from 90 to 100 °C. By removing the alcohol formed by the reaction of the alcoholate with the amine by way of azeotropic distillation, the equilibrium of the alcoholate / amine system will be driven to the direction of the M_{b} salt of a compound H₂N-X₂, which can then react with an ester functionality of the compound of formula (II) or (II').

From the mixture obtained from (ii.b.1), the compound of formula (IV) can be separated according any suitable method, if so desired. Preferably, such separating comprises
(ii.b.2.1) adding an organic solvent to the reaction mixture obtained from (ii.b.1);
(ii.b.2.2) adding an acid to the mixture obtained from (ii.b.2.1), obtaining the compound of formula (IV) dissolved in the organic solvent according to (ii.b.2.1);
(ii.b.2.3) separating the organic solvent from the compound of formula (IV);
(ii.b.2.4) extracting the compound of formula (IV) with an extracting agent.

Preferably, the organic solvent according to (ii.b.2.1) comprises one or more of ethyl acetate, ispropyl acetate, butyl acetate, methyl tert-butyl ether, and dichloromethane, preferably ethyl acetate. Preferably, the acid according to (ii.b.2.2) comprises ammonium chloride, preferably aqueous ammonium chloride, more preferably saturated aqueous ammonium chloride, wherein more preferably the acid is saturated aqueous ammonium chloride. Preferably, the separating the organic solvent according to (ii.b.2.3) comprises evaporating the organic solvent. Preferably, the extracting agent according to (ii.b.2.4) comprises, preferably is, methyl tert-butyl ether.

It is disclosed a composition comprising the compound of formula (IV), obtainable or obtained from (ii.b.2.4), and to the compound of formula (IV), obtainable or obtained from (ii.b.2.4).

### (c)

According to a third preferred embodiment of the present invention, said converting according to (ii) is realized by a process wherein the amide bond is formed by a process comprising (ii.c.1) reacting the compound of formula (III) and/or of formula (III'), preferably of formula
(III) with a compound H₂N-X₂;
wherein X₂ is a substituted or unsubstituted alkyl, alkenyl or cycloalkyl residue.

Primary and secondary alkylamines, alkenylamines and cycloalkylamines are sufficiently reactive so that they can be reacted directly with the ester functionality of the compound of formula (III) and/or of formula (III'), preferably of formula (III), by methods well known in the art, such as direct reaction at elevated temperatures.

Regarding the reaction conditions for (ii.c.1), no specific restrictions exist provide that the compound of formula (IV) is obtained.

### (d)

According to a fourth preferred embodiment of the present invention, said converting according to (ii) is realized by a process wherein the amide bond is formed by a process comprising
(ii.d.1) saponification of a compound of formula (III) and/or of formula (III'), preferably of formula (III).

Regarding the saponification conditions for (ii.d.1), no specific restrictions exist and will be suitably chosen by the skilled person. Preferably, the saponification for (ii.d.1) will be chosen so that the carboxylate or carbonic acid obtained by saponification is obtained as intermediate. Preferably, based on this intermediate, the process further comprises
(ii.d.2) converting the carboxylate or carbonic acid to an activated carbonic acid derivative capable of forming an amide bond with a primary amine prior to a further reacting with the compound H₂N-X₂ or an alkali or earth alkali metal salt thereof.

Said converting according to (ii.d.2) can be carried out using all suitable activating agents known by the skilled person provided that the respectively obtained carbonic acid derivative is more susceptible to nucleophilic attack than a free carboxyl group. By way of example, acyl chlorides and thioesters are activated carbonic acid derivatives. Regarding the reaction conditions for (ii.d.2), no specific restrictions exist provide that the activated carbonic acid derivative is obtained. For the preparation of activated carbonic acid derivatives reference is made to Jerry March, Advanced organic chemistry, John Wiley and sons, 7th edition.

Based on the activated carbonic acid derivative, it is preferred to prepare the compound of formula (IV) by
(ii.d.3) reacting the activated carbonic acid derivative with the compound H₂N-X₂ or an alkali or earth alkali metal salt thereof.

With regard to preferred, more preferred and especially preferred residues X₂ and the respectively preferred compounds of formula (IV), reference is made to the respective disclosure hereinabove in the context of the first preferred embodiment in section (a) and the second preferred embodiment in section (b).

Regarding the reaction conditions for (ii.d.3), no specific restrictions exist provide that the compound of formula (IV) is obtained.

### (e)

According to a fifth preferred embodiment of the present invention, said converting according to (ii) is realized by a process wherein the amide bond is formed by a process comprising
(ii.e.1) saponification of a compound of formula (II) and/or of formula (II'), preferably of formula (III).

Regarding the saponification conditions for (ii.e.1), no specific restrictions exist and will be suitably chosen by the skilled person. Preferably, the saponification for (ii.e.1) will be chosen so that the carboxylate or carbonic acid obtained by saponification is obtained as intermediate. Preferably, based on this intermediate, the process further comprises
(ii.e.2) converting the carboxylate or carbonic acid to an activated carbonic acid derivative capable of forming an amide bond with a primary amine prior to a further reacting with the compound H₂N-X₂ or an alkali or earth alkali metal salt thereof.

Said converting according to (ii.e.2) can be carried out using all suitable activating agents known by the skilled person provided that the respectively obtained carbonic acid derivative is more susceptible to nucleophilic attack than a free carboxyl group. By way of example, acyl chlorides and thioesters are activated carbonic acid derivatives. Regarding the reaction conditions for (ii.d.2), no specific restrictions exist provide that the activated carbonic acid derivative is obtained. For the preparation of activated carbonic acid derivatives reference is made to Jerry March, Advanced organic chemistry, John Wiley and sons, 7th edition.

Based on the activated carbonic acid derivative, it is preferred to prepare the compound of formula (IV) by
(ii.e.3) reacting the activated carbonic acid derivative with the compound H₂N-X₂ or an alkali or earth alkali metal salt thereof.

With regard to preferred, more preferred and especially preferred residues X₂ and the respectively preferred compounds of formula (IV), reference is made to the respective disclosure hereinabove in the context of the first preferred embodiment in section (a) and the second preferred embodiment in section (b).

Regarding the reaction conditions for (ii.e.3), no specific restrictions exist provide that the compound of formula (IV) is obtained.

### (2)

According to the inventive concept, it is further preferred that a 1,1-disubstituted dicarboxylic acid ester wherein one carboxylic acid group is derivatized as amide group is reacted with the alkali metal salts or alkaline earth metal salts of an amine and to convert, in a second step, the alkali metal salt or alkaline earth metal salt to the asymmetric bisamide.

Therefore, the present invention relates to a process for preparing an asymmetric malonic acid diamide of formula (IV) 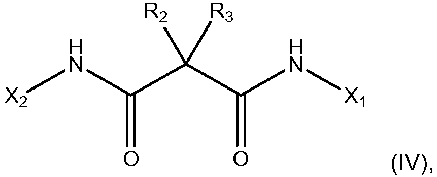 or a pharmaceutically acceptable salt thereof, the process comprising
(i) reacting a compound of formula (III) 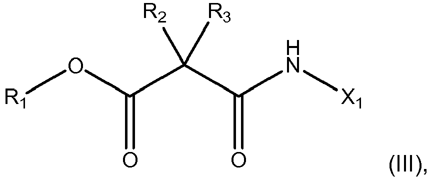 or a compound of formula (III') 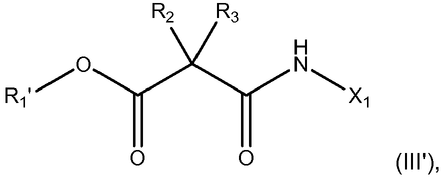 or a mixture of the compound of formula (III) and the compound of formula (III') with an M_{b} salt of a compound H₂N-X₂, obtaining a reaction mixture comprising an M_{b} salt of the compound of formula (IV);
(ii) converting the M_{b} salt of the compound of formula (IV) to the compound of formula (IV); wherein
   R₁ and R₁' are alkyl or aryl, and R₁ and R₁' are the same or different;
   R₂ and R₃ are alkyl, alkoxy, aryl or heteroaryl and R₂ and R₃ are the same or different, or R₂ and R₃, together with the C atom at which R₂ and R₃ are attached, form a cycle;
   X₁ is a substituted aromatic or heteroaromatic residue;
   X₂ is a substituted or unsubstituted alkyl, alkenyl, cycloalkyl, aromatic or heteroaromatic residue wherein X₁ is different from X₂;
   M_{b} is an alkali or alkaline earth metal.

Regarding step (i) according to this section (2), full reference is made to step (ii.a.2) according to section (1) above. All reaction conditions and preferred reaction conditions, and all definitions of compounds and residues and preferred compounds and residues described in (1), step (ii.1.2) are identical to those of step (i) in this section (2).

From the mixture obtained from (i) - as mentioned, in all aspects identical to (ii.a.2) of section (1) above -, the compound of formula (IV) can be separated according any suitable method, if so desired. Preferably, such separating comprises
(ii.1) adding an organic solvent to the reaction mixture obtained from (i);
(ii.2) adding an acid to the mixture obtained from (ii.a.1), obtaining the compound of formula (IV) dissolved in the organic solvent according to (ii.1)
(ii.3) separating the organic solvent from the compound of formula (IV);
(ii.4) extracting the compound of formula (IV) with an extracting agent.

Preferably, the organic solvent according to (ii.1) comprises one or more of ethyl acetate, ispropyl acetate, butyl acetate, methyl tert-butyl ether, and dichloromethane, preferably ethyl acetate. Preferably, the acid according to (ii.2) comprises ammonium chloride, preferably aqueous ammonium chloride, more preferably saturated aqueous ammonium chloride, wherein more preferably the acid is saturated aqueous ammonium chloride. Preferably, the separating the organic solvent according to (ii.3) comprises evaporating the organic solvent. Preferably, the extracting agent according to (ii.4) comprises, preferably is, methyl tert-butyl ether.

It is disclosed a composition comprising the compound of formula (IV), obtainable or obtained from (ii.4), and to the compound of formula (IV), obtainable or obtained from (ii.4).

With regard to the compound of formula (III), the compound of formula (III'), or the compound of formula (III) and the compound of formula (III') used as starting material in step (i) above, in this section (2), no restrictions exist how these compound(s) is/are provided and/or prepared. Preferably, the compound of formula (III) or the compound of formula (III') or the mixture of the compound of formula (II) and the compound of formula (III'), preferably the compound of formula (III), is obtainable or obtained by a process as defined in section (1), above, in particular obtainable or obtained by step (ii.a.1) of the process as defined in section (1) above.

### (1) + (2)

The compound of formula (IV), obtained according to the processes as described in sections (1) and (2) above, can be further processed to form a salt. Therefore, the present invention also relates to the process as described above in section (1) and the process as described above in section (2), further comprising (iii) preparing a pharmaceutically acceptable salt of the compound of formula (IV).

Preferably, the salt is a malate, preferably a monomalate, or a succinate, preferably a monosuccinate, or a phosphate, preferably a monophosphate.

### (3)

The disclosure further comprises a process for enriching a 1,1-disubstituted dicarboxylic acid ester wherein one carboxylic acid group is derivatized as an amide group from a mixture which, in addition to this compound, contains the respective 1,1-disubstituted dicarboxylic acid ester wherein both carboxylic acid groups are derivatized as amide groups.

Therefore, also disclosed herein is a process for enriching a compound of formula (III) 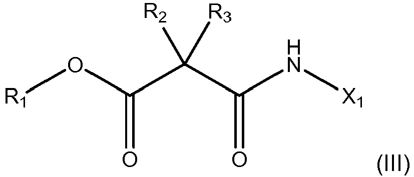 from a starting mixture comprising the compound of formula (III) and a compound of formula (S) 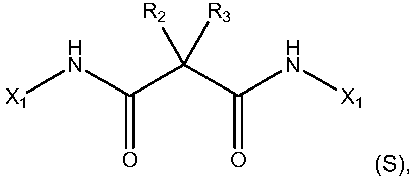 said process comprising contacting the starting mixture with an extracting agent comprising isopentane, obtaining a liquid phase comprising the extracting agent and the compound of formula (III), wherein in said liquid phase, the molar ratio of the compound of formula (III) relative to the compound (S) is higher than the respective molar ratio in the starting mixture; wherein
R₁ is alkyl or aryl;
R₂ and R₃, together with the C atom at which R₂ and R₃ are attached, form a cycle having 3 carbon atoms;
X₁ is 4-haloaniline, preferably 4-fluoroaniline.

With regard to preferred definitions of the residue R₁, reference is made to the respective disclosure in section (1) above. R₁ is C1-C6 alkyl, preferably C1-C3 alkyl, more preferably C1 or C2 alkyl, wherein more preferably, the compound of formula (III) is the compound

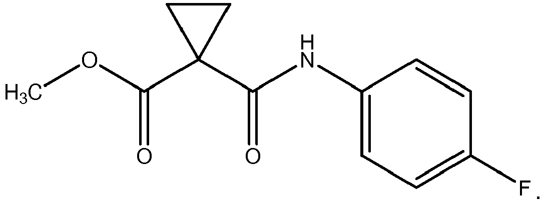

While there are no specific restrictions concerning the further composition of the starting mixture comprising the compound of formula (III) and the compound of formula (S), it is preferred that it comprises the compound of formula (III), the compound of formula (S), and a reaction solvent, wherein the reaction solvent preferably comprises one or more aprotic solvents, more preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran, wherein more preferably, the starting mixture comprises the compound of formula (III), the compound of formula (S), and toluene, said starting mixture optionally comprising water.

More preferably, said starting mixture is obtainable or obtained by the step (ii.a.1.1) of the process as described in section (1) hereinabove. Therefore, the process for enriching a compound of formula (III) preferably further comprises preparing the starting mixture by a method comprising step (ii.a.1.1) as described in section (1) hereinabove, preferably comprising said step (ii.a.1.1) as last step.

Further preferably, the enriching comprises extracting the compound of formula (III) with dichloromethane as a first extracting agent from the starting mixture, separating the first extracting agent, preferably by evaporation, obtaining a mixture comprising the compound of formula (III), and extracting the compound of formula (III) from the mixture with isopentane as a second extracting agent, obtaining the liquid phase. The extracting agent comprising isopentane is then preferably separated from the liquid phase, wherein said separation agent preferably comprising, more preferably consisting of, evaporating the extracting agent comprising isopentane. From said separating, preferably evaporation, the compound of formula (III) is preferably obtained as a solid, more preferably as a crystalline solid.

Prior to said enriching, the compound of formula (III) can have, preferably has a degree of purity in the range of from 75:25 to 95:5, more preferably from 85:15 to 95:1, more preferably from 92:8 to 95:5, more preferably from 93:7 to 95:5 in the starting mixture, wherein after the enriching, the compound of formula (III) preferably has a degree of purity in the range of from 98:2 to 100:0, more preferably from 99:1 to 100:0, more preferably from 99.5:0.5 to 100:0, more preferably from 99.9:0.1 to 100:0 in the liquid phase, wherein the degree of purity is defined as the molar ratio of the compound of formula (III) relative to the compound of formula (S).

### (4)

The inventive concept further generally comprises the use of an alkali or alkaline earth metal salt of an amine for preparing an asymmetric bisamide. In particular, the present invention also relates to the use of an M_{b} salt of a compound H₂N-X₂ for preparing an asymmetric malonic acid diamide formula (IV) wherein 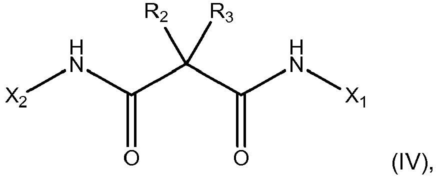
R₂ and R₃ are alkyl, alkoxy, aryl or heteroaryl and R₂ and R₃ are the same or different, or R₂ and R₃, together with the C atom at which R₂ and R₃ are attached, form a cycle;
X₁ is a substituted aromatic or heteroaromatic residue;
X₂ is a substituted or unsubstituted alkyl, alkenyl, cycloalkyl, aromatic or heteroaromatic residue wherein X₁ is different from X₂;
M_{b} is an alkali or alkaline earth metal.

Regarding the definitions of R₂, R₃, X₁, X₂ and M_{b}, full reference is made to the respective definitions in section (1) hereinabove.

Preferably, the present invention relates to said use for preparing a compound of formula (IV) wherein the compound of formula (IV) is 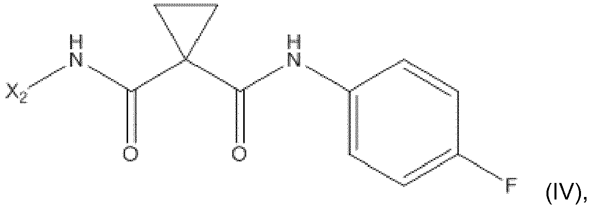 wherein X₂ is a substituted aromatic or heteroaromatic residue different from para-C₆H₄-F.

More preferably, the present invention relates to said use for preparing a compound of formula (IV) selected from the group consisting of 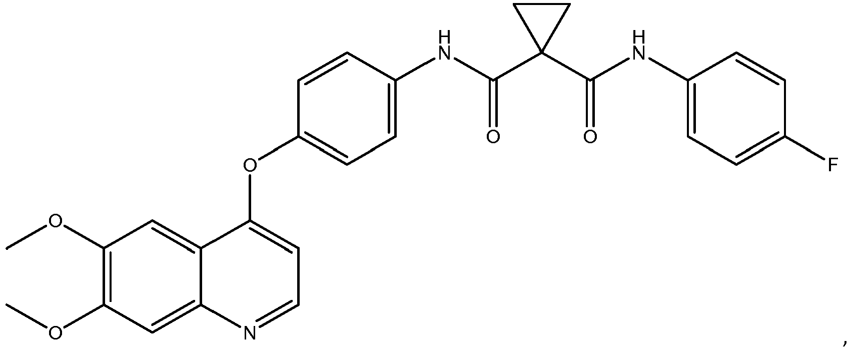 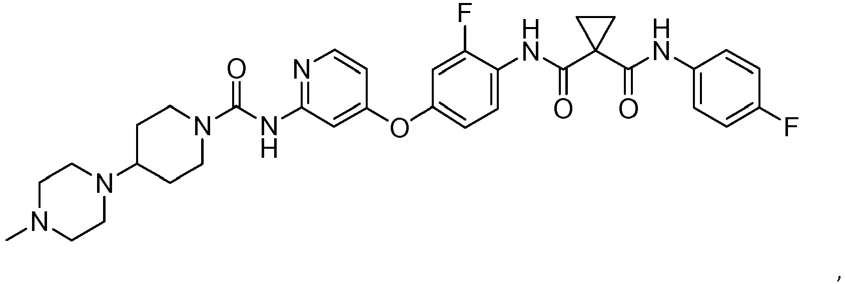 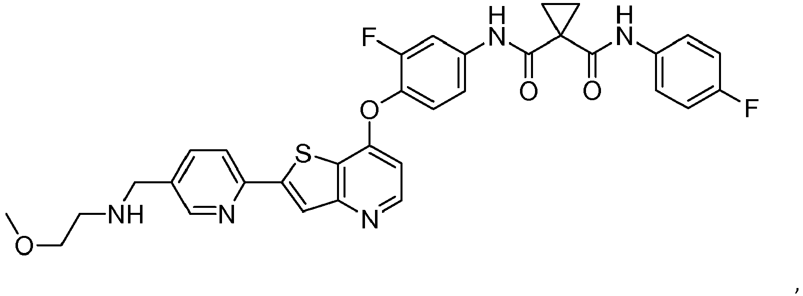 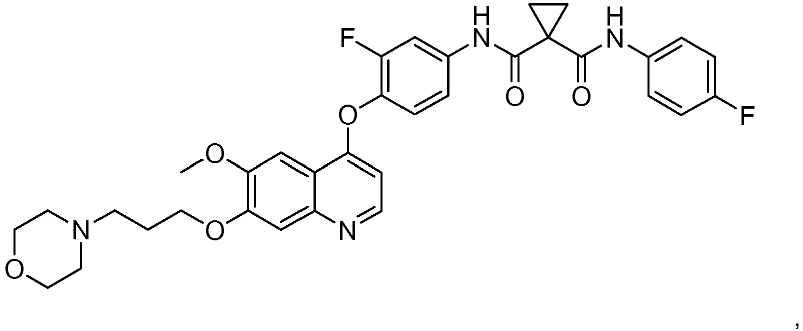 and

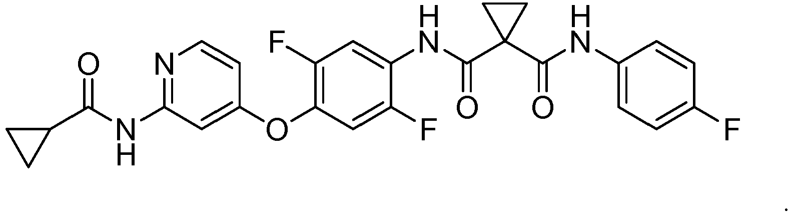

The present invention is further illustrated by the following embodiments and combinations of embodiments resulting from the respective dependencies and references as indicated:
1. A process for preparing an asymmetric malonic acid diamide of formula (IV) 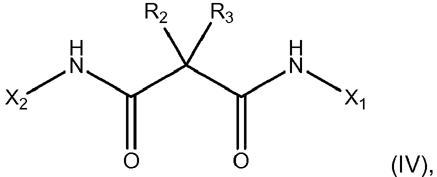 or a pharmaceutically acceptable salt thereof, the process comprising
   (i) reacting a compound of formula (I) 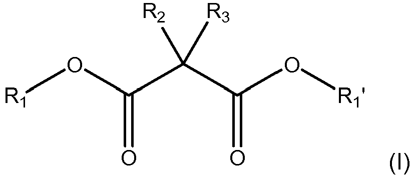 with an Mₐ salt of a compound H₂N-X₁, obtaining a reaction mixture comprising an Mₐ salt of formula (II) 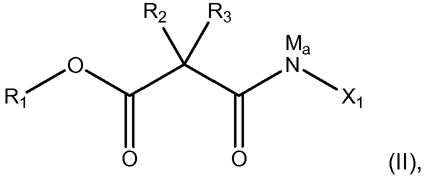 or an Mₐ salt of formula (II') 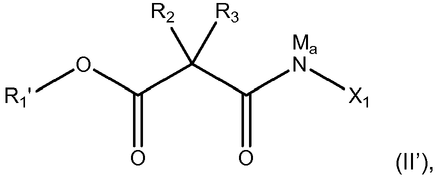 or a mixture of the Mₐ salt of formula (II) and the Mₐ salt of formula (II');
   (ii) converting the Mₐ salt of formula (II) and/or of formula (II') to the compound of formula (IV) comprising forming an amide bond between the carbon atom of the ester moiety comprised in the compound of formula (II) and/or formula (II'), preferably of formula (II), and the nitrogen atom comprised in a compound H₂N-X₂; wherein
      R₁ and R₁' are alkyl or aryl, and R₁ and R₁' are the same or different;
      R₂ and R₃ are alkyl, alkoxy, aryl or heteroaryl and R₂ and R₃ are the same or different, or
      R₂ and R₃, together with the C atom at which R₂ and R₃ are attached, form a cycle;
      X₁ is a substituted aromatic or heteroaromatic residue;
      X₂ is a substituted or unsubstituted alkyl, alkenyl, cycloalkyl, aromatic or heteroaromatic residue wherein X₁ is different from X₂;
      Mₐ is an alkali or alkaline earth metal.
2. The process of embodiment 1, wherein R₁ is C1-C6 alkyl, preferably C1-C3 alkyl, more preferably C1 or C2 alkyl, and wherein R₁' is C1-C6 alkyl, preferably C1-C3 alkyl, more preferably C1 or C2 alkyl.
3. The process of embodiment 1 or 2, wherein R₁ and R₁' are the same, the process comprising
   (i) reacting a compound of formula (I) 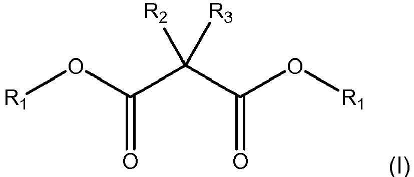 with an Mₐ salt of a compound H₂N-X₁ obtaining an Mₐ salt of formula (II) 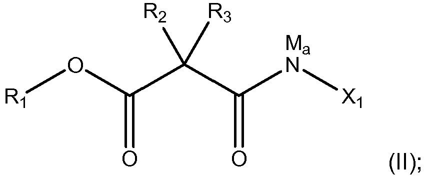
   (ii) converting the Mₐ salt of formula (II) to the compound of formula (IV).
4. The process of embodiment 3, wherein R₁ is methyl or ethyl, preferably wherein R₁ is methyl.
5. The process of any one of embodiments 1 to 4, wherein R₂ and R₃ are C1-C6 alkyl, C1-C6 alkoxy, aryl having from 5 to 10 atoms constituting the aryl structure, or heteroaryl having from 5 to 10 atoms constituting the heteroaryl structure, and wherein R₂ and R₃ are the same or different.
6. The process of any one of embodiments 1 to 5, wherein R₂ and R₃ are C1-C6 alkyl, C1-C6 alkoxy, aryl having from 5 to 10 atoms constituting the aryl structure, or heteroaryl having from 5 to 10 atoms constituting the heteroaryl structure, and wherein R₂ and R₃ are the same or different and substituted with one or more of alkyl, preferably C1-C6 alkyl, alkoxy, preferably C1-C6 alkoxy, aryl, preferably having from 5 to 10 atoms constituting the aryl structure, and heteroaryl, preferably having from 5 to 10 atoms constituting the heteroaryl structure.
7. The process of any one of embodiments 1 to 6, wherein R₂ and R₃, together with the C atom at which R₂ and R₃ are attached, form a cycle having from 3 to 6, preferably from 3 to 5, preferably 3 or 4 atoms.
8. The process of any one of embodiments 1 to 7, wherein R₂ and R₃, together with the C atom at which R₂ and R₃ are attached, form a cycle having 3 carbon atoms.
9. The process of any one of embodiments 1 to 8, wherein the compound of formula (I) is 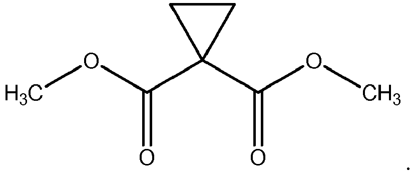
10. The process of any one of embodiments 1 to 9, wherein Mₐ is Mg, Na or K, preferably Na.
11. The process of any one of embodiments 1 to 10, wherein the Mₐ salt of the compound H₂N-X₁ is MₐHN-X₁.
12. The process of any one of embodiments 1 to 11, wherein X₁ is a substituted aromatic residue having from 5 to 10 atoms constituting the aromatic structure or a substituted heteroaromatic residue having from 5 to 10 atoms constituting the heteroaromatic structure.
13. The process of embodiment 12, wherein X₁ is an aromatic residue having from 5 to 10 atoms constituting the aromatic structure substituted with one or more of halogen, preferably F and CI, alkyl, preferably C1-C6 alkyl, alkoxy, preferably C1-C6 alkoxy, aryloxy, and heteroaryloxy, or a heteroaromatic residue having from 5 to 10 atoms constituting the heteroaromatic structure substituted with one or more of halogen, preferably F and CI, alkyl, preferably C1-C6 alkyl, alkoxy, preferably C1-C6 alkoxy, aryloxy, and heteroaryloxy.
14. The process of any one of embodiments 1 to 13, wherein X₁ is a monosubstituted aromatic residue having from 5 to 10 atoms constituting the aromatic structure or a monosubstituted heteroaromatic residue having from 5 to 10 atoms constituting the heteroaromatic structure, preferably a para- or ortho-monosubstituted aromatic residue having from 5 to 10 atoms constituting the aromatic structure or a para- or ortho-monosubstituted heteroaromatic residue having from 5 to 10 atoms constituting the heteroaromatic structure, wherein the substituent is preferably halogen, more preferably F or Cl, more preferably F.
15. The process of any one of embodiments 1 to 14, wherein the compound H₂N-X₁ is a 4-haloaniline, preferably 4-fluoroaniline.
16. The process of any one of embodiments 1 to 15, wherein the compound of formula (I) is 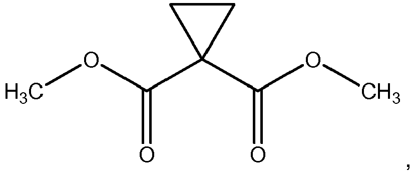 the compound H₂N-X₁ is 4-fluoroaniline and the Mₐ salt of formula (II) is 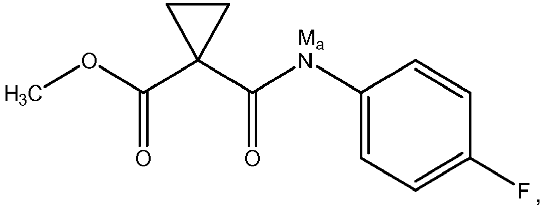 preferably 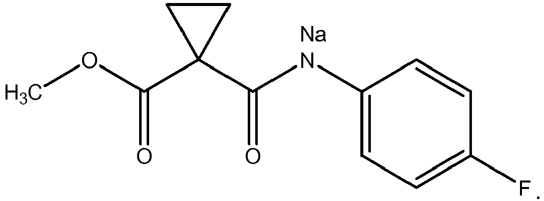
17. The process of any one of embodiments 1 to 16, wherein (i) comprises reacting the compound of formula (I) with the Mₐ salt of a compound H₂N-X₁ in a reaction solvent.
18. The process of embodiment 17, wherein the reaction solvent comprises one or more aprotic solvents, preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran.
19. The process of embodiment 17 or 18, wherein the reaction solvent comprises, preferably is, toluene.
20. The process of any one of embodiments 1 to 19, wherein (i) comprises providing the compound of formula (I), the compound H₂N-X₁, and a compound MₐORₐ wherein Rₐ is alkyl.
21. The process of embodiment 20, wherein Rₐ is R₁.or R₁', preferably R₁.
22. The process of embodiment 20 or 21, wherein Rₐ is C1-C6 alkyl, preferably C1-C4 alkyl, more preferably methyl, ethyl, or tert-butyl, more preferably methyl or ethyl, more preferably methyl.
23. The process of any one of embodiments 20 to 22, wherein the compound of formula (I) is provided in a solvent for the compound of formula (I), wherein the solvent for the compound of formula (I) preferably comprises one or more aprotic solvents, preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran.
24. The process of embodiment 23, wherein the solvent for the compound of formula (I) comprises, preferably is, the reaction solvent, preferably toluene.
25. The process of any of one embodiments 20 to 24, wherein the compound H₂N-X₁ is provided in a solvent for the compound H₂N-X₁, wherein the solvent for the compound H₂N-X₁ preferably comprises one or more aprotic solvents, preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran.
26. The process of embodiment 25, wherein the solvent for the compound H₂N-X₁ comprises, preferably is, the reaction solvent, preferably toluene.
27. The process of any one of embodiments 20 to 26, wherein the compound MₐORₐ is provided in a solvent for the compound MₐORₐ which preferably comprises, more preferably is, HORₐ, more preferably methanol.
28. The process of any one of embodiments 20 to 27, wherein (i) comprises reacting the compound H₂N-X₁ with the compound MₐORₐ, obtaining the Mₐ salt of the compound H₂N-X₁.
29. The process of embodiment 28, wherein the solvent for the compound H₂N-X₁ and a compound HORₐ obtained from reacting the compound H₂N-X₁ with the compound MₐORₐ form an azeotropic mixture, and wherein reacting the compound H₂N-X₁ with the compound MₐORₐ, is carried out at a temperature allowing for distilling off the azeotropic mixture.
30. The process of embodiment 29, wherein reacting the compound H₂N-X₁ with the compound MₐORₐ comprises distilling off the azeotropic mixture.
31. The process of embodiment 29 or 30, wherein the azeotropic mixture comprises toluene and methanol.
32. The process of any one of embodiments 29 to 31, wherein the compound HORₐ forms an azeotropic mixture with the solvent for the compound of formula (I), and wherein reacting the compound of formula (I) with the Mₐ salt of the compound H₂N-X₁ according to (i) is carried out at a temperature allowing for distilling off the azeotropic mixture.
33. The process of embodiment 32, wherein reacting the compound of formula (I) with the Mₐ salt of the compound H₂N-X₁ according to (i) comprises distilling off the azeotropic mixture.
34. The process of embodiment 32 or 33, wherein the azeotropic mixture comprises toluene and methanol.
35. The process of any one of embodiments 20 to 34, wherein prior to reacting according to (i), the molar ratio of the compound of formula (I) provided according to (i) relative to the compound H₂N-X₁ provided according to (i) is in the range of from 2:1 to 1:1, preferably in the range of from 1.5:1 to 1:1, more preferably in the range of from 1.1:1 to 1:1.
36. The process of any one of embodiments 20 to 35, wherein prior to reacting according to (i), the molar ratio of the compound MₐORₐ provided according to (i) relative to the compound H₂N-X₁ provided according to (i) is in the range of from 2:1 to 1:1, preferably in the range of from 1.5:1 to 1:1, more preferably in the range of from 1.1:1 to 1:1.
37. The process of any one of embodiments 20 to 36, wherein (i) further comprises separating the compound HORₐ, at least partially formed during reacting according to (i), from the Mₐ salt of the compound H₂N-X₁, preferably by distillation or co-distillation.
38. The process of any one of embodiments 1 to 37, wherein the Mₐ salt of formula (II) and/or of formula (II'), preferably the Mₐ salt of formula (II), is obtained from (i) as a solid.
39. The process of embodiment 38, wherein the solid Mₐ salt of formula (II) and/or of formula (II'), preferably the solid Mₐ salt of formula (II), is separated from the reaction mixture.
40. The process of any one of embodiments 1 to 39, wherein converting the Mₐ salt of formula (II) and/or of formula (II'), preferably of formula (II), to the compound of formula (IV) according to (ii) comprises
   (ii.a.1) converting the Mₐ salt of formula (II) to a compound of formula (III) 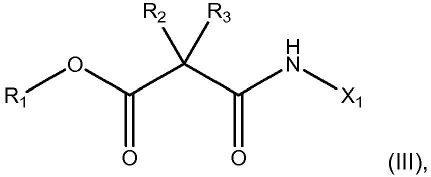 or the Mₐ salt of formula (II') to a compound of formula (III') 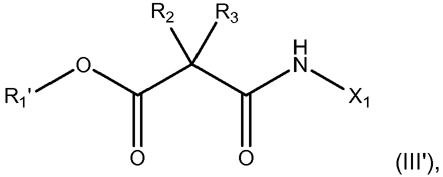 or the mixture of the Mₐ salt of formula (II) and the Mₐ salt of formula (II') to a mixture of the compounds of formula (III) and formula (III').
41. The process of embodiment 40, wherein converting according to (ii.a.1) comprises
   (ii.a.1.1) protonating the amide nitrogen in the compound of formula (II) and/or formula (II'), preferably of formula (II), preferably by bringing the compound of formula (II) and/or formula (II'), preferably of formula (II), in contact with an acid.
42. The process of embodiment 41, wherein the acid comprises, preferably is, one or more of hydrochloric acid, sulphuric acid, formic acid, and acetic acid, preferably hydrochloric acid.
43. The process of embodiment 41 or 42, wherein protonating, preferably protonating by bringing in contact with the acid, is carried out at a temperature in the range of from -10 °C to 50 °C, preferably from 0 to 40 °C, more preferably from 10 to 30 °C.
44. The process of any one of embodiments 41 to 43, wherein prior to protonating according to (ii.a.1.1), the molar ratio of the one or more acids, relative to the compound of formula (II) and/or formula (II'), preferably of formula (II), is in the range of from 1.0:1 to 1.5:1, preferably of from 1.0:1 to 1.1:1.
45. The process of any one of embodiments 41 to 44, wherein the amount of the acid, brought into contact with 1 mol of the compound of formula (II) and/or formula (II'), preferably of formula (II), is in the range of from 0.01 to 0.2 mol per second, preferably of from 0.05 to 0.1 mol per second.
46. The process of any one of embodiment 41 to 45, wherein the acid, brought into contact with the compound of formula (II) and/or formula (II'), preferably of formula (II), is comprised in an aqueous system, obtaining the compound of formula (III) and/or of formula (III'), preferably of formula (III).
47. The process of embodiment 46, wherein at least 5 weight-%, preferably at least 50 weight-%, more preferably at least 90 weight-%, more preferably at least 99 weight-% of the aqueous system consist of water, wherein more preferably, the aqueous system is water.
48. The process of embodiment 46 or 47, wherein the obtained compound of formula (III) and/or of formula (III'), preferably of formula (III), is not comprised in the aqueous system.
49. The process of any one of embodiments 46 to 48, wherein (ii.a.1) further comprises
   (ii.a.1.2) separating the obtained compound of formula (III) and/or of formula (III'), preferably of formula (III), from the aqueous system.
50. The process of embodiment 49, wherein the separating comprises extracting the compound of formula (III) and/or of formula (III'), preferably of formula (III), preferably with an extracting agent including one or more of dichloromethane, isopentane, cyclohexane, heptane, and toluene, more preferably one or more of dichloromethane and isopentane.
51. The process of embodiment 49 or 50, wherein the separating comprises extracting the compound of formula (III) and/or of formula (III'), preferably of formula (III) with isopentane as extracting agent.
52. The process of embodiment 51, wherein the separating comprises extracting the compound of formula (III) and/or of formula (III'), preferably of formula (III), with dichloromethane as extracting agent and separating the extracting agent, preferably by evaporation, obtaining a residue comprising the compound of formula (III) and/or of formula (III'), preferably of formula (III), and extracting the compound of formula (III) and/or of formula (III'), preferably of formula (III) from the residue with isopentane as extracting agent.
53. The process of any one of embodiments 50 to 52, further comprising separating the extracting agent, preferably the isopentane, from the compound of formula (III) and/or of formula (III'), preferably of formula (III), said separating the extracting agent preferably comprising, more preferably consisting of, evaporating the extracting agent.
54. The process of embodiment 53, wherein from separating, preferably evaporating, the extracting agent, the compound of formula (III) and/or of formula (III'), preferably of formula (III), is obtained as a solid, preferably as a crystalline solid.
55. The process of any one of embodiments 41 to 54, wherein after (ii.a.1.1) and prior to (ii.a.1.2), the compound of formula (III) and/or of formula (III'), preferably of formula (III), has a degree of purity in the range of from 75:25 to 95:5, preferably from 85:15 to 95:1, more preferably from 92:8 to 95:5, more preferably from 93:7 to 95:5, wherein the degree of purity is defined as the molar ratio of the compound of formula (III) and/or of formula (III'), preferably of formula (III), relative to the respective symmetric malonic acid diamide of formula (S) 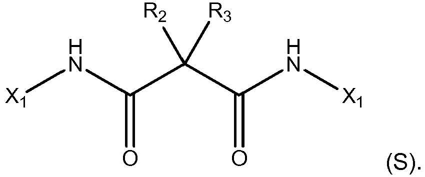
56. The process of any one of embodiments 49 to 55, wherein after (ii.a.2), the compound of formula (III) and/or of formula (III'), preferably of formula (III), has a degree of purity in the range of from 98:2 to 100:0, preferably from 99:1 to 100:0, more preferably from 99.5:0.5 to 100:0, more preferably from 99.9:0.1 to 100:0, wherein the degree of purity is defined as the molar ratio of the compound of formula (III) and/or of formula (III'), preferably of formula (III), relative to the respective symmetric malonic acid diamide of formula (S) 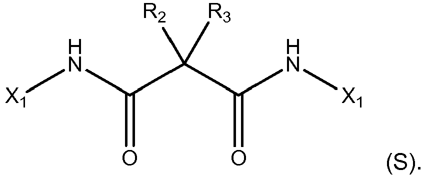
57. The process of any one of embodiments 40 to 56, wherein the amide bond is formed by
   (ii.a.2) reacting the compound of formula (III) and/or of formula (III'), preferably of formula (III), with an M_{b} salt of the compound H₂N-X₂;
   wherein M_{b} is an alkali or alkaline earth metal.
58. The process of embodiment 57, wherein M_{b} is Mg, Na or K, preferably Na.
59. The process of embodiment 57 or 58, wherein the M_{b} salt of the compound H₂N-X₂ is M_{b}HN-X₂.
60. The process of any one of embodiments 57 to 59, wherein X₂ is a substituted aromatic or heteroaromatic residue having from 5 to 10 atoms constituting the aromatic or heteroaromatic structure, preferably having from 6 to 10 atoms constituting the aromatic or heteroaromatic structure.
61. The process of embodiment 60, wherein X₂ is a substituted, preferably a mono-, di- or tri-substituted, aromatic or heteroaromatic residue having from 6 to 10 atoms, preferably 6 atoms, constituting the aromatic or heteroaromatic structure.
62. The process of embodiment 61, wherein X₂ is mono-, di- or tri-substituted phenyl.
63. The process of any one of embodiments 60 to 62, wherein X₂ is an aromatic or heteroaromatic residue having from 5 to 10 atoms constituting the aromatic or heteroaromatic structure, preferably having from 6 to 10 atoms constituting the aromatic or heteroaromatic structure, substituted with one or more of halogen, alkyl, preferably C1-C6 alkyl, alkoxy, preferably C1-C6 alkoxy, alkylthioyl, preferably C1-C6 alkylthioyl, and optionally substituted aryloxy or heteroaryloxy preferably having from 5 to 10 atoms constituting the aromatic or heteroaromatic structure.
64. The process of any one of embodiments 60 to 63, wherein X₂ is an aromatic or heteroaromatic residue having from 5 to 10 atoms constituting the aromatic or heteroaromatic structure, preferably having from 6 to 10 atoms constituting the aromatic or heteroaromatic structure, mono-substituted with substituted aryloxy or heteroaryloxy preferably having from 5 to 10 atoms, more preferably having from 6 to 10 atoms, constituting the aromatic or heteroaromatic structure.
65. The process of embodiment 64, wherein the substituted aryloxy or heteroaryloxy is substituted with one or more of halogen, preferably F and Cl, alkyl, preferably C1-C6 alkyl, alkoxy, preferably C1-C6 alkoxy, alkylthioyl, preferably C1-C6 alkylthioyl, and optionally substituted aryloxy or heteroaryloxy preferably having from 5 to 10 atoms constituting the aromatic or heteroaromatic structure.
66. The process of embodiment 65, wherein the substituted aryloxy or heteroaryloxy is 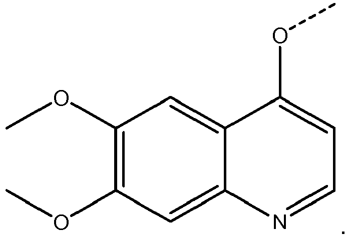
67. The process of any one of embodiments 60 to 66, wherein the compound H₂N-X₂ is 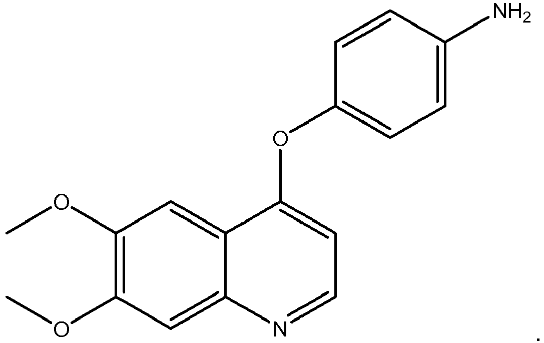
68. The process of any one of embodiments 60 to 67, wherein the compound of formula (I) is 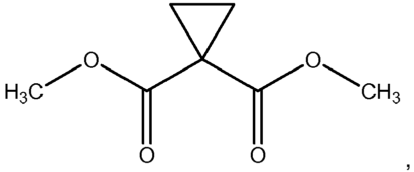 the compound H₂N-X₁ is 4-fluoroaniline, the Mₐ salt of formula (II) is 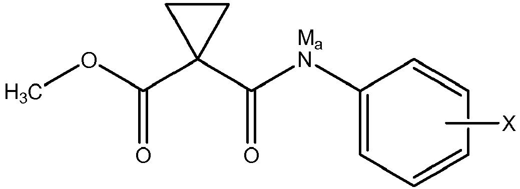 wherein X is halogen, preferably F and Cl, alkyl, preferably C1-C6 alkyl, alkoxy, preferably C1-C6 alkoxy, aryloxy, or heteroaryloxy, the compound of formula (II) is 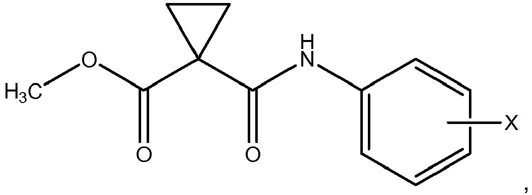 the compound H₂N-X₂ is 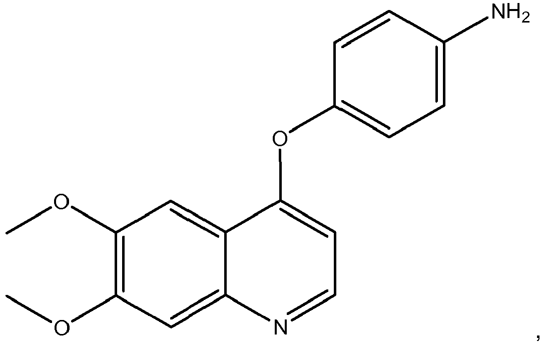 and the compound of formula (IV) is 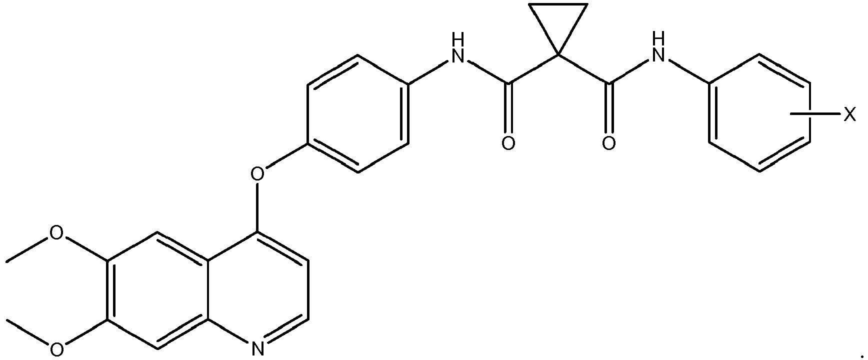
69. The process of embodiment 68, wherein the compound of formula (II) is 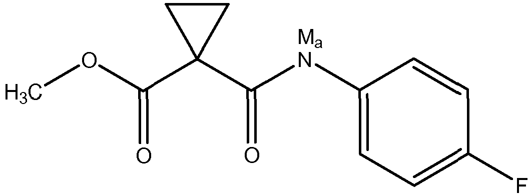 and the compound of formula (IV) is 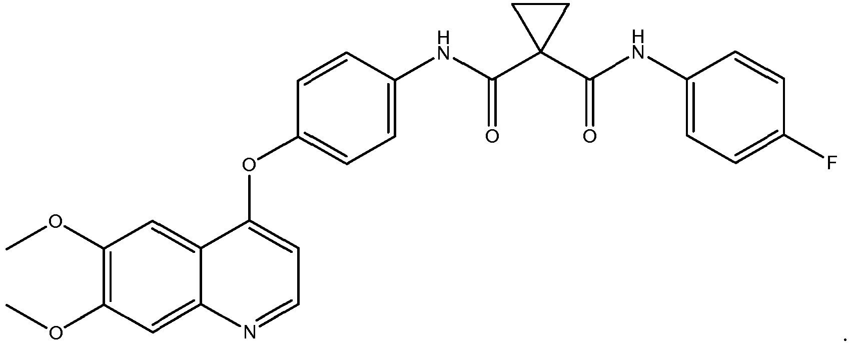
70. The process of any one of embodiments 60 to 63, wherein X₂ is 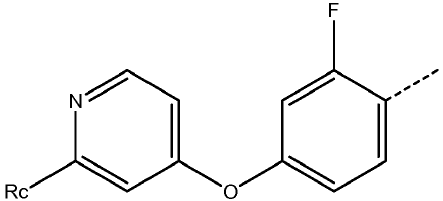 and wherein Rc is halogen, azide, nitro, phtalimido, hydroxycarbonyl, alkoxycarbonyl, cyano, carbonylamino, substituted carbamoyl-N-alkyl-amino, substituted carbamoyl-N-alkyl-amino, or N-acylamino.
71. The process of embodiment 70, wherein the compound of formula (IV) is 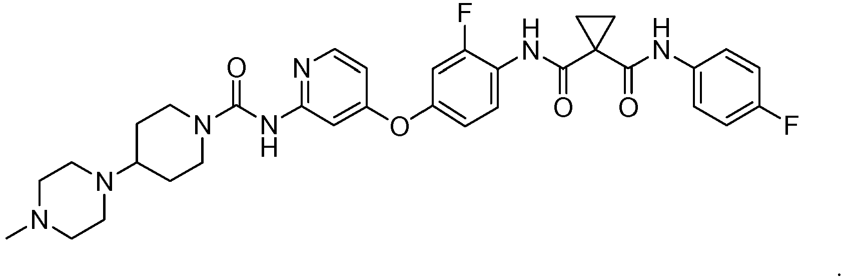
72. The process of any one of embodiments 60 to 63, wherein X₂ is 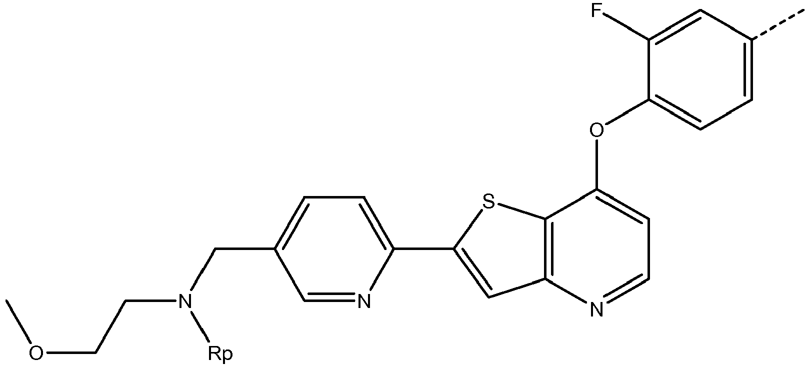 wherein Rp is hydroxyl, alkoxy, aryloxy or a nitrogen protecting group, preferably allyl, Boc, Troc, or Trityl.
73. The process of embodiment 72, wherein the compound of formula (IV) is 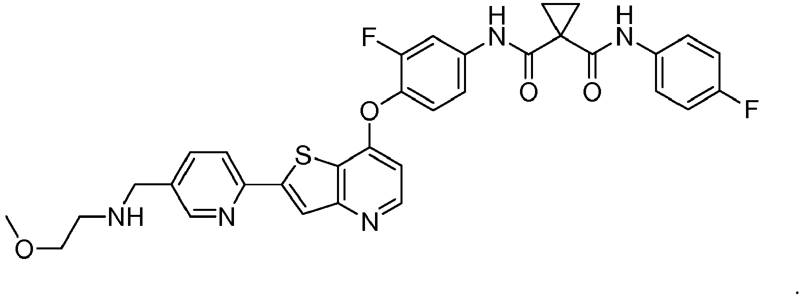
74. The process of any one of embodiments 60 to 63, wherein the compound of formula (IV) is 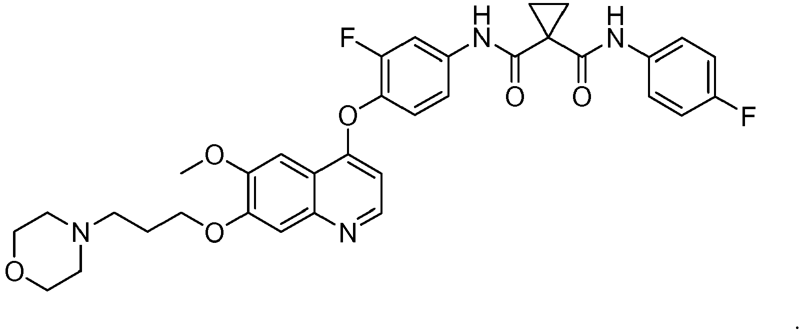
75. The process of any one of embodiments 60 to 63, wherein X₂ is an aromatic or heteroaromatic residue having from 5 to 10 atoms constituting the aromatic or heteroaromatic structure, preferably having from 6 to 10 atoms constituting the aromatic or heteroaromatic structure, tri-substituted with one or more of halogen, and substituted aryloxy or heteroaryloxy preferably having from 5 to 10 atoms constituting the aromatic or heteroaromatic structure, more preferably having from 6 to 10 atoms constituting the aromatic or heteroaromatic structure.
76. The process of embodiment 75, wherein the compound of formula (IV) is 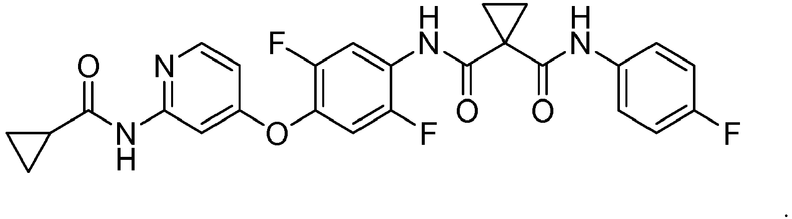
77. The process of any one of embodiments 57 to 76, wherein (ii.a.2) comprises reacting the compound of formula (III) and/or of formula (III'), preferably of formula (III), with the M_{b} salt of a compound H₂N-X₂ in a reaction solvent.
78. The process of embodiment 77, wherein the reaction solvent comprises one or more aprotic solvents, preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran.
79. The process of embodiment 77 or 78, wherein the reaction solvent comprises, preferably is, toluene.
80. The process of any one of embodiments 57 to 79, wherein (ii.a.2) comprises providing the compound of formula (III) and/or of formula (III'), preferably of formula (III), the compound H₂N-X_{b}, and a compound M_{b}OR_{b} wherein R_{b} is alkyl.
81. The process of embodiment 80, wherein R_{b} is C1-C6 alkyl, preferably C1-C4 alkyl, more preferably methyl, ethyl, or tert-butyl, more preferably methyl or ethyl, more preferably methyl.
82. The process of embodiment 80 or 81, wherein the compound of formula (III) and/or of formula (III'), preferably of formula (III), is provided in a solvent for the compound of formula (III) and/or of formula (III'), preferably of formula (III), wherein the solvent for compound of formula (III) and/or of formula (III'), preferably of formula (III), preferably comprises one or more aprotic solvents, preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran.
83. The process of embodiment 82, wherein the solvent for the compound of formula (III) and/or of formula (III'), preferably of formula (III), comprises, preferably is, toluene.
84. The process of any of one embodiments 80 to 83, wherein the compound H₂N-X₂ is provided in a solvent for the compound H₂N-X₂, wherein the solvent for the compound H₂N-X₂ preferably comprises one or more aprotic solvents, preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran.
85. The process of embodiment 84, wherein the solvent for the compound H₂N-X₂ comprises, preferably is, toluene.
86. The process of any one of embodiments 80 to 85, wherein the compound M_{b}OR_{b} is provided in a solvent for the compound M_{b}OR_{b}, wherein the solvent for the compound M_{b}OR_{b} preferably comprises one or more aprotic solvents, preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran.
87. The process of embodiment 86, wherein the solvent for the compound M_{b}OR_{b} comprises, preferably is, toluene.
88. The process of any one of embodiments 80 to 87, wherein (ii.a.2) comprises reacting the compound M_{b}OR_{b} with the compound H₂N-X₂, obtaining the M_{b} salt of the compound H₂N-X₂; and adding the M_{b} salt of H₂N-X₂ to the compound of formula (III) and/or of formula (III'), preferably of formula (III).
89. The process of any one of embodiments 80 to 87, wherein (ii.a.2) comprises reacting the compound M_{b}OR_{b} with a mixture of the compound H₂N-X₂ and the compound of formula (III) and/or of formula (III'), preferably of formula (III).
90. The process of embodiment 88 or 89, wherein (ii.a.2) further comprises separating the compound HORₐ, at least partially formed during reacting the compound M_{b}OR_{b} with the compound H₂N-X₂, from the Mₐ salt of the compound H₂N-X₁, preferably by distillation or co-distillation.
91. The process of any one of embodiments 80 to 90, wherein the molar ratio of the compound of formula (III) and/or of formula (III'), preferably of formula (III), provided according to (ii.a.2), relative to the compound H₂N-X₂ provided according to (ii.a.2) is in the range of from 1.5:1 to 1:1, preferably from 1.2:1 to 1:1, more preferably from 1.1:1 to 1:1.
92. The process of any one of embodiments 80 to 91, wherein the molar ratio of the compound M_{b}OR_{b} provided according to (ii.a.2) relative to the compound H₂N-X₂ provided according to (ii.a.2) is in the range of from 2.5:1 to 2:1, preferably from 2.2:1 to 2:1, more preferably from 2.1:1 to 2:1.
93. The process of any one of embodiments 77 to 92, wherein (i), (ii.a.1) and (ii.a.2) are carried out in the same solvent, preferably in toluene.
94. The process of any one of embodiments 57 to 93, wherein a compound HOR₁ or a compound HOR₁' or a mixture of compounds HOR₁ and HOR₁', obtained from reacting the compound of formula (III) and/or of formula (III'), preferably of formula (III), with the M_{b} salt of the compound H₂N-X₂, forms an azeotropic mixture with the solvent for the compound of formula (III) and/or of formula (III'), preferably of formula (III), and wherein reacting the compound of formula (III) and/or of formula (III'), preferably of formula (III), with the M_{b} salt of the compound H₂N-X₂ according to (ii.2) is carried out at a temperature allowing for distilling off the azeotropic mixture.
95. The process of embodiment 94, wherein reacting the compound of formula (III) and/or of formula (III'), preferably of formula (III), with the M_{b} salt of the compound H₂N-X₂ according to (ii.a.2) comprises distilling off the azeotropic mixture.
96. The process of any one of embodiments 57 to 95, further comprising
   (ii.a.3.1) adding an organic solvent to the reaction mixture obtained from (ii.a.2);
   (ii.a.3.2) adding an acid to the mixture obtained from (ii.a.3.1), obtaining the compound of formula (IV) dissolved in the organic solvent according to (ii.a.3.1)
   (ii.a.3.3) separating the organic solvent from the compound of formula (IV);
   (ii.a.3.4) extracting the compound of formula (IV) with an extracting agent.
97. The process of embodiment 96, wherein the organic solvent according to (ii.a.3.1) comprises one or more of ethyl acetate, ispropyl acetate, butyl acetate, methyl tert-butyl ether, and dichloromethane, preferably ethyl acetate.
98. The process of embodiment 96 or 97, wherein the acid according to (ii.a.3.2) comprises ammonium chloride, preferably aqueous ammonium chloride, more preferably saturated aqueous ammonium chloride, wherein more preferably the acid is saturated aqueous ammonium chloride.
99. The process of any one of embodiments 96 to 98, wherein separating the organic solvent according to (ii.a.3.3) comprises evaporating the organic solvent.
100. The process of any one of embodiments 96 to 99, wherein the extracting agent according to (ii.a.3.4) comprises, preferably is, methyl tert-butyl ether.
101. The process of any one of embodiments 1 to 39, wherein the amide bond is formed by
   (ii.b.1) reacting the compound of formula (II) and/or of formula (II') with an M_{b} salt of a compound H₂N-X₂;
   wherein M_{b} is an alkali or alkaline earth metal.
102. The process of embodiment 101, wherein M_{b} is Mg, Na or K, preferably Na.
103. The process of embodiment 101 or 102, wherein the M_{b} salt of the compound H₂N-X₂ is M_{b}HN-X₂.
104. The process of any one of embodiments 101 to 103, wherein X₂ is a substituted aromatic or heteroaromatic residue having from 5 to 10 atoms constituting the aromatic or heteroaromatic structure, preferably having from 6 to 10 atoms constituting the aromatic or heteroaromatic structure.
105. The process of embodiment 104, wherein X₂ is a substituted, preferably a mono-, di- or tri-substituted, aromatic or heteroaromatic residue having from 6 to 10 atoms, preferably 6 atoms, constituting the aromatic or heteroaromatic structure.
106. The process of embodiment 105, wherein X₂ is mono-, di- or tri-substituted phenyl.
107. The process of any one of embodiments 104 to 106, wherein X₂ is an aromatic or heteroaromatic residue having from 5 to 10 atoms constituting the aromatic or heteroaromatic structure, preferably having from 6 to 10 atoms constituting the aromatic or heteroaromatic structure, substituted with one or more of halogen, alkyl, preferably C1-C6 alkyl, alkoxy, preferably C1-C6 alkoxy, alkylthioyl, preferably C1-C6 alkylthioyl, and optionally substituted aryloxy or heteroaryloxy preferably having from 5 to 10 atoms constituting the aromatic or heteroaromatic structure.
108. The process of any one of embodiments 104 to 107, wherein X₂ is an aromatic or heteroaromatic residue having from 5 to 10 atoms constituting the aromatic or heteroaromatic structure, preferably having from 6 to 10 atoms constituting the aromatic or heteroaromatic structure, mono-substituted with substituted aryloxy or heteroaryloxy preferably having from 5 to 10 atoms, more preferably having from 6 to 10 atoms, constituting the aromatic or heteroaromatic structure.
109. The process of embodiment 108, wherein the substituted aryloxy or heteroaryloxy is substituted with one or more of halogen, preferably F and Cl, alkyl, preferably C1-C6 alkyl, alkoxy, preferably C1-C6 alkoxy, alkylthioyl, preferably C1-C6 alkylthioyl, and optionally substituted aryloxy or heteroaryloxy preferably having from 5 to 10 atoms constituting the aromatic or heteroaromatic structure.
110. The process of embodiment 109, wherein the substituted aryloxy or heteroaryloxy is 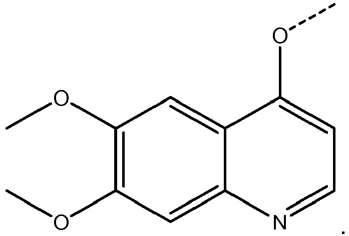
111. The process of any one of embodiments 104 to 110, wherein the compound H₂N-X₂ is 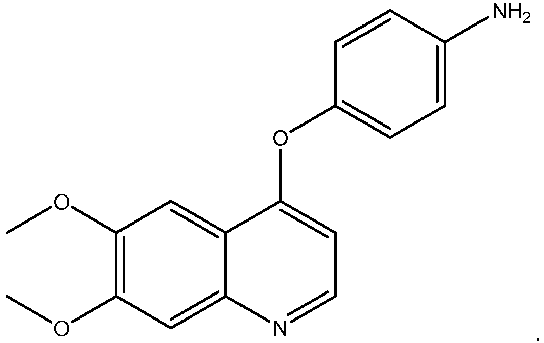
112. The process of any one of embodiments 104 to 111, wherein the compound of formula (I) is 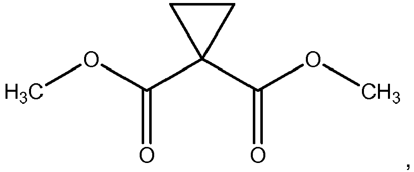 the compound H₂N-X₁ is 4-fluoroaniline, the Mₐ salt of formula (II) is 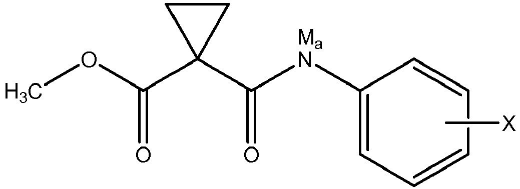 wherein X is halogen, preferably F and Cl, alkyl, preferably C1-C6 alkyl, alkoxy, preferably C1-C6 alkoxy, aryloxy, or heteroaryloxy, the compound of formula (II) is 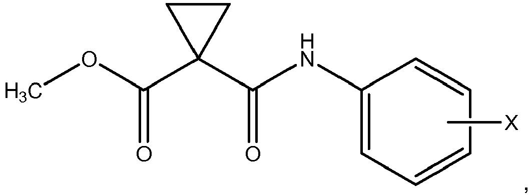 the compound H₂N-X₂ is 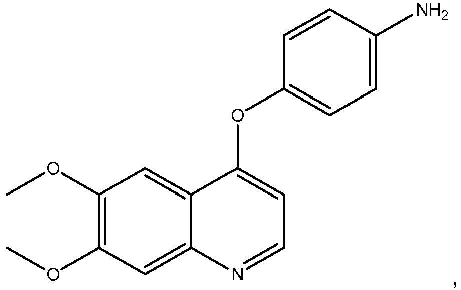 and the compound of formula (IV) is 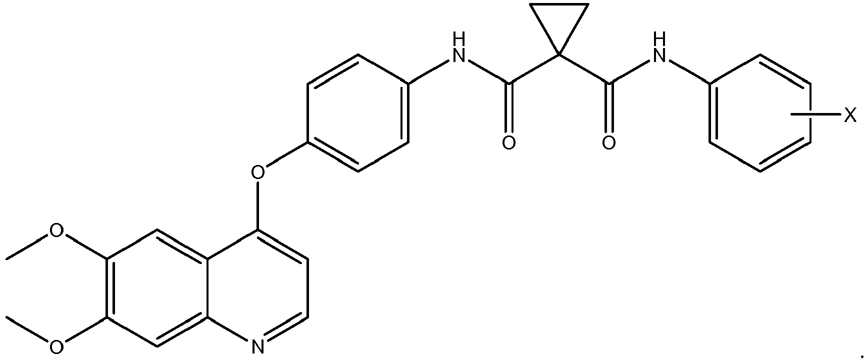
113. The process of embodiment 112, wherein the compound of formula (II) is 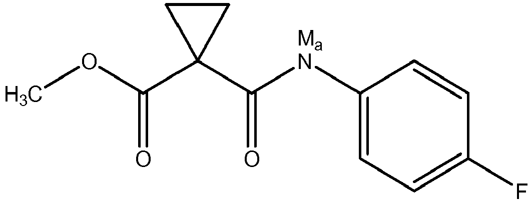 and the compound of formula (IV) is 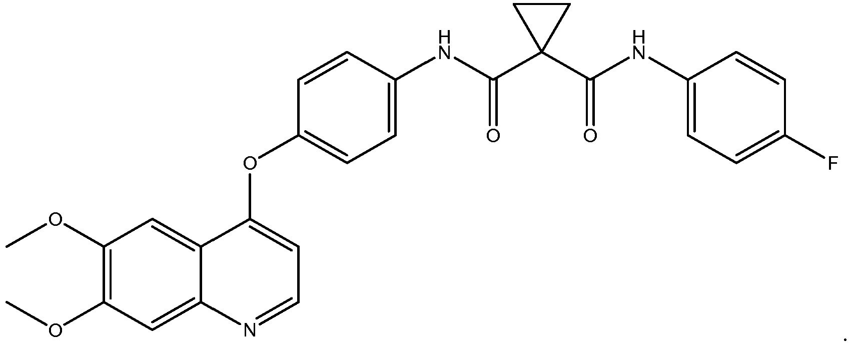
114. The process of any one of embodiments 104 to 107, wherein X₂ is 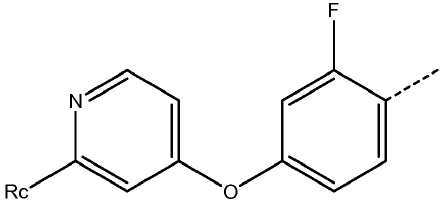 and wherein Rc is halogen, azide, nitro, phtalimido, hydroxycarbonyl, alkoxycarbonyl, cyano, carbonylamino, substituted carbamoyl-N-alkyl-amino, substituted carbamoyl-N-alkyl-amino, or N-acylamino.
115. The process of embodiment 114, wherein the compound of formula (IV) is 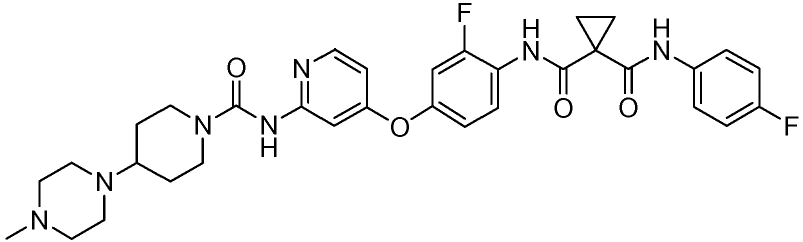
116. The process of any one of embodiments 104 to 107, wherein X₂ is 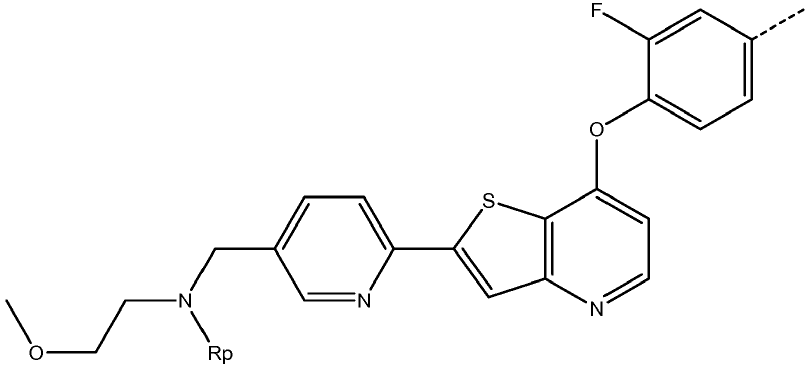 wherein Rp is hydroxyl, alkoxy, aryloxy or a nitrogen protecting group, preferably allyl, Boc, Troc, or Trityl.
117. The process of embodiment 116, wherein the compound of formula (IV) is 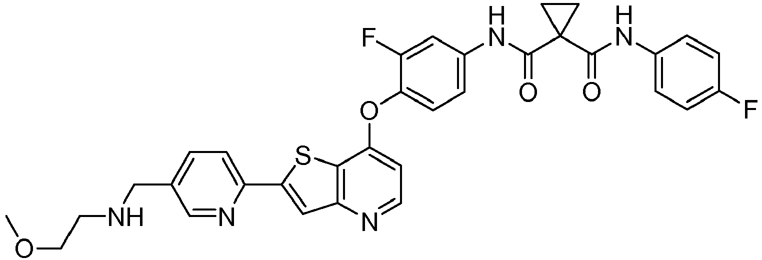
118. The process of any one of embodiments 104 to 107, wherein the compound of formula (IV) is 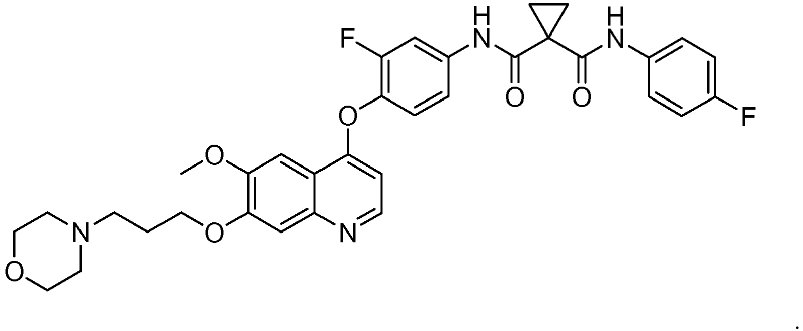
119. The process of any one of embodiments 104 to 107, wherein X₂ is an aromatic or heteroaromatic residue having from 5 to 10 atoms constituting the aromatic or heteroaromatic structure, preferably having from 6 to 10 atoms constituting the aromatic or heteroaromatic structure, tri-substituted with one or more of halogen, and substituted aryloxy or heteroaryloxy preferably having from 5 to 10 atoms constituting the aromatic or heteroaromatic structure, more preferably having from 6 to 10 atoms constituting the aromatic or heteroaromatic structure.
120. The process of embodiment 119, wherein the compound of formula (IV) is 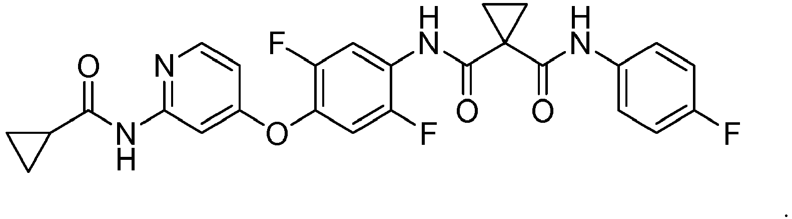
121. The process of any one of embodiments 101 to 120, wherein (ii.b.1) comprises reacting the compound of formula (II) and/or of formula (II'), preferably of formula (II), with the M_{b} salt of a compound H₂N-X₂ in a reaction solvent.
122. The process of embodiment 121, wherein the reaction solvent comprises one or more aprotic solvents, preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran.
123. The process of embodiment 121 or 122, wherein the reaction solvent comprises, preferably is, toluene.
124. The process of any one of embodiments 101 to 123, wherein (ii.b.1) comprises providing the compound of formula (II) and/or of formula (II'), preferably of formula (II), the compound H₂N-X_{b}, and a compound M_{b}OR_{b} wherein R_{b} is alkyl.
125. The process of embodiment 124, wherein R_{b} is C1-C6 alkyl, preferably C1-C4 alkyl, more preferably methyl, ethyl, or tert-butyl, more preferably methyl or ethyl, more preferably methyl.
126. The process of embodiment 124 or 125, wherein the compound of formula (II) and/or of formula (II'), preferably of formula (II), is provided in a solvent for the compound of formula (II) and/or of formula (II'), preferably of formula (II), wherein the solvent for compound of formula (II) and/or of formula (II'), preferably of formula (II), preferably comprises one or more aprotic solvents, preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran.
127. The process of embodiment 126, wherein the solvent for the compound of formula (II) and/or of formula (II'), preferably of formula (II), comprises, preferably is, toluene.
128. The process of any of one embodiments 124 to 127, wherein the compound H₂N-X₂ is provided in a solvent for the compound H₂N-X₂, wherein the solvent for the compound H₂N-X₂ preferably comprises one or more aprotic solvents, preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran.
129. The process of embodiment 128, wherein the solvent for the compound H₂N-X₂ comprises, preferably is, toluene.
130. The process of any one of embodiments 124 to 129, wherein the compound M_{b}OR_{b} is provided in a solvent for the compound M_{b}OR_{b}, wherein the solvent for the compound M_{b}OR_{b} preferably comprises one or more aprotic solvents, preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran.
131. The process of embodiment 130, wherein the solvent for the compound M_{b}OR_{b} comprises, preferably is, toluene.
132. The process of any one of embodiments 124 to 131, wherein (ii.b.1) comprises reacting the compound M_{b}OR_{b} with the compound H₂N-X₂, obtaining the M_{b} salt of the compound H₂N-X₂; and adding the M_{b} salt of H₂N-X₂ to the compound of formula (II) and/or of formula (II'), preferably of formula (II).
133. The process of any one of embodiments 124 to 131, wherein (ii.b.1) comprises adding the compound H₂N-X₂ to the Mₐ salt of compound of formula (II) and/or of formula (II'), or the mixture of the Mₐ salt of formula (II) and the Mₐ salt of formula (II'), and then adding the compound M_{b}OR_{b} to the reaction mixture.
134. The process of embodiment 133, wherein (ii.b.1) further comprises separating the compound HOR_{b}, at least partially formed during reacting the compound M_{b}OR_{b} with the compound H₂N-X₂, from the reaction mixture, preferably by distillation or co-distillation.
135. The process of any one of embodiments 124 to 134, wherein the molar ratio of the compound of formula (II) and/or of formula (II'), preferably of formula (II), provided according to (ii.b.1), relative to the compound H₂N-X₂ provided according to (ii.b.1) is in the range of from 1.5:1 to 1:1, preferably from 1.2:1 to 1:1, more preferably from 1.1:1 to 1:1.
136. The process of any one of embodiments 124 to 135, wherein the molar ratio of the compound M_{b}OR_{b} provided according to (ii.b.1) relative to the compound H₂N-X₂ provided according to (ii.b.1) is in the range of from 1.5:1 to 1:1, preferably from 1.2:1 to 1:1, more preferably from 1.1:1 to 1:1.
137. The process of any one of embodiments 124 to 136, wherein (i) and (ii.b.1) are carried out in the same solvent, preferably in toluene.
138. The process of any one of embodiments 124 to 137 which is carried out in a one-pot format.
139. The process of any one of embodiments 124 to 138, wherein a compound HOR₁ or a compound HOR₁' or a mixture of compounds HOR₁ and HOR₁', obtained from reacting the compound of formula H₂N-X₂ with the compound M_{b}OR_{b} forms an azeotropic mixture with the solvent for the compound of formula (II) and/or of formula (II'), preferably of formula (II), and wherein amide bond formation according to (ii.b.1) is carried out at a temperature allowing for distilling off the azeotropic mixture.
140. The process of embodiment 139, wherein reacting the compound of formula (II) and/or of formula (II'), preferably of formula (II), with the M_{b} salt of the compound H₂N-X₂ according to (ii.b.1) comprises distilling off the azeotropic mixture.
141. The process of any one of embodiments 101 to 140, further comprising
   (ii.b.2.1) adding an organic solvent to the reaction mixture obtained from (ii.b.1);
   (ii.b.2.2) adding an acid to the mixture obtained from (ii.b.2.1), obtaining the compound of formula (IV) dissolved in the organic solvent according to (ii.b.2.1);
   (ii.b.2.3) separating the organic solvent from the compound of formula (IV);
   (ii.b.2.4) extracting the compound of formula (IV) with an extracting agent.
142. The process of embodiment 141, wherein the organic solvent according to (ii.b.2.1) comprises one or more of ethyl acetate, ispropyl acetate, butyl acetate, methyl tert-butyl ether, and dichloromethane, preferably ethyl acetate.
143. The process of embodiment 141 or 142, wherein the acid according to (ii.b.2.2) comprises ammonium chloride, preferably aqueous ammonium chloride, more preferably saturated aqueous ammonium chloride, wherein more preferably the acid is saturated aqueous ammonium chloride.
144. The process of any one of embodiments 141 to 143, wherein separating the organic solvent according to (ii.b.2.3) comprises evaporating the organic solvent.
145. The process of any one of embodiments 141 to 144, wherein the extracting agent according to (ii.b.2.4) comprises, preferably is, methyl tert-butyl ether.
146. The process of any one of embodiments 1 to 39, wherein the amide bond is formed by
   (ii.c.1) reacting the compound of formula (III) and/or of formula (III'), preferably of formula (III) with a compound H₂N-X₂;
   wherein X₂ is a substituted or unsubstituted alkyl, alkenyl or cycloalkyl residue.
147. The process of any one of embodiments 1 to 39, wherein the amide bond is formed by a process comprising
   (ii.d.1) saponification of a compound of formula (III) and/or of formula (III'), preferably of formula (III).
148. The process of any one of embodiments 1 to 39, wherein the amide bond is formed by a process comprising
   (ii.e.1) saponification of a compound of formula (II) and/or of formula (II'), preferably of formula (II).
149. The process of embodiment 147 or 148, wherein the carboxylate or carbonic acid obtained by saponification of the compound of formula (II) and/or of formula (II'), preferably of formula (II), or obtained by saponification of compound of formula (III) and/or of formula (III'), preferably of formula (III), is formed as an intermediate.
150. The process of embodiment 149, further comprising converting the carboxylate or carbonic acid to an activated carbonic acid derivative capable of forming an amide bond with a primary amine prior to a further reacting with the compound H₂N-X₂.
151. The process of embodiment 150, wherein the activated carbonic acid derivative capable of forming an amide bond with a primary amine is a carbonic acid chloride.
152. The process of any one of embodiments 1 to 151, further comprising
   (iii) preparing a pharmaceutically acceptable salt of the compound of formula (IV).
153. The process of embodiment 152, wherein the salt is a malate, preferably a monomalate, a succinate, preferably a mono succinate, or a phosphate, preferably a monophosphate.
154. A process for preparing an asymmetric malonic acid diamide of formula (IV) 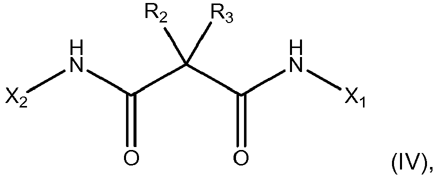 or a pharmaceutically acceptable salt thereof, the process comprising
   (i) reacting a compound of formula (III) 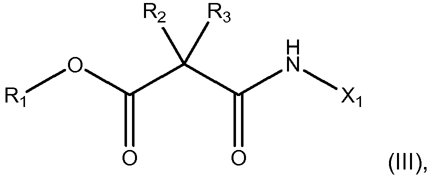 or a compound of formula (III') 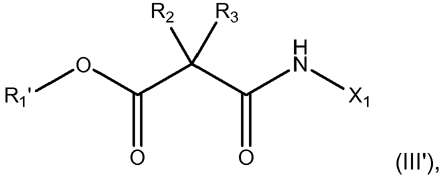 or a mixture of the compound of formula (III) and the compound of formula (III') with an M_{b} salt of a compound H₂N-X₂, obtaining a reaction mixture comprising an M_{b} salt of the compound of formula (IV);
   (ii) converting the M_{b} salt of the compound of formula (IV) to the compound of formula (IV);
      wherein
      R₁ and R₁' are alkyl or aryl, and R₁ and R₁' are the same or different;
      R₂ and R₃ are alkyl, alkoxy, aryl or heteroaryl and R₂ and R₃ are the same or different, or R₂ and R₃, together with the C atom at which R₂ and R₃ are attached, form a cycle;
      X₁ is a substituted aromatic or heteroaromatic residue;
      X₂ is a substituted or unsubstituted alkyl, alkenyl, cycloalkyl, aromatic or heteroaromatic residue wherein X₁ is different from X₂;
      M_{b} is an alkali or alkaline earth metal.
155. The process of embodiment 154, wherein R₁ is C1-C6 alkyl, preferably C1-C3 alkyl, more preferably C1 or C2 alkyl, and wherein R₁' is C1-C6 alkyl, preferably C1-C3 alkyl, more preferably C1 or C2 alkyl.
156. The process of embodiment 155, wherein R₁ is methyl or ethyl, preferably wherein R₁ is methyl.
157. The process of any one of embodiments 154 to 156, wherein R₂ and R₃ are C1-C6 alkyl, C1-C6 alkoxy, aryl having from 5 to 10 atoms constituting the aryl structure, or heteroaryl having from 5 to 10 atoms constituting the heteroaryl structure, and wherein R₂ and R₃ are the same or different.
158. The process of any one of embodiments 154 to 157, wherein R₂ and R₃ are C1-C6 alkyl, C1-C6 alkoxy, aryl having from 5 to 10 atoms constituting the aryl structure, or heteroaryl having from 5 to 10 atoms constituting the heteroaryl structure, and wherein R₂ and R₃ are the same or different and substituted with one or more of alkyl, preferably C1-C6 alkyl, alkoxy, preferably C1-C6 alkoxy, aryl, preferably having from 5 to 10 atoms constituting the aryl structure, and heteroaryl, preferably having from 5 to 10 atoms constituting the heteroaryl structure.
159. The process of any one of embodiments 154 to 158, wherein R₂ and R₃, together with the C atom at which R₂ and R₃ are attached, form a cycle having from 3 to 6, preferably from 3 to 5, preferably 3 or 4 atoms.
160. The process of any one of embodiments 154 to 159, wherein R₂ and R₃, together with the C atom at which R₂ and R₃ are attached, form a cycle having 3 carbon atoms.
161. The process of any one of embodiments 154 to 160, wherein X₁ is a substituted aromatic residue having from 5 to 10 atoms constituting the aromatic structure or a substituted heteroaromatic residue having from 5 to 10 atoms constituting the heteroaromatic structure.
162. The process of embodiment 161, wherein X₁ is an aromatic residue having from 5 to 10 atoms constituting the aromatic structure substituted with one or more of halogen, preferably F and Cl, alkyl, preferably C1-C6 alkyl, alkoxy, preferably C1-C6 alkoxy, aryloxy, and heteroaryloxy, or a heteroaromatic residue having from 5 to 10 atoms constituting the heteroaromatic structure substituted with one or more of halogen, preferably F and Cl, alkyl, preferably C1-C6 alkyl, alkoxy, preferably C1-C6 alkoxy, aryloxy, and heteroaryloxy.
163. The process of any one of embodiments 1 to 162, wherein X₁ is a monosubstituted aromatic residue having from 5 to 10 atoms constituting the aromatic structure or a monosubstituted heteroaromatic residue having from 5 to 10 atoms constituting the heteroaromatic structure, preferably a para- or ortho-monosubstituted aromatic residue having from 5 to 10 atoms constituting the aromatic structure or a para- or ortho-monosubstituted heteroaromatic residue having from 5 to 10 atoms constituting the heteroaromatic structure, wherein the substituent is preferably halogen, more preferably F or Cl, more preferably F.
164. The process of any one of embodiments 154 to 163, wherein M_{b} is Mg, Na or K, preferably Na.
165. The process of any one of embodiments 154 to 164, wherein the M_{b} salt of the compound H₂N-X₂ is M_{b}HN-X₂.
166. The process of any one of embodiments 154 to 165, wherein X₂ is a substituted aromatic or heteroaromatic residue having from 5 to 10 atoms constituting the aromatic or heteroaromatic structure, preferably having from 6 to 10 atoms constituting the aromatic or heteroaromatic structure.
167. The process of embodiment 166, wherein X₂ is a substituted, preferably a mono-, di- or tri-substituted, aromatic or heteroaromatic residue having from 6 to 10 atoms, preferably 6 atoms, constituting the aromatic or heteroaromatic structure.
168. The process of embodiment 167, wherein X₂ is mono-, di- or tri-substituted phenyl.
169. The process of any one of embodiments 166 to 168, wherein X₂ is an aromatic or heteroaromatic residue having from 5 to 10 atoms constituting the aromatic or heteroaromatic structure, preferably having from 6 to 10 atoms constituting the aromatic or heteroaromatic structure, substituted with one or more of halogen, alkyl, preferably C1-C6 alkyl, alkoxy, preferably C1-C6 alkoxy, alkylthioyl, preferably C1-C6 alkylthioyl, and optionally substituted aryloxy or heteroaryloxy preferably having from 5 to 10 atoms constituting the aromatic or heteroaromatic structure.
170. The process of any one of embodiments 166 to 169, wherein X₂ is an aromatic or heteroaromatic residue having from 5 to 10 atoms constituting the aromatic or heteroaromatic structure, preferably having from 6 to 10 atoms constituting the aromatic or heteroaromatic structure, mono-substituted with substituted aryloxy or heteroaryloxy preferably having from 5 to 10 atoms, more preferably having from 6 to 10 atoms, constituting the aromatic or heteroaromatic structure.
171. The process of embodiment 170, wherein the substituted aryloxy or heteroaryloxy is substituted with one or more of halogen, preferably F and Cl, alkyl, preferably C1-C6 alkyl, alkoxy, preferably C1-C6 alkoxy, alkylthioyl, preferably C1-C6 alkylthioyl, and optionally substituted aryloxy or heteroaryloxy preferably having from 5 to 10 atoms constituting the aromatic or heteroaromatic structure.
172. The process of embodiment 170, wherein the substituted aryloxy or heteroaryloxy is 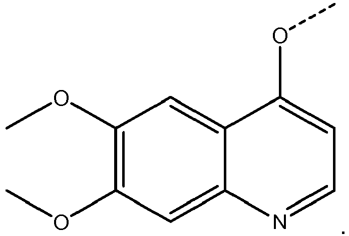
173. The process of any one of embodiments 166 to 172, wherein the compound H₂N-X₂ is 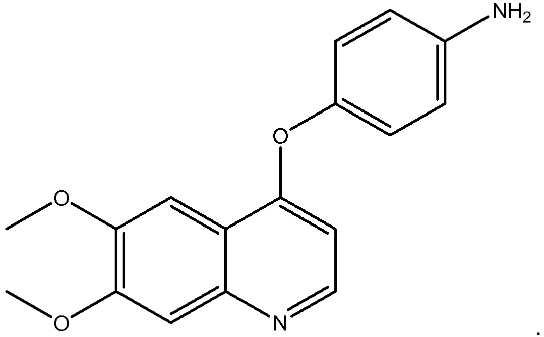
174. The process of any one of embodiments 166 to 173, wherein the compound of formula (I) is 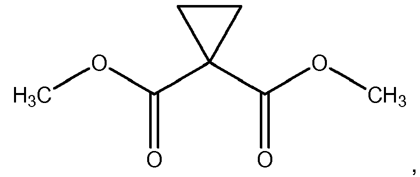 the compound H₂N-X₁ is 4-fluoroaniline, the Mₐ salt of formula (II) is 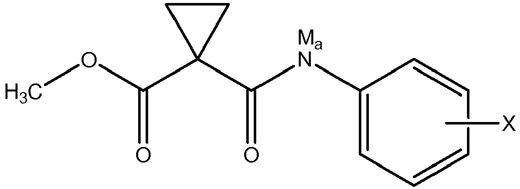 wherein X is halogen, preferably F and Cl, alkyl, preferably C1-C6 alkyl, alkoxy, preferably C1-C6 alkoxy, aryloxy, or heteroaryloxy, the compound of formula (II) is 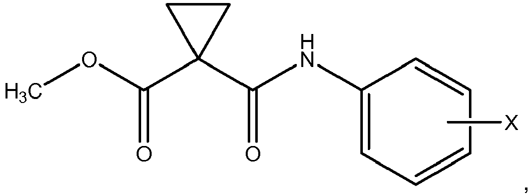 the compound H₂N-X₂ is 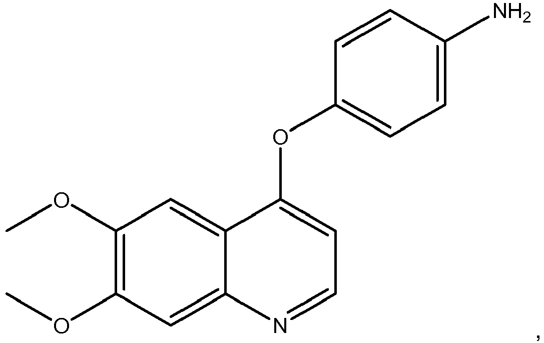 and the compound of formula (IV) is 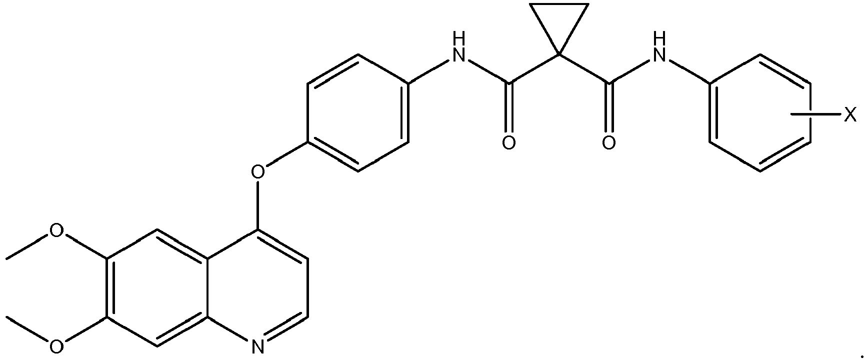
175. The process of embodiment 174, wherein the compound of formula (II) is 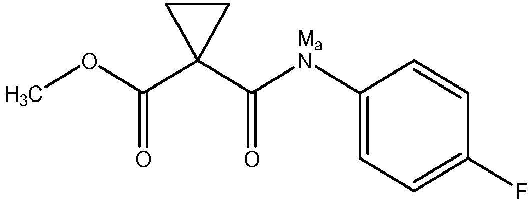 and the compound of formula (IV) is 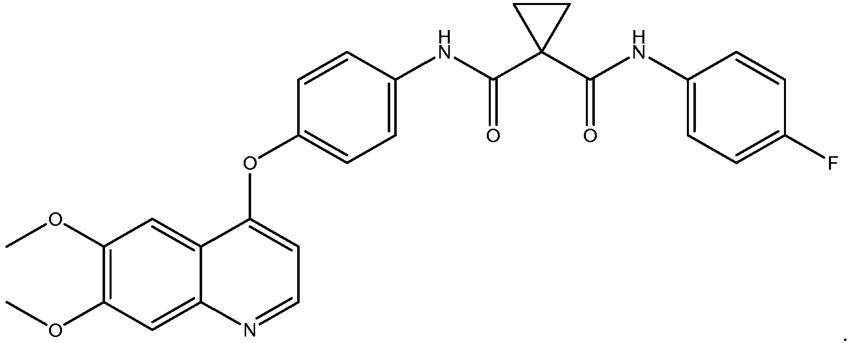
176. The process of any one of embodiments 166 to 169, wherein X₂ is 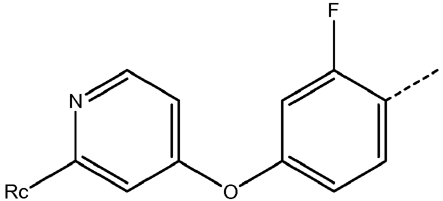 and wherein Rc is halogen, azide, nitro, phtalimido, hydroxycarbonyl, alkoxycarbonyl, cyano, carbonylamino, substituted carbamoyl-N-alkyl-amino, substituted carbamoyl-N-alkyl-amino, or N-acylamino.
177. The process of embodiment 176, wherein the compound of formula (IV) is 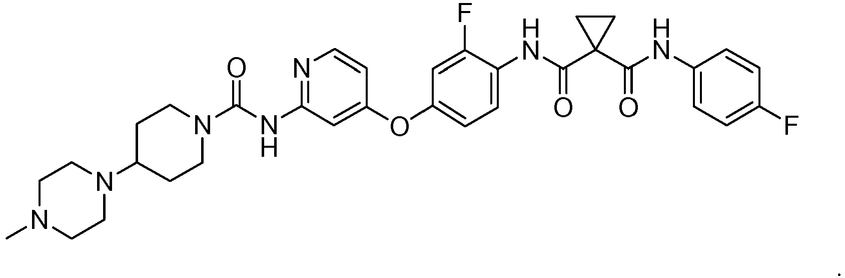
178. The process of any one of embodiments 166 to 169, wherein X₂ is 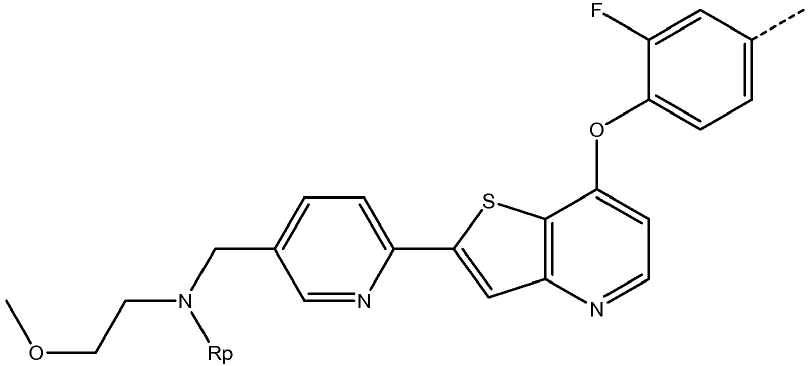 wherein Rp is hydroxyl, alkoxy, aryloxy or a nitrogen protecting group, preferably allyl, Boc, Troc, or Trityl.
179. The process of embodiment 178, wherein the compound of formula (IV) is 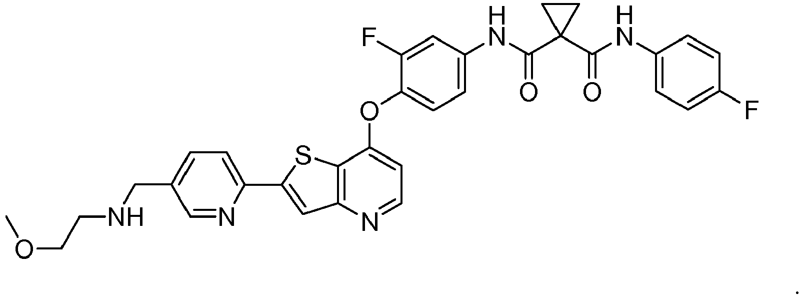
180. The process of any one of embodiments 166 to 169, wherein the compound of formula (IV) is 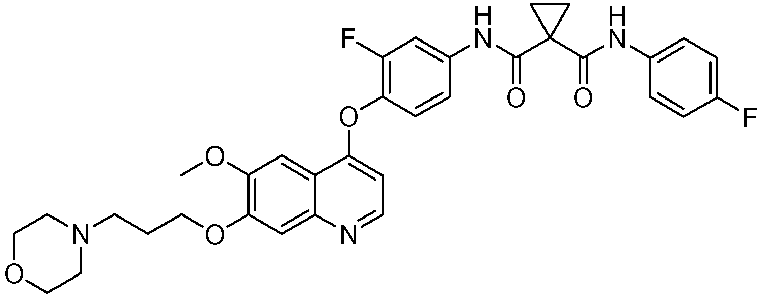
181. The process of any one of embodiments 166 to 169, wherein X₂ is an aromatic or heteroaromatic residue having from 5 to 10 atoms constituting the aromatic or heteroaromatic structure, preferably having from 6 to 10 atoms constituting the aromatic or heteroaromatic structure, tri-substituted with one or more of halogen, and substituted aryloxy or heteroaryloxy preferably having from 5 to 10 atoms constituting the aromatic or heteroaromatic structure, more preferably having from 6 to 10 atoms constituting the aromatic or heteroaromatic structure.
182. The process of embodiment 180, wherein the compound of formula (IV) is 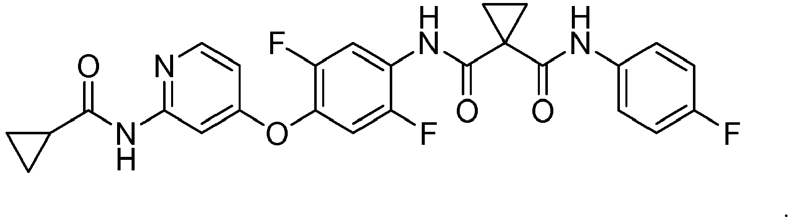
183. The process of any one of embodiments 154 to 182, wherein (i) comprises reacting the compound of formula (III) and/or of formula (III'), preferably of formula (III), with the M_{b} salt of a compound H₂N-X₂ in a reaction solvent.
184. The process of embodiment 183, wherein the reaction solvent comprises one or more aprotic solvents, preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran.
185. The process of embodiment 183 or 184, wherein the reaction solvent comprises, preferably is, toluene.
186. The process of any one of embodiments 154 to 185, wherein (i) comprises providing the compound of formula (III) and/or of formula (III'), preferably of formula (III), the compound H₂N-X_{b}, and a compound M_{b}OR_{b} wherein R_{b} is alkyl.
187. The process of embodiment 186, wherein R_{b} is C1-C6 alkyl, preferably C1-C4 alkyl, more preferably methyl, ethyl, or tert-butyl, more preferably methyl or ethyl, more preferably methyl.
188. The process of embodiment 186 or 187, wherein the compound of formula (III) and/or of formula (III'), preferably of formula (III), is provided in a solvent for the compound of formula (III) and/or of formula (III'), preferably of formula (III), wherein the solvent for compound of formula (III) and/or of formula (III'), preferably of formula (III), preferably comprises one or more aprotic solvents, preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran.
189. The process of embodiment 188, wherein the solvent for the compound of formula (III) and/or of formula (III'), preferably of formula (III), comprises, preferably is, toluene.
190. The process of any of one embodiments 186 to 189, wherein the compound H₂N-X₂ is provided in a solvent for the compound H₂N-X₂, wherein the solvent for the compound H₂N-X₂ preferably comprises one or more aprotic solvents, preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran.
191. The process of embodiment 190, wherein the solvent for the compound H₂N-X₂ comprises, preferably is, toluene.
192. The process of any one of embodiments 186 to 191, wherein the compound M_{b}OR_{b} is provided in a solvent for the compound M_{b}OR_{b}, wherein the solvent for the compound M_{b}OR_{b} preferably comprises one or more aprotic solvents, preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran.
193. The process of embodiment 192, wherein the solvent for the compound M_{b}OR_{b} comprises, preferably is, toluene.
194. The process of any one of embodiments 186 to 193, wherein (i) comprises reacting the compound M_{b}OR_{b} with the compound H₂N-X₂, obtaining the M_{b} salt of the compound H₂N-X₂; and adding the M_{b} salt of H₂N-X₂ to the compound of formula (III) and/or of formula (III'), preferably of formula (III).
195. The process of any one of embodiments 186 to 194, wherein (i) comprises reacting the compound M_{b}OR_{b} with a mixture of the compound H₂N-X₂ and the compound of formula (III) and/or of formula (III'), preferably of formula (III).
196. The process of embodiment 194 or 195, wherein (i) further comprises separating the compound HOR_{b}, at least partially formed during reacting the compound M_{b}OR_{b} with the compound H₂N-X₂, from the M_{b} salt of the compound H₂N-X₁, preferably by distillation or co-distillation.
197. The process of embodiment 196, wherein the compound HOR_{b} forms an azeotropic mixture with the solvent for the compound of formula (III) and/or of formula (III'), preferably of formula (III), and wherein reacting the compound of formula (III) and/or of formula (III'), preferably of formula (III), with the M_{b} salt of the compound H₂N-X₂ according to (i) is carried out at a temperature allowing for distilling off the azeotropic mixture.
198. The process of embodiment 197, wherein reacting the compound of formula (III) and/or of formula (III'), preferably of formula (III), with the M_{b} salt of the compound H₂N-X₂ according to (i) comprises distilling off the azeotropic mixture.
199. The process of any one of embodiments 186 to 198, wherein the molar ratio of the compound of formula (III) and/or of formula (III'), preferably of formula (III), provided according to (i), relative to the compound H₂N-X₂ provided according to (i) is in the range of from 1.5:1 to 1:1, preferably from 1.2:1 to 1:1, more preferably from 1.1:1 to 1:1.
200. The process of any one of embodiments 186 to 199, wherein the molar ratio of the compound M_{b}OR_{b} provided according to (i) relative to the compound H₂N-X₂ provided according to (i) is in the range of from 2.5:1 to 2:1, preferably from 2.2:1 to 2:1, more preferably from 2.1:1 to 2:1.
201. The process of any one of embodiments 154 to 200, wherein (ii) comprises
   (ii.1) adding an organic solvent to the reaction mixture obtained from (i);
   (ii.2) adding an acid to the mixture obtained from (ii.a.1), obtaining the compound of formula (IV) dissolved in the organic solvent according to (ii.1)
   (ii.3) separating the organic solvent from the compound of formula (IV);
   (ii.4) extracting the compound of formula (IV) with an extracting agent.
202. The process of embodiment 201, wherein the organic solvent according to (ii.a.1) comprises one or more of ethyl acetate, ispropyl acetate, butyl acetate, methyl tert-butyl ether, and dichloromethane, preferably ethyl acetate.
203. The process of embodiment 201 or 202, wherein the acid according to (ii.a.2) comprises ammonium chloride, preferably aqueous ammonium chloride, more preferably saturated aqueous ammonium chloride, wherein more preferably the acid is saturated aqueous ammonium chloride.
204. The process of any one of embodiments 201 to 203, wherein separating the organic solvent according to (ii.a.3) comprises evaporating the organic solvent.
205. The process of any one of embodiments 201 to 204, wherein the extracting agent according to (ii.a.4) comprises, preferably is, methyl tert-butyl ether.
206. The process of any one of embodiments 154 to 205, wherein the compound of formula (III) or the compound of formula (III') or the mixture of the compound of formula (II) and the compound of formula (III'), preferably the compound of formula (III), is obtainable or obtained by a process as defined in any one of embodiments 40 to 56.
207. The process of any one of embodiments 154 to 205, comprising preparing the compound of formula (III) or the compound of formula (III') or the mixture of the compound of formula (II) and the compound of formula (III'), preferably the compound of formula (III) by a process as defined in any one of embodiments 40 to 56.
208. The process of any one of embodiments 154 to 207, further comprising
   (iii) preparing a pharmaceutically acceptable salt of the compound of formula (IV).
209. The process of embodiment 208, wherein the salt is a malate, preferably a monomalate, a succinate, preferably a monosuccinate, or a phosphate, preferably a monophosphate.
210. A process for enriching a compound of formula (III) 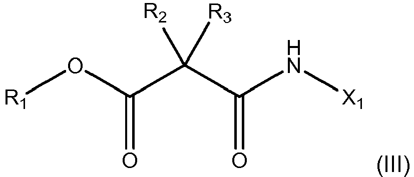 from a starting mixture comprising the compound of formula (III) and a compound of formula (S) 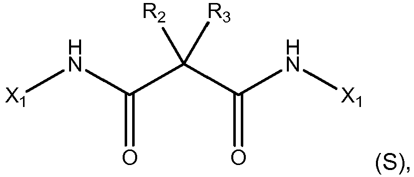 said process comprising contacting a mixture with an extracting agent comprising isopentane, obtaining a liquid phase comprising the extracting agent and the compound of formula (III), wherein in said liquid phase, the molar ratio of the compound of formula (III) relative to the compound (S) is higher than the respective molar ratio in the starting mixture; wherein
   R₁ is alkyl or aryl;
   R₂ and R₃, together with the C atom at which R₂ and R₃ are attached, form a cycle having 3 carbon atoms;
   X₁ is 4-haloaniline, preferably 4-fluoroaniline.
211. The process of embodiment 210, wherein R₁ is C1-C6 alkyl, preferably C1-C3 alkyl, more preferably C1 or C2 alkyl, wherein more preferably, the compound of formula (III) is the compound 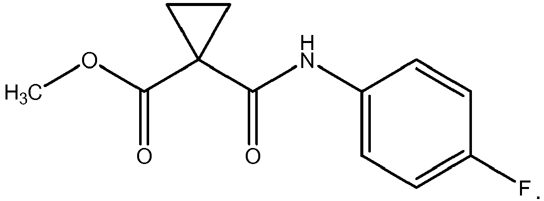
212. The process of embodiment 210 or 211, wherein the starting mixture comprises the compound of formula (III), the compound of formula (S), and a reaction solvent, wherein the reaction solvent preferably comprises one or more aprotic solvents, more preferably one or more of C5-C8 alkanes, benzene substituted with one or more C1-C2 alkyl residues, C1-C4 dialkylethers, 5- and 6-membered cyclic ethers, more preferably one or more of cyclohexane, toluene, xylene and tetrahydrofuran, wherein more preferably, the starting mixture comprises the compound of formula (III), the compound of formula (S), and toluene, said starting mixture optionally comprising water.
213. The process of embodiment 211 or 212, wherein the enriching comprises extracting the compound of formula (III) with isopentane as the extracting agent from the starting mixture, obtaining the liquid phase.
214. The process of embodiment 211 or 212, wherein the enriching comprises extracting the compound of formula (III) with dichloromethane as a first extracting agent from the starting mixture, separating the first extracting agent, preferably by evaporation, obtaining a mixture comprising the compound of formula (III), and extracting the compound of formula (III) from the mixture with isopentane as a second extracting agent, obtaining the liquid phase.
215. The process of any one of embodiments 210 to 214, further comprising separating the extracting agent comprising isopentane from the liquid phase, said separating the extracting agent preferably comprising, more preferably consisting of, evaporating the extracting agent comprising isopentane.
216. The process of embodiment 215, wherein from separating, preferably evaporating, the extracting agent comprising isopentane, the compound of formula (III) is obtained as a solid, preferably as a crystalline solid.
217. The process of any one of embodiments 210 to 216, wherein prior to the enriching, the compound of formula (III) has a degree of purity in the range of from 75:25 to 95:5, preferably from 85:15 to 95:1, more preferably from 92:8 to 95:5, more preferably from 93:7 to 95:5 in the starting mixture, and wherein after the enriching, the compound of formula (III) has a degree of purity in the range of from 98:2 to 100:0, preferably from 99:1 to 100:0, more preferably from 99.5:0.5 to 100:0, more preferably from 99.9:0.1 to 100:0 in the liquid phase, wherein the degree of purity is defined as the molar ratio of the compound of formula (III) relative to the compound of formula (S).
218. Use of an M_{b} salt of a compound H₂N-X₂ for preparing an asymmetric malonic acid diamide formula (IV) 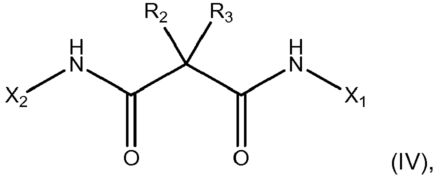 wherein
   R₂ and R₃ are alkyl, alkoxy, aryl or heteroaryl and R₂ and R₃ are the same or different, or R₂ and R₃, together with the C atom at which R₂ and R₃ are attached, form a cycle;
   X₁ is a substituted aromatic or heteroaromatic residue;
   X₂ is a substituted or unsubstituted alkyl, alkenyl, cycloalkyl, aromatic or heteroaromatic residue wherein X₁ is different from X₂;
   M_{b} is an alkali or alkaline earth metal.
219. The use of embodiments 218, wherein M_{b} is Mg, Na or K, preferably Na, wherein
220. The use of embodiment 218 or 219, wherein the M_{b} salt of the compound H₂N-X₂ is M_{b}HN-X₂.
221. The use of any one of embodiments 218 to 220, wherein R₂ and R₃ are C1-C6 alkyl, C1-C6 alkoxy, aryl having from 5 to 10 atoms constituting the aryl structure, or heteroaryl having from 5 to 10 atoms constituting the heteroaryl structure, and wherein R₂ and R₃ are the same or different.
222. The use of any one of embodiments 218 to 220, wherein R₂ and R₃ are C1-C6 alkyl, C1-C6 alkoxy, aryl having from 5 to 10 atoms constituting the aryl structure, or heteroaryl having from 5 to 10 atoms constituting the heteroaryl structure, and wherein R₂ and R₃ are the same or different and substituted with one or more of alkyl, preferably C1-C6 alkyl, alkoxy, preferably C1-C6 alkoxy, aryl, preferably having from 5 to 10 atoms constituting the aryl structure, and heteroaryl, preferably having from 5 to 10 atoms constituting the heteroaryl structure.
223. The use of any one of embodiments 218 to 222, wherein R₂ and R₃, together with the C atom at which R₂ and R₃ are attached, form a cycle having from 3 to 6, preferably from 3 to 5, preferably 3 or 4 atoms.
224. The use of any one of embodiments 218 to 223, wherein R₂ and R₃, together with the C atom at which R₂ and R₃ are attached, form a cycle having 3 carbon atoms.
225. The use of any one of embodiments 218 to 224, wherein X₁ is a substituted aromatic residue having from 5 to 10 atoms constituting the aromatic structure or a substituted heteroaromatic residue having from 5 to 10 atoms constituting the heteroaromatic structure.
226. The use of embodiment 225, wherein X₁ is an aromatic residue having from 5 to 10 atoms constituting the aromatic structure substituted with one or more of halogen, preferably F and Cl, alkyl, preferably C1-C6 alkyl, alkoxy, preferably C1-C6 alkoxy, aryloxy, and heteroaryloxy, or a heteroaromatic residue having from 5 to 10 atoms constituting the heteroaromatic structure substituted with one or more of halogen, preferably F and Cl, alkyl, preferably C1-C6 alkyl, alkoxy, preferably C1-C6 alkoxy, aryloxy, and heteroaryloxy.
227. The use of any one of embodiments 218 to 226, wherein X₁ is a monosubstituted aromatic residue having from 5 to 10 atoms constituting the aromatic structure or a monosubstituted heteroaromatic residue having from 5 to 10 atoms constituting the heteroaromatic structure, preferably a para- or ortho-monosubstituted aromatic residue having from 5 to 10 atoms constituting the aromatic structure or a para- or ortho-monosubstituted heteroaromatic residue having from 5 to 10 atoms constituting the heteroaromatic structure, wherein the substituent is preferably halogen, more preferably F or Cl, more preferably F.
228. The use of any one of embodiments 218 to 227, wherein X₂ is a substituted aromatic or heteroaromatic residue having from 5 to 10 atoms constituting the aromatic or heteroaromatic structure, preferably having from 6 to 10 atoms constituting the aromatic or heteroaromatic structure.
229. The use of embodiment 228, wherein X₂ is a substituted, preferably a mono-, di- or tri-substituted, aromatic or heteroaromatic residue having from 6 to 10 atoms, preferably 6 atoms, constituting the aromatic or heteroaromatic structure.
230. The use of embodiment 229, wherein X₂ is mono-, di- or tri-substituted phenyl.
231. The use of any one of embodiments 228 to 230, wherein X₂ is an aromatic or heteroaromatic residue having from 5 to 10 atoms constituting the aromatic or heteroaromatic structure, preferably having from 6 to 10 atoms constituting the aromatic or heteroaromatic structure, substituted with one or more of halogen, alkyl, preferably C1-C6 alkyl, alkoxy, preferably C1-C6 alkoxy, alkylthioyl, preferably C1-C6 alkylthioyl, and optionally substituted aryloxy or heteroaryloxy preferably having from 5 to 10 atoms constituting the aromatic or heteroaromatic structure.
232. The use of any one of embodiments 228 to 231, wherein X₂ is an aromatic or heteroaromatic residue having from 5 to 10 atoms constituting the aromatic or heteroaromatic structure, preferably having from 6 to 10 atoms constituting the aromatic or heteroaromatic structure, mono-substituted with substituted aryloxy or heteroaryloxy preferably having from 5 to 10 atoms, more preferably having from 6 to 10 atoms, constituting the aromatic or heteroaromatic structure.
233. The use of embodiment 232, wherein the substituted aryloxy or heteroaryloxy is substituted with one or more of halogen, preferably F and Cl, alkyl, preferably C1-C6 alkyl, alkoxy, preferably C1-C6 alkoxy, alkylthioyl, preferably C1-C6 alkylthioyl, and optionally substituted aryloxy or heteroaryloxy preferably having from 5 to 10 atoms constituting the aromatic or heteroaromatic structure.
234. The use of any one of embodiments 218 to 220, wherein the compound of formula (IV) is the compound 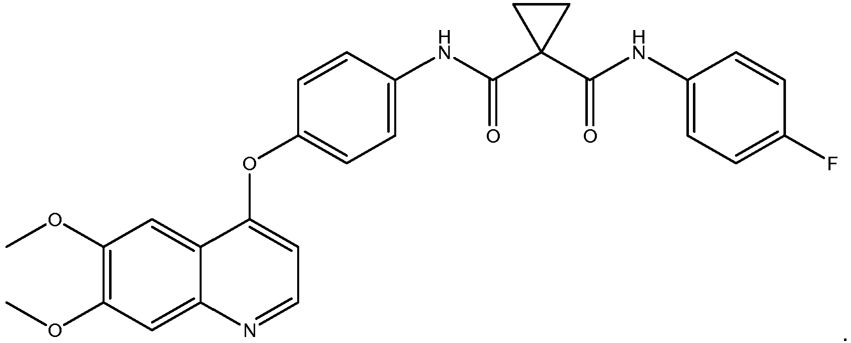
235. The use of any one of embodiments 218 to 220, wherein the compound of formula (IV) is the compound 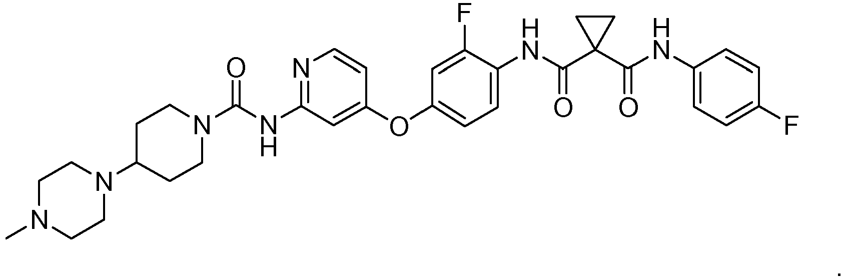
236. The use of any one of embodiments 218 to 220, wherein the compound of formula (IV) is the compound 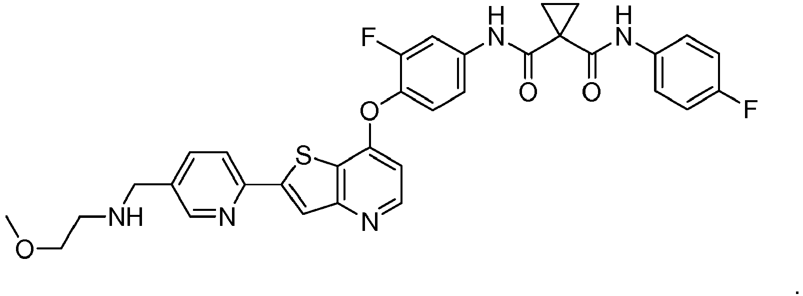
237. The use of any one of embodiments 218 to 220, wherein the compound of formula (IV) is the compound 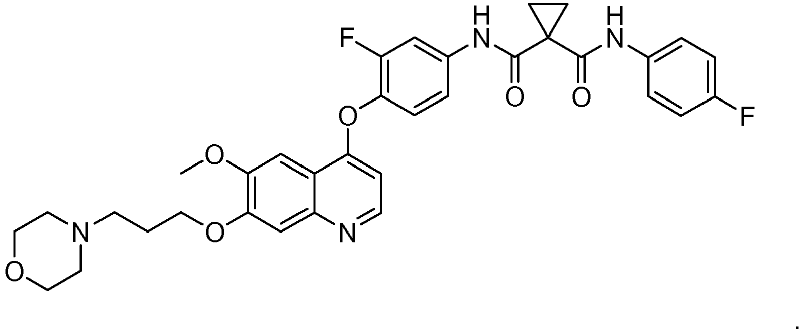
238. The use of any one of embodiments 218 to 220, wherein the compound of formula (IV) is the compound 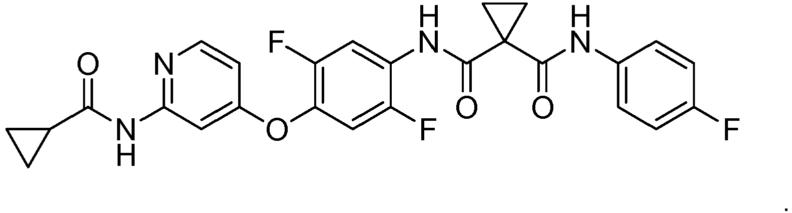
239. An Mₐ salt of formula (II) 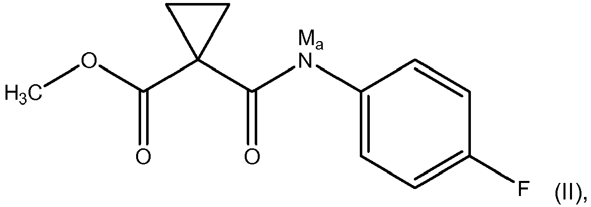 wherein Mₐ is an alkali metal, preferably sodium or potassium.
240. The Mₐ salt of embodiment 239, being 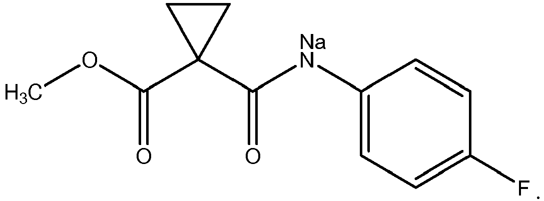
241. Use of the Mₐ salt of embodiment 239 or 240 for the preparation, preferably for a two-pot process preparation, of an asymmetric malonic acid diamide of formula (IV) 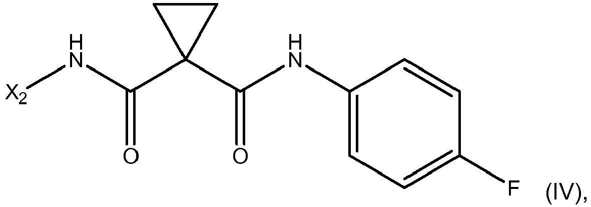 wherein X₂ is a substituted aromatic or heteroaromatic residue different from para-C₆H₄-F, X₂ preferably being 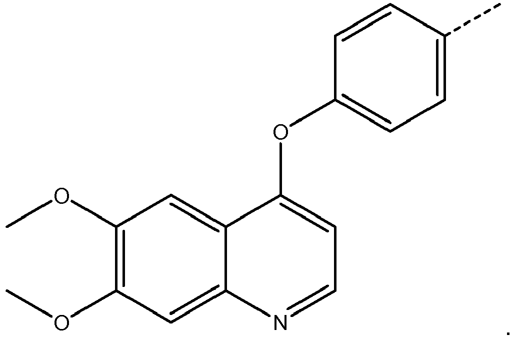
242. It is disclosed a composition comprising an Mₐ salt of formula (II) 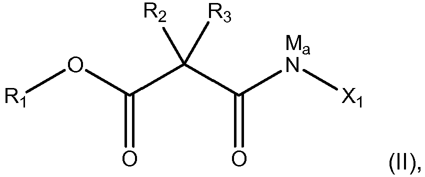 or an Mₐ salt of formula (II') 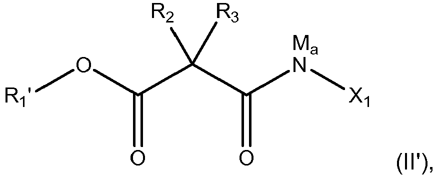 or a mixture of the Mₐ salt of formula (II) and the Mₐ salt of formula (II'), with a degree of purity in the range of from 75:25 to 95:5, for example from 85:15 to 95:, from 92:8 to 95.5, from 93:7 to 95:5, wherein the degree of purity is defined as the molar amount of the Mₐ salt of formula (II) or of the Mₐ salt of formula (II') or of the mixture of the Mₐ salt of formula (II) and the Mₐ salt of formula (II'), comprised in the composition, relative to the molar amount of an Mₐ salt of the symmetric malonic acid diamide of formula (S) 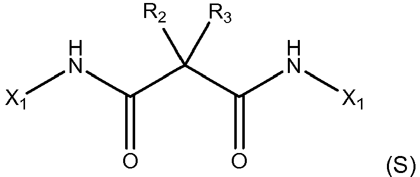 comprised in the composition, wherein
   R₁ and R₁' are alkyl or aryl and R₁ and R₁' are the same or different;
   R₂ and R₃ are alkyl, alkoxy, aryl or heteroaryl and R₂ and R₃ are the same or different, or R₂ and R₃, together with the C atom at which R₂ and R₃ are attached, form a cycle;
   X₁ is a substituted aromatic or heteroaromatic residue;
   Mₐ i is an alkali or alkaline earth metal.
243. It is disclosed that the composition of item 242, comprising an Mₐ salt of formula (II) 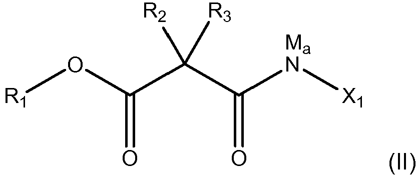 with a degree of purity in the range of from 75:25 to 95:5, for example from 85:15 to 95:1, from 92:8 to 95.5, from 93:7 to 95:5, wherein the degree of purity is defined as the molar amount of the Mₐ salt of formula (II) comprised in the composition, relative to the molar amount of an Mₐ salt of the symmetric malonic acid diamide of formula (S) 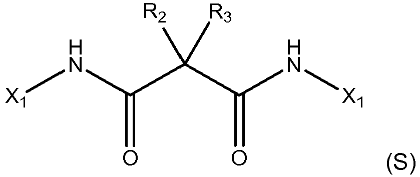 comprised in the composition.
244. It is disclosed the composition of item 242 or 243 comprising the Mₐ salt of formula (II) 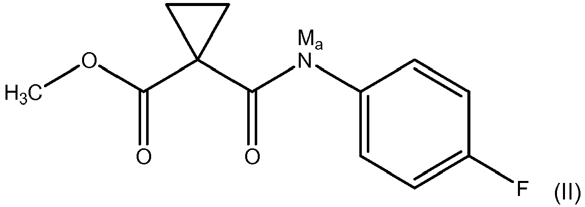 with a degree of purity in the range of from 75:25 to 95:5, for example from 85:15 to 95:1, from 92:8 to 95.5, from 93:7 to 95:5, wherein the degree of purity is defined as the molar amount of the Mₐ salt of formula (II) comprised in the composition relative to the molar amount of the Mₐ salt of the symmetric malonic acid diamide of formula (S) 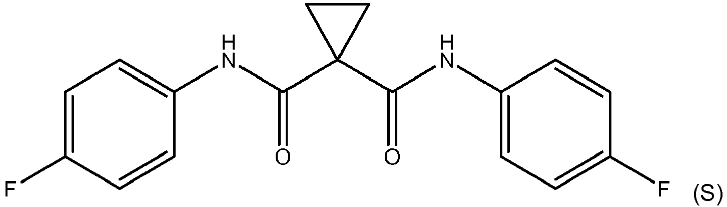 comprised in the composition.
245. It is disclosed the composition of any one of items 242 to 244, directly obtainable or directly obtained by a stage (i) of a process as defined in any one of embodiments 1 to 37.
246. It is disclosed a composition comprising a compound of formula (III) 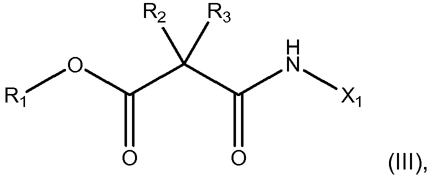 or a compound of formula (III') 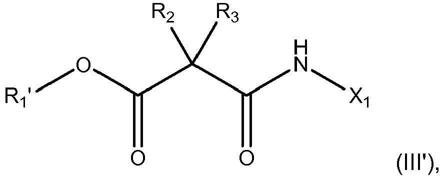 or a mixture of the compounds of formula (III) and formula (III'), wherein the compound of formula (III) or the compound of formula (III') or the mixture of the compounds of formula (III) and formula (III') is dissolved in a solvent system, said solvent system for example comprising or consisting of, one or more of dichloromethane, isopentane, cyclohexane, heptane, and toluene, for example one or more of dichloromethane and isopentane, for example isopentane, wherein
   R₁ and R₁' are alkyl or aryl and R₁ and R₁' are the same or different;
   R₂ and R₃ are alkyl, alkoxy, aryl or heteroaryl and R₂ and R₃ are the same or different, or R₂ and R₃, together with the C atom at which R₂ and R₃ are attached, form a cycle;
   X₁ is a substituted aromatic or heteroaromatic residue.
247. It is disclosed the composition of item 246, comprising a compound of formula (III) 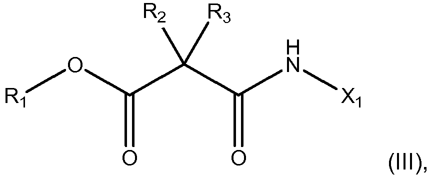 wherein the compound of formula (III) is dissolved in a solvent system, said solvent system for example comprising or consisting of, one or more of dichloromethane, isopentane, cyclohexane, heptane, and toluene, for example one or more of dichloromethane and isopentane, or for example isopentane, wherein
   R₁ is alkyl or aryl;
   R₂ and R₃ are alkyl, alkoxy, aryl or heteroaryl and R₂ and R₃ are the same or different, or R₂ and R₃, together with the C atom at which R₂ and R₃ are attached, form a cycle;
   X₁ is a substituted aromatic or heteroaromatic residue.
248. It is disclosed the composition of item 246 or 247, wherein the compound of formula (III) is 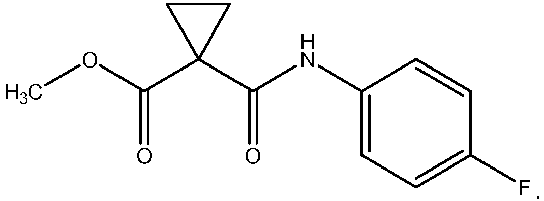
249. It is disclosed the composition of any one of items 246 to 248, further comprising a compound of formula (S) 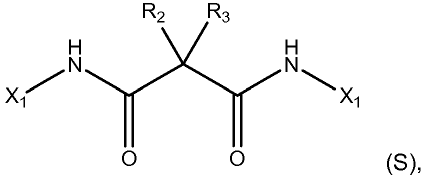 wherein the compound of formula (S) is not dissolved in the solvent system, the compound of formula (S) for example being 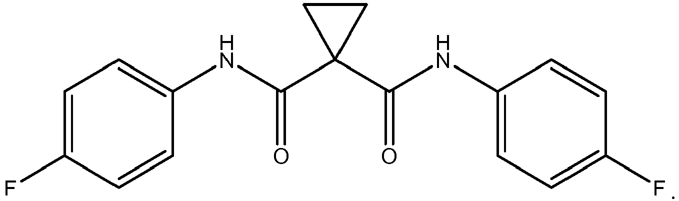
250. It is disclosed a composition comprising one or more aprotic solvents, an M_{b} salt of a compound H₂N-X₂, and a compound of formula (III) 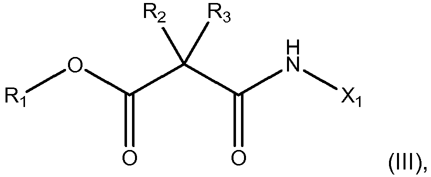 or a compound of formula (III') 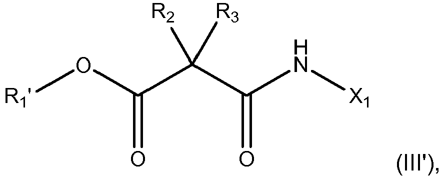 or a mixture of the compounds of formula (III) and formula (III'), wherein
   R₁ and R₁' are alkyl or aryl and R₁ and R₁' are the same or different;
   R₂ and R₃ are alkyl, alkoxy, aryl or heteroaryl and R₂ and R₃ are the same or different, or R₂ and R₃, together with the C atom at which R₂ and R₃ are attached, form a cycle;
   X₁ is a substituted aromatic or heteroaromatic residue,
   X₂ is a substituted or unsubstituted alkyl, alkenyl, cycloalkyl, aromatic or heteroaromatic residue wherein X₁ is different from X₂;
   M_{b} is an alkali or alkaline earth metal.
251. It is disclosed the composition of item 250, comprising one or more aprotic solvents, an M_{b} salt of a compound H₂N-X₂, and a compound of formula (III) 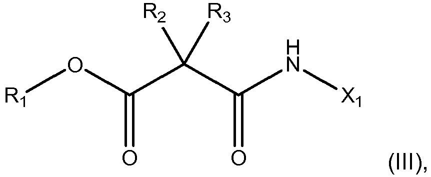 wherein
   R₁ is alkyl or aryl;
   R₂ and R₃ are alkyl, alkoxy, aryl or heteroaryl and R₂ and R₃ are the same or different, or R₂ and R₃, together with the C atom at which R₂ and R₃ are attached, form a cycle;
   X₁ is a substituted aromatic or heteroaromatic residue,
   X₂ is a substituted or unsubstituted alkyl, alkenyl, cycloalkyl, aromatic or heteroaromatic residue wherein X₁ is different from X₂;
   M_{b} is an alkali or alkaline earth metal.
252. It is disclosed the composition of item 250 or 251, wherein the compound of formula (III) is 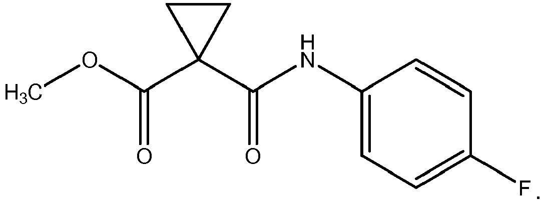
253. It is disclosed the composition of any one of items 250 to 252, wherein the one or more aprotic solvents are one or more of C5-C8 alkanes, benzene substituted with one or more C1-C2 alkyl residues, C1-C4 dialkylethers, 5- and 6-membered cyclic ethers, for example one or more of cyclohexane, toluene, xylene and tetrahydrofuran, for example toluene.
254. It is disclosed the composition of any one of items 250 to 253, wherein the M_{b} salt of the compound H₂N-X₂ is M_{b}HN-X₂, wherein M_{b} is for example sodium or potassium, for example sodium.
255. It is disclosed the composition of any one of items 250 to 254, wherein X₂ is a substituted aromatic or heteroaromatic residue having from 5 to 10 atoms constituting the aromatic or heteroaromatic structure, for example having from 6 to 10 atoms constituting the aromatic or heteroaromatic structure, for example a substituted, for example a mono-, di- or tri-substituted, aromatic or heteroaromatic structure having 6 atoms constituting the aromatic or heteroaromatic structure, for example mono-, di- or tri-substituted phenyl.
256. It is disclosed the composition of any one of items 250 to 255, wherein X₂ is 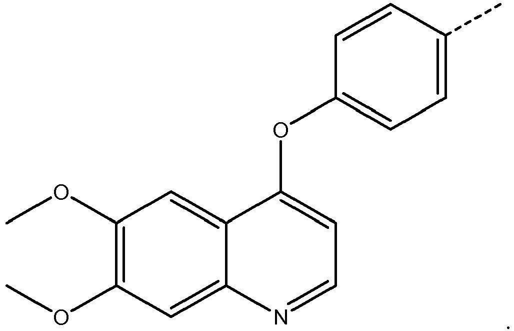
257. It is disclosed the use of a composition according to any one of items 242 to 256 for the preparation, for example for a two-pot process preparation, of a compound of formula (IV) 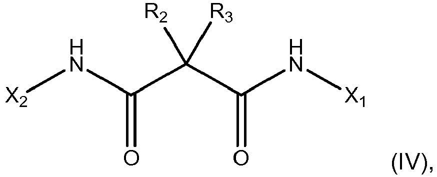 wherein
   R₂ and R₃ are alkyl, alkoxy, aryl or heteroaryl and R₂ and R₃ are the same or different, or R₂ and R₃, together with the C atom at which R₂ and R₃ are attached, form a cycle;
   X₁ is a substituted aromatic or heteroaromatic residue;
   X₂ is a substituted or unsubstituted alkyl, alkenyl, cycloalkyl, aromatic or heteroaromatic residue wherein X₁ is different from X₂.
258. It is disclosed the use of item 257, wherein the compound of formula (IV) is 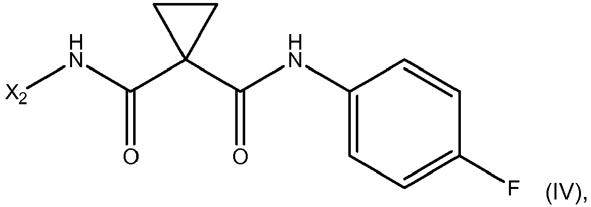 wherein X₂ is a substituted aromatic or heteroaromatic residue different from para-C₆H₄-F, X₂ for example being 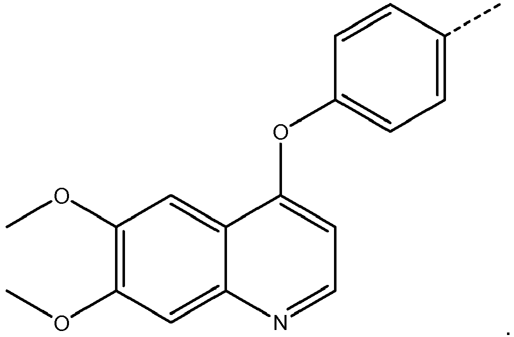
259. It is disclosed the use of item 258 , wherein X₂ is 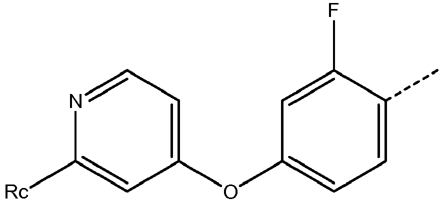 and wherein Rc is halogen, azide, nitro, phtalimido, hydroxycarbonyl, alkoxycarbonyl, cyano, carbonylamino, substituted carbamoyl-N-alkyl-amino, substituted carbamoyl-N-alkyl-amino, or N-acylamino.
260. It is disclosed the use of item 259 , wherein the compound of formula (IV) is 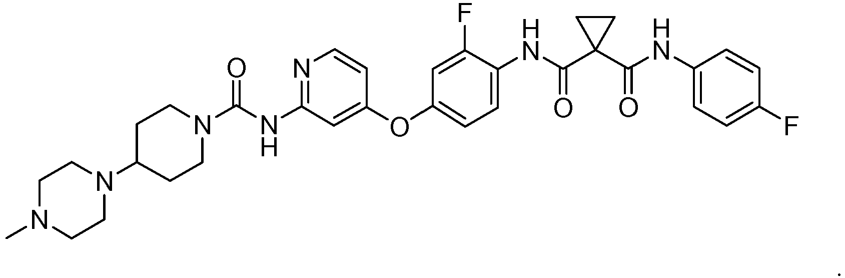
261. It is disclosed the use of item 258, wherein X₂ is 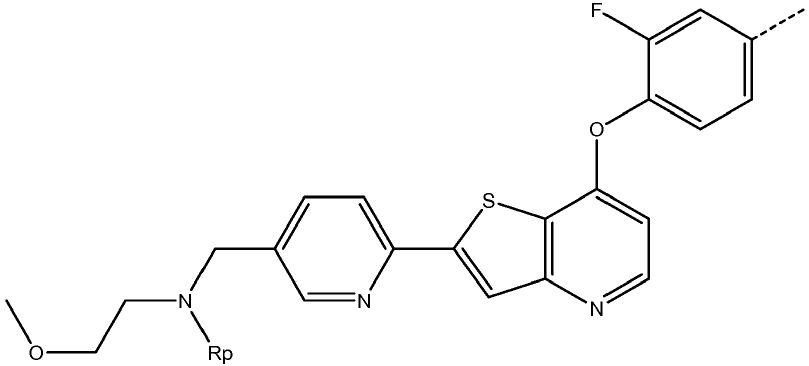 wherein Rp is hydroxyl, alkoxy, aryloxy or a nitrogen protecting group, for example allyl, Boc, Troc, or Trityl.
262. It is disclosed the use of item 261, wherein the compound of formula (IV) is 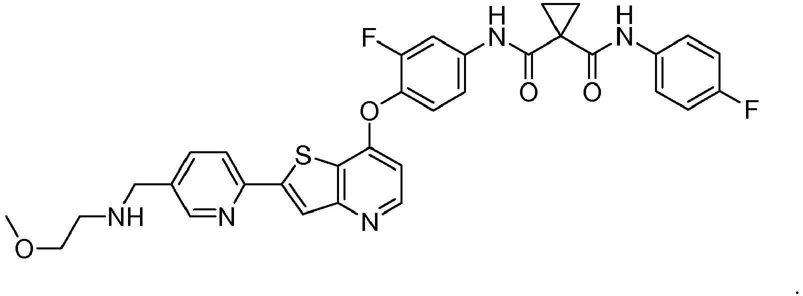
263. It is disclosed the use of item 258, wherein the compound of formula (IV) is 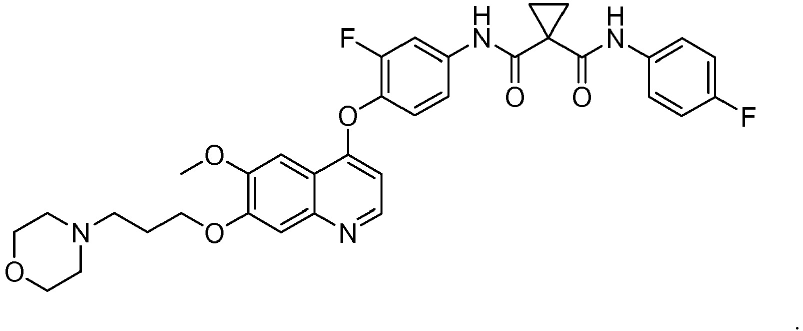
264. It is disclosed the use of item 258, wherein X₂ is tri-substituted with one or more of halogen, and substituted aryloxy or heteroaryloxy for example having from 5 to 10 atoms constituting the aromatic or heteroaromatic structure, for example having from 6 to 10 atoms constituting the aromatic or heteroaromatic structure.
265. It is disclosed the use of item 264, wherein the compound of formula (IV) is 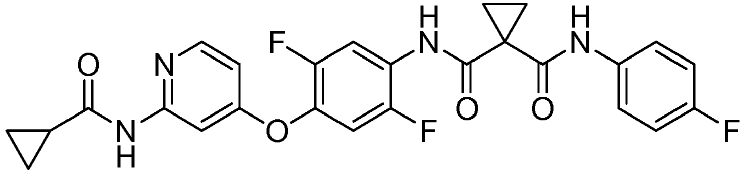
266. It is disclosed the use of an Mₐ salt of formula (II) 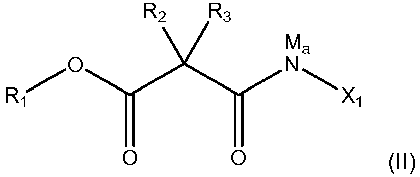 for the preparation of compound of formula (IV) 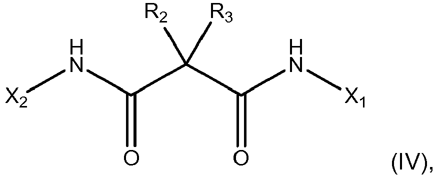 wherein
   R₁ is alkyl or aryl;
   R₂ and R₃ are alkyl, alkoxy, aryl or heteroaryl and R₂ and R₃ are the same or different, or R₂ and R₃, together with the C atom at which R₂ and R₃ are attached, form a cycle;
   X₁ is a substituted aromatic or heteroaromatic residue;
   X₂ is a substituted or unsubstituted alkyl, alkenyl, cycloalkyl, aromatic or heteroaromatic residue wherein X₁ is different from X₂;
   Mₐ is an alkali or alkaline earth metal.
267. It is disclosed the use of item 266, wherein the compound of formula (IV) is 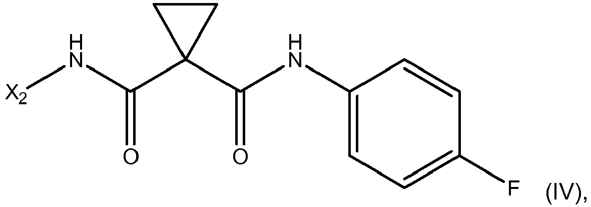 wherein X₂ is a substituted (hetero)aromatic residue different from para-C₆H₄-F, X₂ preferably being 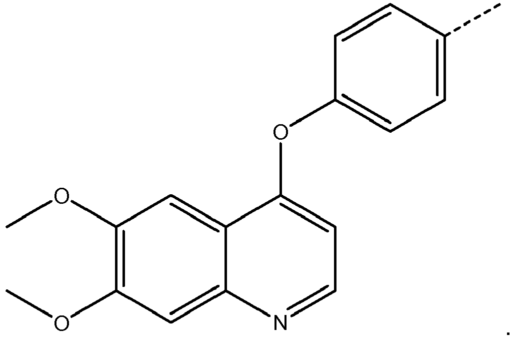
268. It is disclosed tthe use of item 267, wherein X₂ is di-substituted with one or more of halogen, and substituted aryloxy or heteroaryloxy for example having from 5 to 10 atoms constituting the aromatic or heteroaromatic structure, for example having from 6 to 10 atoms constituting the aromatic or heteroaromatic structure.
269. It is disclosed the use of item 268, wherein X₂ is 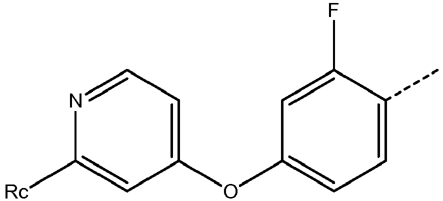 and wherein Rc is halogen, azide, nitro, phtalimido, hydroxycarbonyl, alkoxycarbonyl, cyano, carbonylamino, substituted carbamoyl-N-alkyl-amino, substituted carbamoyl-N-alkyl-amino, or N-acylamino.
270. It is disclosed the use of item 269, wherein the compound of formula (IV) is 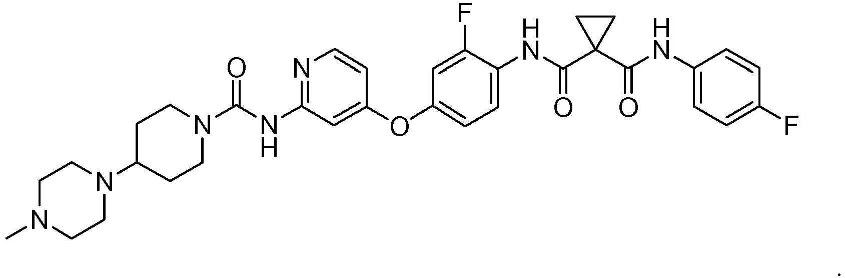
271. It is disclosed the use of item 270, wherein X₂ is 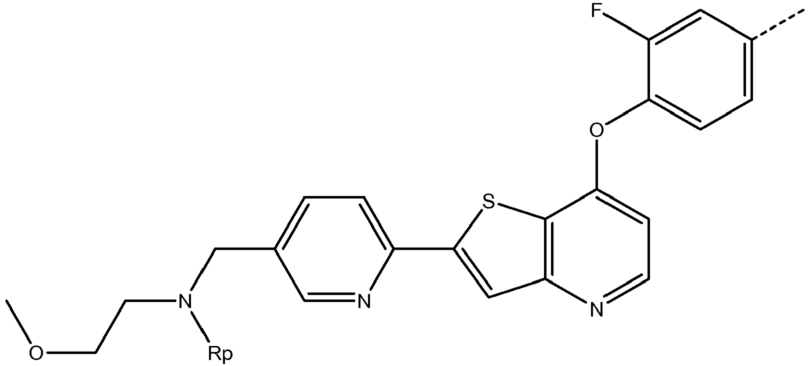 wherein Rp is hydroxyl, alkoxy, aryloxy or a nitrogen protecting group, preferably allyl, Boc, Troc, or Trityl.
272. It is disclosed the use of item 271, wherein the compound of formula (IV) is 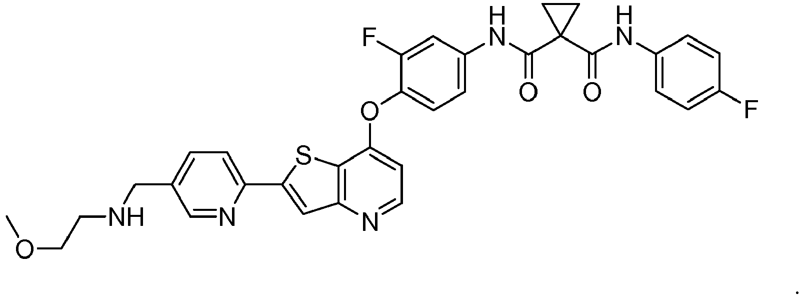
273. It is disclosed the use of item 266, wherein the compound of formula (IV) is 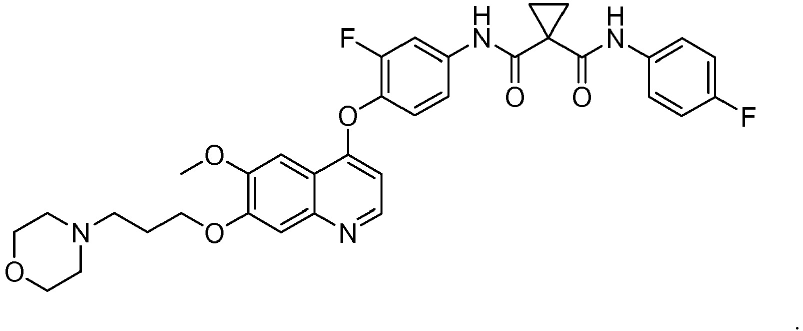
274. It is disclosed the use of item 266, wherein X₂ is tri-substituted with one or more of halogen, and substituted aryloxy or heteroaryloxy for example having from 5 to 10 atoms constituting the aromatic or heteroaromatic structure, for example having from 6 to 10 atoms constituting the aromatic or heteroaromatic structure.
275. It is disclosed the use of item 274, wherein the compound of formula (IV) is

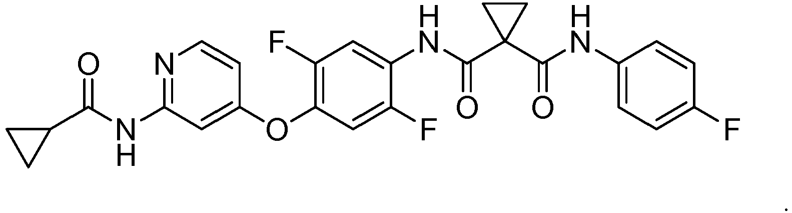

The present invention is further illustrated by the following examples.

### EXAMPLES

### Example 1: Preparation of methyl 1-((4-fluorophenyl)carbamoyl)cyclopropane carboxylate

4-Fluoraniline (1.11 g, 10 mmol) was dissolved in toluene (12 mL), sodium methoxide (0.54 g, 10 mmol) were added. Then the mixture was stirred 30 minutes under inert atmosphere wherein the bath temperature was raised from 80 to 100 °C and about 2 mL of azeotropic methanol/toluene mixture was evaporated. Next, dimethyl cyclopropane-1,1-dicarboxylate (1.58 g, 10 mmol) in toluene (5 mL) was added and azetropic distillation under stirring and inert gas flow was continued for another 30 minutes. Upon cooling to room temperature water (10 mL) and 1N hydrochloric acid (10 mL 1M) were added, the mixture was stirred for 10 minutes and extracted with dichloromethane (10 mL). The organic layer was dried over magnesium sulfate und the solvent was evaporated. The residue (2.07 g) was extracted with iso-pentane (12x10 mL) Evaporation of the solvent provided 1.32 g (56%) of the crystalline title compound (m.p.: 76 °C).

### Example 2: Preparation of N-(4-((6,7-dimethoxyquinolin-4-yl)oxy)phenyl)-N-(4-fluorophenyl)cyclo-propane-1,1-dicarboxamide (cabozantinib)

### Experiment 1:

4-((6,7-dimethoxyquinolin-4-yl)oxy)aniline (90 mg, 0.30 mmol) and sodium methoxide (36 mg, 0.67 mmol) in toluene (5 mL) were stirred at 100 °C under inert gas flow whereby the majority of the solvent evaporated. Next, toluene (5 mL) and a solution of methyl 1-((4-fluorophenyl)carbamoyl)cyclopropanecarboxylate (76 mg, 0.32 mmol) in toluene (5 mL) was added and azeotropic distillation, the bath temperature raised to 120 °C and stirring under inert gas flow was continued for another 50 minutes. Within this period additional solvent (10 mL) was added in order to compensate evaporation. Upon cooling to room temperature the residue was dissolved in ethyl acetate and washed with saturated aqueous ammonium chloride solution. The organic layer was dried over sodium sulfate und the solvent was evaporated. The residue (140 mg) was extracted with MTBE (3×1mL) providing 100 mg of the title compound as a yellow solid. Purity of the material determined by ¹H-NMR was found to be 30 %.

### Experiment 2:

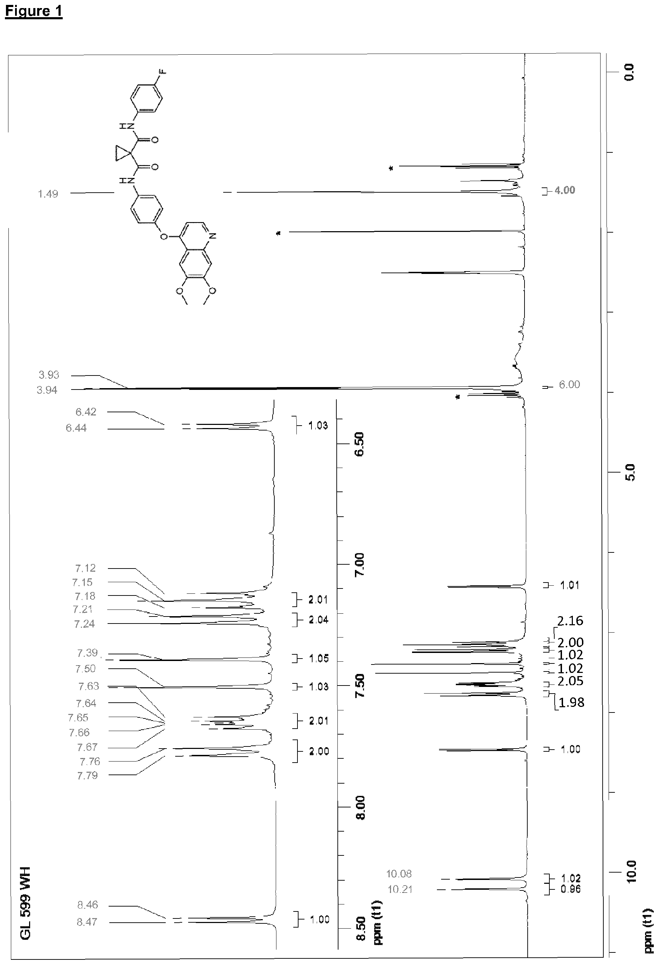

4-((6,7-dimethoxyquinolin-4-yl)oxy)aniline (240 mg, 0.81 mmol) and sodium methoxide (115 mg, 2.1 mmol) in toluene (15 mL) were stirred at 100 °C for 1 hour under inert gas flow whereby the majority of the solvent evaporated. Next, a solution of methyl 1-((4-fluorophenyl)carbamoyl)cyclopropanecarboxylate (230 mg, 0.97 mmol) in toluene (15 mL) was added and toluene/methanol azeotrope distilled off. The bath temperature was raised to 110 °C and stirring under inert gas flow was continued for another 4 hours. Within this period additional portions of toluene (3 x 20 mL) were added in order to compensate evaporation. Upon cooling to room temperature the residue was dissolved in ethyl acetate (30 mL) and washed sequentially with saturated aqueous ammonium chloride (30 mL) and bicarbonate (10 mL) solutions. The organic layer was dried over magnesium sulfate und the solvent was evaporated. The residue was extracted with MTBE (3 × 3 mL) providing after drying in vacuo 230 mg of pure title compound as a slightly brownish powder in 62 % yield. The respectively obtained material was subjected to ¹H-NMR analysis; the ¹H-NMR is shown in Fig. 1.

### BRIEF DESCRIPTION OF THE FIGURES

- Fig. 1: shows the ¹H-NMR spectrum of the material obtained from Example 2, Experiment 2.

### CITED LITERATURE

- WO 2005/030140
- WO 2010/083414
- F. D. Chattaway et al. , J.Chem. Soc. 97, 339 pp. 1910
- US 5,334,747
- Sechi et al., Molecules 2008, 13, pp. 2442-2461

## Claims

1. A process for preparing an asymmetric malonic acid diamide of formula (IV) 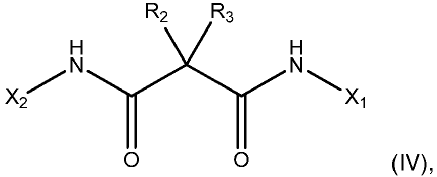 or a pharmaceutically acceptable salt thereof, the process comprising
(i) reacting a compound of formula (I) 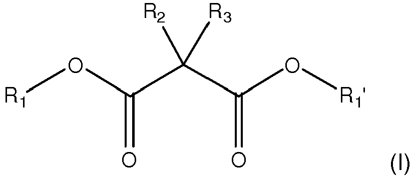 with an Mₐ salt of a compound H₂N-X₁, obtaining a reaction mixture comprising an Mₐ salt of formula (II) 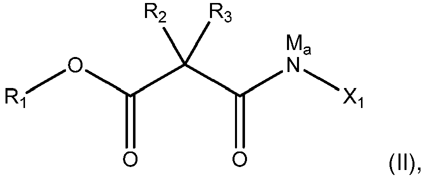 or an Mₐ salt of formula (II') 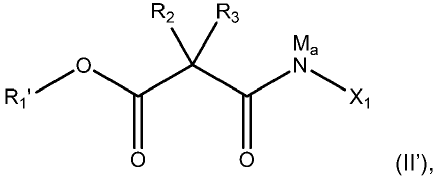 or a mixture of the Mₐ salt of formula (II) and the Mₐ salt of formula (II');
(ii) converting the Mₐ salt of formula (II) and/or of formula (II') to the compound of formula (IV) comprising forming an amide bond between the carbon atom of the ester moiety comprised in the compound of formula (II) and/or formula (II'), preferably of formula (II), and the nitrogen atom comprised in a compound H₂N-X₂;
wherein
R₁ and R₁' are alkyl or aryl, and R₁ and R₁' are the same or different;
R₂ and R₃ are alkyl, alkoxy, aryl or heteroaryl and R₂ and R₃ are the same or different, or R₂ and R₃, together with the C atom at which R₂ and R₃ are attached, form a cycle;
X₁ is a substituted aromatic or heteroaromatic residue;
X₂ is a substituted or unsubstituted alkyl, alkenyl, cycloalkyl, aromatic or heteroaromatic residue wherein X₁ is different from X₂;
Mₐ is an alkali or alkaline earth metal.

2. The process of claim 1, wherein R₁ and R₁' are the same, the process comprising
(i) reacting a compound of formula (I) 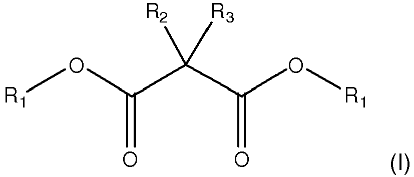 with an Mₐ salt of a compound H₂N-X₁ obtaining an Mₐ salt of formula (II) 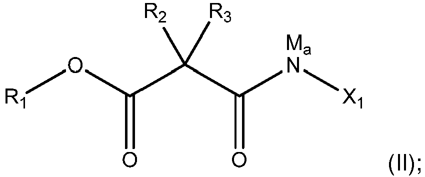
(ii) converting the Mₐ salt of formula (II) to the compound of formula (IV).

3. The process of claim 1 or 2, wherein the compound of formula (I) is

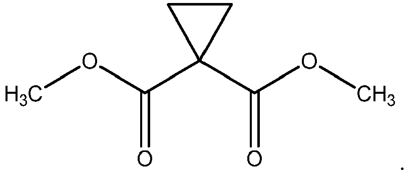

4. The process of any one of claims 1 to 3, wherein Mₐ is Mg, Na or K.

5. The process of any one of claims 1 to 4, wherein the compound of formula (I) is 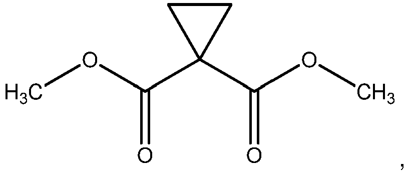 the compound H₂N-X₁ is 4-fluoroaniline and the Mₐ salt of formula (II) is

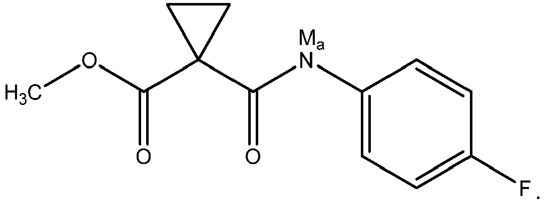

6. The process of claim 5, wherein the Mₐ salt of formula (II) is

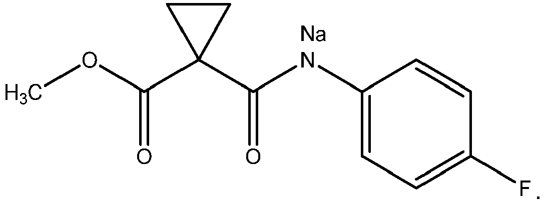

7. The process of any one of claims 1 to 6, wherein converting the Mₐ salt of formula (II) and/or of formula (II'), preferably of formula (II), to the compound of formula (IV) according to (ii) comprises
(ii.a.1) converting the Mₐ salt of formula (II) to a compound of formula (III) 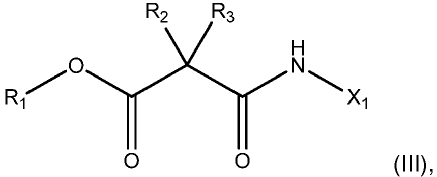 or the Mₐ salt of formula (II') to a compound of formula (III') 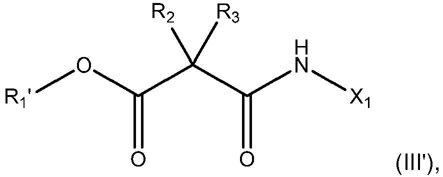 or the mixture of the Mₐ salt of formula (II) and the Mₐ salt of formula (II') to a mixture of the compounds of formula (III) and formula (III').

8. The process of claim 7, wherein the amide bond is formed by
(ii.a.2) reacting the compound of formula (III) and/or of formula (III'), preferably of formula (III), with an M_{b} salt of the compound H₂N-X₂;
wherein M_{b} is an alkali or alkaline earth metal.

9. The process of any one of claims 1 to 6, wherein the amide bond is formed by
(ii.b.1) reacting the compound of formula (II) and/or of formula (II') with an M_{b} salt of a compound H₂N-X₂;
wherein M_{b} is an alkali or alkaline earth metal.

10. A process for preparing an asymmetric malonic acid diamide of formula (IV) 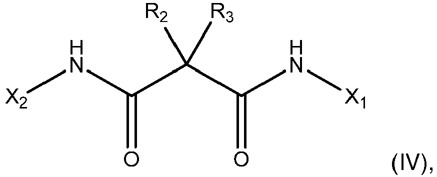 or a pharmaceutically acceptable salt thereof, the process comprising
(i) reacting a compound of formula (III) 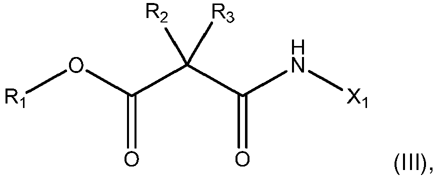 or a compound of formula (III') 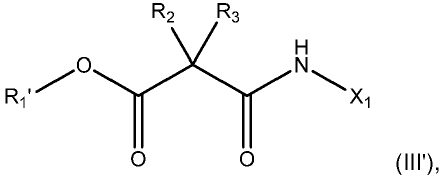 or a mixture of the compound of formula (III) and the compound of formula (III') with an M_{b} salt of a compound H₂N-X₂, obtaining a reaction mixture comprising an M_{b} salt of the compound of formula (IV);
(ii) converting the M_{b} salt of the compound of formula (IV) to the compound of formula (IV);
wherein
R₁ and R₁' are alkyl or aryl, and R₁ and R₁' are the same or different;
R₂ and R₃ are alkyl, alkoxy, aryl or heteroaryl and R₂ and R₃ are the same or different, or R₂ and R₃, together with the C atom at which R₂ and R₃ are attached, form a cycle;
X₁ is a substituted aromatic or heteroaromatic residue;
X₂ is a substituted or unsubstituted alkyl, alkenyl, cycloalkyl, aromatic or heteroaromatic residue wherein X₁ is different from X₂;
M_{b} is an alkali or alkaline earth metal.

11. Use of an M_{b} salt of a compound H₂N-X₂ for preparing an asymmetric malonic acid diamide formula (IV) 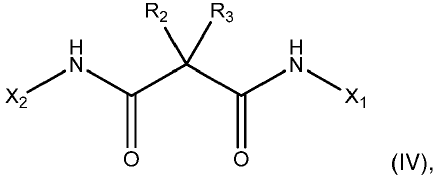 wherein
R₂ and R₃ are alkyl, alkoxy, aryl or heteroaryl and R₂ and R₃ are the same or different, or R₂ and R₃, together with the C atom at which R₂ and R₃ are attached, form a cycle;
X₁ is a substituted aromatic or heteroaromatic residue;
X₂ is a substituted or unsubstituted alkyl, alkenyl, cycloalkyl, aromatic or heteroaromatic residue wherein X₁ is different from X₂;
M_{b} is an alkali or alkaline earth metal.

12. The use of claim 11, wherein R₂ and R₃, together with the C atom at which R₂ and R₃ are attached, form a cycle having 3 carbon atoms.

13. The use of claim 11 or 12, wherein the compound of formula (IV) is 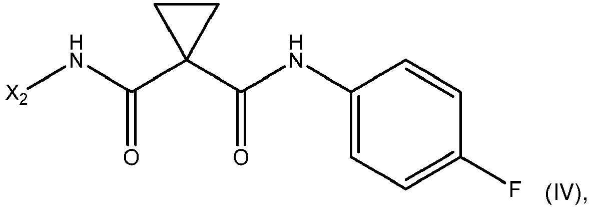 wherein X₂ is a substituted aromatic or heteroaromatic residue different from para-C₆H₄-F, X₂ preferably being

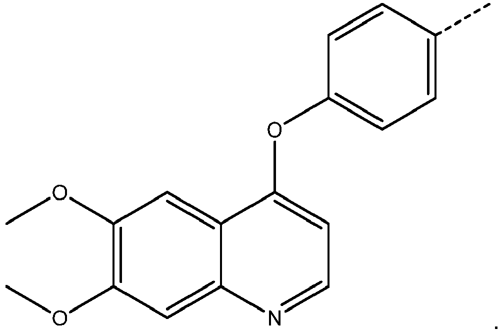

14. The use of any one of claims 11 to 13, wherein the compound of formula (IV) is the compound

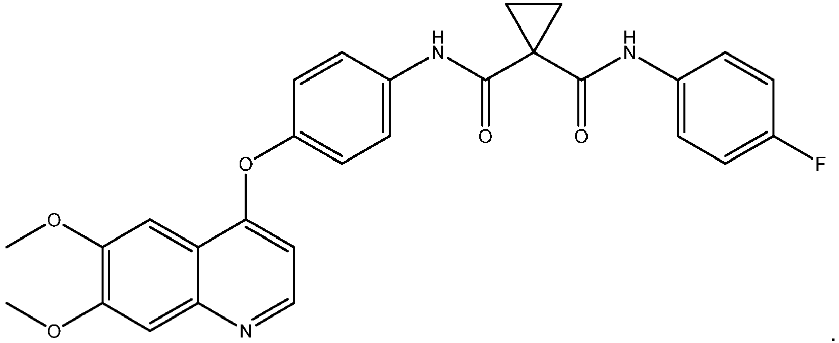

15. An Mₐ salt of formula (II) 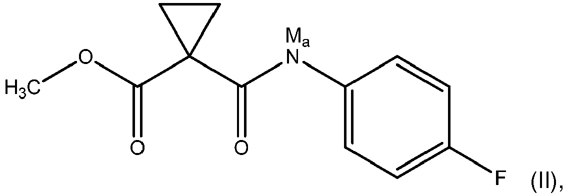 wherein Mₐ is an alkali metal.

## Patentansprüche

1. Verfahren zur Herstellung eines unsymmetrischen Malonsäurediamids der Formel (IV) 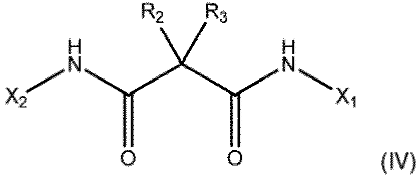 oder eines pharmazeutisch unbedenklichen Salzes davon, wobei das Verfahren Folgendes umfasst:
(i) Umsetzen einer Verbindung der Formel (I) 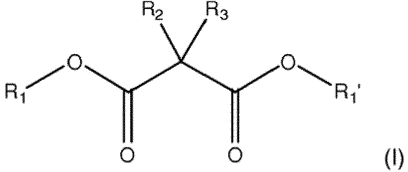 mit einem Mₐ-Salz einer Verbindung H₂N-X₁ unter Erhalt einer Reaktionsmischung, die ein Mₐ-Salz der Formel (II) 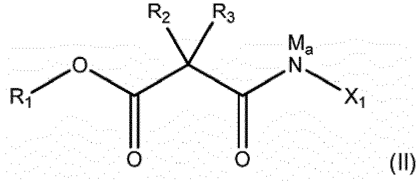 oder ein Mₐ-Salz der Formel (II') 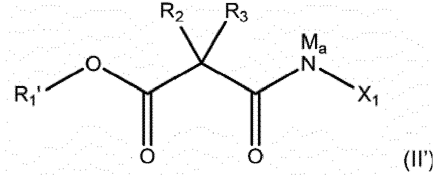 oder eine Mischung des Mₐ-Salzes der Formel (II) und des Mₐ-Salzes der Formel (II') umfasst;
(ii) Umwandeln des Mₐ-Salzes der Formel (II) und/oder der Formel (II') in die Verbindung der Formel (IV) mit Bildung einer Amidbindung zwischen dem Kohlenstoffatom der Estergruppierung in der Verbindung der Formel (II) und/oder Formel (II'), vorzugsweise der Formel (II), und dem Stickstoffatom in einer Verbindung H₂N-X₂;
wobei
R₁ und R₁' für Alkyl oder Aryl stehen und R₁ und R₁' gleich oder verschieden sind;
R₂ und R₃ für Alkyl, Alkoxy, Aryl oder Heteroaryl stehen und R₂ und R₃ gleich oder verschieden sind oder
R₂ und R₃ zusammen mit dem C-Atom, an das R₂ und R₃ gebunden sind, einen Ring bilden;
X₁ für einen substituierten aromatischen oder heteroaromatischen Rest steht;
X₂ für einen substituierten oder unsubstituierten Alkylrest, Alkenylrest, Cycloalkylrest, aromatischen Rest oder heteroaromatischen Rest steht, wobei X₁ von X₂ verschieden ist;
Mₐ für ein Alkali- oder Erdalkalimetall steht.

2. Verfahren nach Anspruch 1, wobei R₁ und R₁' gleich sind, wobei das Verfahren Folgendes umfasst:
(i) Umsetzen einer Verbindung der Formel (I) 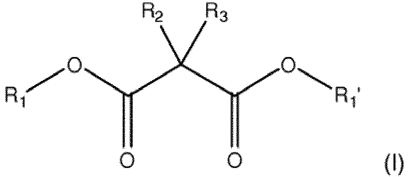 mit einem Mₐ-Salz einer Verbindung H₂N-X₁ unter Erhalt einer Reaktionsmischung, die ein Mₐ-Salz der Formel (II) 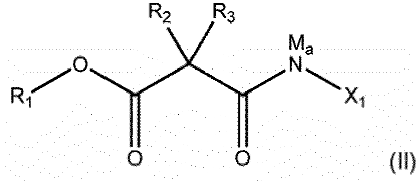 umfasst;
(ii) Umwandeln des Mₐ-Salzes der Formel (II) in die Verbindung der Formel (IV).

3. Verfahren nach Anspruch 1 oder 2, wobei es sich bei der Verbindung der Formel (I) um 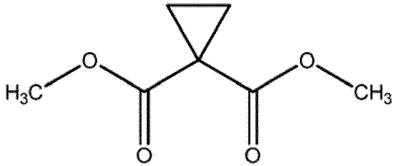 handelt.

4. Verfahren nach einem der Ansprüche 1 bis 3, wobei Mₐ für Mg, Na oder K steht.

5. Verfahren nach einem der Ansprüche 1 bis 4, wobei es sich bei der Verbindung der Formel (I) um 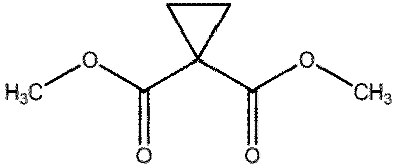 handelt, es sich bei der Verbindung H₂N-X₁ um 4-Fluoranilin handelt und es sich bei dem Mₐ-salz der Formel (II) um 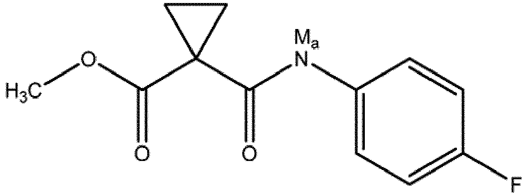 handelt.

6. Verfahren nach Anspruch 5, wobei es sich bei dem Mₐ-salz der Formel (II) um 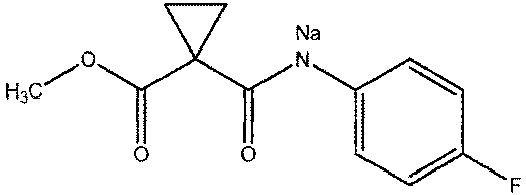 handelt.

7. Verfahren nach einem der Ansprüche 1 bis 6, bei dem die Umwandlung des Mₐ-Salzes der Formel (II) und/oder der Formel (II'), vorzugsweise der Formel (II), in die Verbindung der Formel (IV) gemäß (ii) Folgendes umfasst:
(ii.a.1) Umwandeln des Mₐ-Salzes der Formel (II) in eine Verbindung der Formel (III) 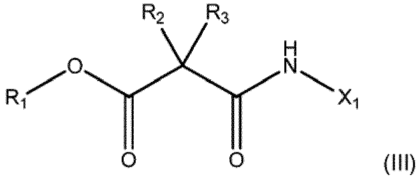 oder des Mₐ-Salzes der Formel (II') in eine Verbindung der Formel (III') 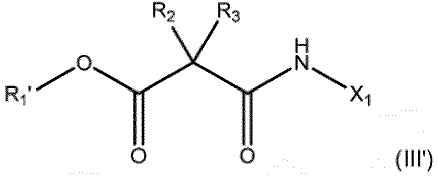 oder der Mischung des Mₐ-Salzes der Formel (II) und des Mₐ-Salzes der Formel (II') in eine Mischung der Verbindungen der Formel (III) und Formel (III').

8. Verfahren nach Anspruch 7, wobei die Amidbindung durch
(ii.a.2) Umsetzen der Verbindung der Formel (III) und/oder der Formel (III'), vorzugsweise der Formel (III), mit einem M_{b}-Salz der Verbindung H₂N-X₂
gebildet wird, wobei M_{b} für ein Alkali- oder Erdalkalimetall steht.

9. Verfahren nach einem der Ansprüche 1 bis 6, wobei die Amidbindung durch
(ii.b.1) Umsetzen der Verbindung der Formel (II) und/oder der Formel (II') mit einem M_{b}-Salz der Verbindung H₂N-X₂
gebildet wird, wobei M_{b} für ein Alkali- oder Erdalkalimetall steht.

10. Verfahren zur Herstellung eines unsymmetrischen Malonsäurediamids der Formel (IV) 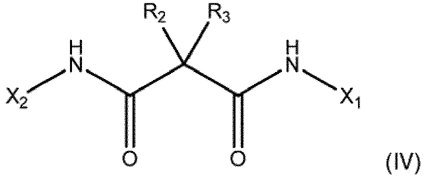 oder eines pharmazeutisch unbedenklichen Salzes davon, wobei das Verfahren Folgendes umfasst:
(i) Umsetzen einer Verbindung der Formel (III) 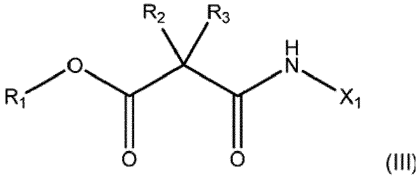 oder einer Verbindung der Formel (III') 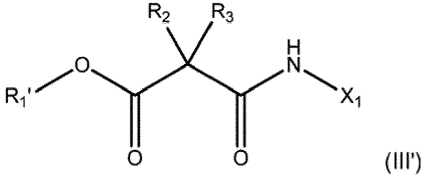 oder einer Mischung der Verbindung der Formel (III) der Verbindung der Formel (III') mit einem M_{b}-Salz einer Verbindung H₂N-X₂ unter Erhalt einer Reaktionsmischung, die ein M_{b}-salz der Verbindung der Formel (IV) umfasst;
(ii) Umwandeln des M_{b}-Salzes der Verbindung der Formel (IV) in die Verbindung der Formel (IV) ;
wobei
R₁ und R₁' für Alkyl oder Aryl stehen und R₁ und R₁' gleich oder verschieden sind;
R₂ und R₃ für Alkyl, Alkoxy, Aryl oder Heteroaryl stehen und R₂ und R₃ gleich oder verschieden sind oder
R₂ und R₃ zusammen mit dem C-Atom, an das R₂ und R₃ gebunden sind, einen Ring bilden;
X₁ für einen substituierten aromatischen oder heteroaromatischen Rest steht;
X₂ für einen substituierten oder unsubstituierten Alkylrest, Alkenylrest, Cycloalkylrest, aromatischen Rest oder heteroaromatischen Rest steht, wobei X₁ von X₂ verschieden ist;
M_{b} für ein Alkali- oder Erdalkalimetall steht.

11. Verwendung eines M_{b}-Salzes der Formel H₂N-X₂ zur Herstellung eines unsymmetrischen Malonsäurediamids der Formel (IV) 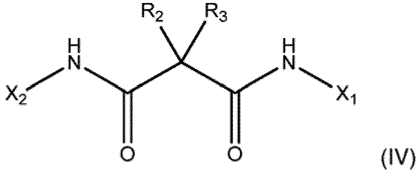 wobei
R₂ und R₃ für Alkyl, Alkoxy, Aryl oder Heteroaryl stehen und R₂ und R₃ gleich oder verschieden sind oder
R₂ und R₃ zusammen mit dem C-Atom, an das R₂ und R₃ gebunden sind, einen Ring bilden;
X₁ für einen substituierten aromatischen oder heteroaromatischen Rest steht;
X₂ für einen substituierten oder unsubstituierten Alkylrest, Alkenylrest, Cycloalkylrest, aromatischen Rest oder heteroaromatischen Rest steht, wobei X₁ von X₂ verschieden ist;
M_{b} für ein Alkali- oder Erdalkalimetall steht.

12. Verwendung nach Anspruch 11, wobei R₂ und R₃ zusammen mit dem C-Atom, an das R₂ und R₃ gebunden sind, einen Ring mit 3 Kohlenstoffatomen bilden.

13. Verwendung nach Anspruch 11 oder 12, wobei es sich bei der Verbindung der Formel (IV) um 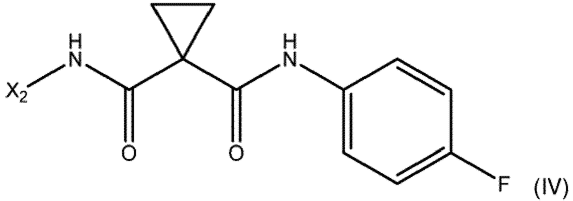 handelt, wobei X₂ für einen substituierten aromatischen oder heteroaromatischen Rest, der von para-C₆H₄-F verschieden ist, steht, wobei X₂ vorzugsweise für 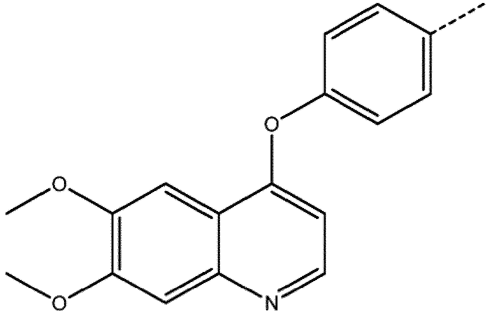 steht.

14. Verwendung nach einem der Ansprüche 11 bis 13, wobei es sich bei der Verbindung der Formel (IV) um die Verbindung 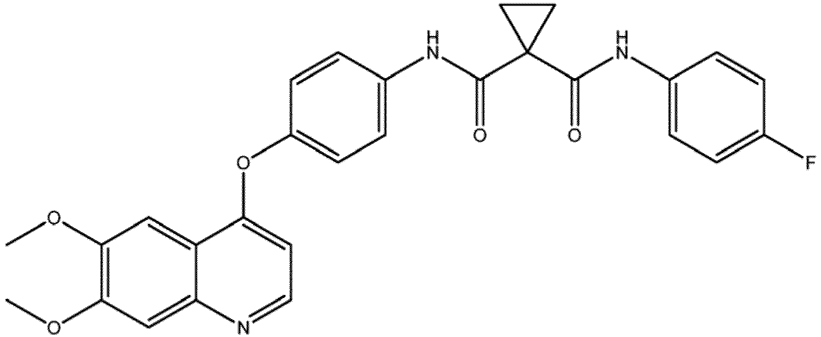 handelt.

15. Mₐ-Salz der Formel (II) 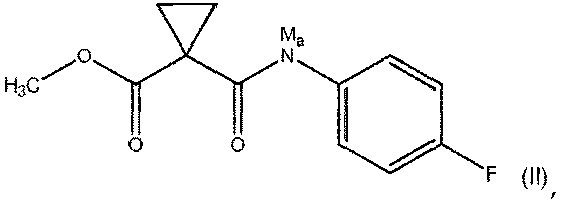 wobei Mₐ für ein Alkalimetall steht.

## Revendications

1. Procédé pour la préparation d'un diamide asymétrique de l'acide malonique de formule (IV) 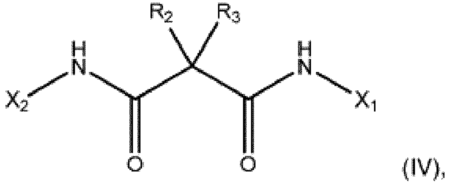 ou d'un sel pharmaceutiquement acceptable correspondant, le procédé comprenant
(i) la mise en réaction d'un composé de formule (I) 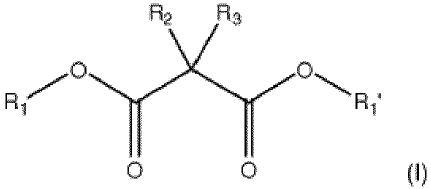 avec un sel de Mₐ d'un composé H₂N-X₁, obtenant un mélange réactionnel comprenant un sel de Mₐ de formule (II) 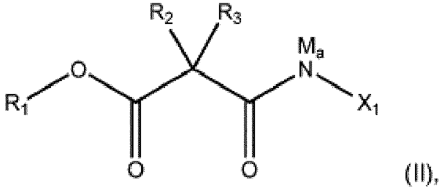 ou un sel de Mₐ de formule (II') 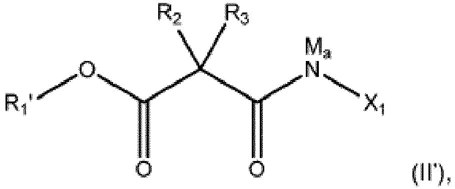 ou un mélange du sel de Mₐ de formule (II) et du sel de Mₐ de formule (II') ;
(ii) la conversion du sel de Mₐ de formule (II) et/ou de formule (II') en composé de formule (IV) comprenant la formation d'une liaison amide entre l'atome de carbone du fragment ester compris dans le composé de formule (II) et/ou de formule (II'), préférablement de formule (II), et l'atome d'azote compris dans un composé H₂N-X₂ ;
R₁ et R₁' étant alkyle ou aryle, et R₁ et R₁' étant identiques ou différents ;
R₂ et R₃ étant alkyle, alcoxyle, aryle ou hétéroaryle et R₂ et R₃ étant identiques ou différents, ou R₂ et R₃, conjointement avec l'atome de C auquel R₂ et R₃ sont fixés, formant un cycle ;
X₁ étant un radical substitué aromatique ou hétéroaromatique ;
X₂ étant un radical alkyle, alcényle, cycloalkyle, aromatique ou hétéroaromatique, substitué ou non substitué, X₁ étant différent de X₂ ;
Mₐ étant un métal alcalin ou alcalino-terreux.

2. Procédé selon la revendication 1, R₁ et R₁' étant identiques, le procédé comprenant
(i) la mise en réaction d'un composé de formule (I) 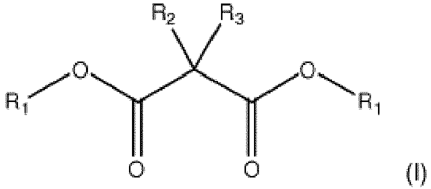 avec un sel de Mₐ d'un composé H₂N-X₁, obtenant un sel de Mₐ de formule (II) 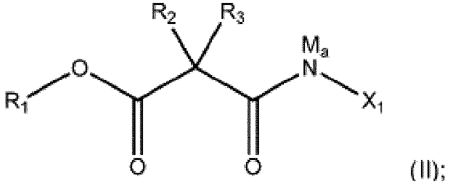
(ii) la conversion du sel de Mₐ de formule (II) en composé de formule (IV).

3. Procédé selon la revendication 1 ou 2, le composé de formule (I) étant

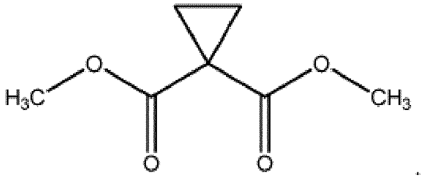

4. Procédé selon l'une quelconque des revendications 1 à 3, Mₐ étant Mg, Na ou K.

5. Procédé selon l'une quelconque des revendications 1 à 4, le composé de formule (I) étant 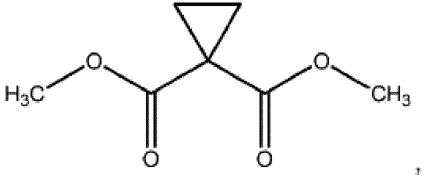 le composé H₂N-X₁ étant la 4-fluoroaniline et le sel de Mₐ de formule (II) étant

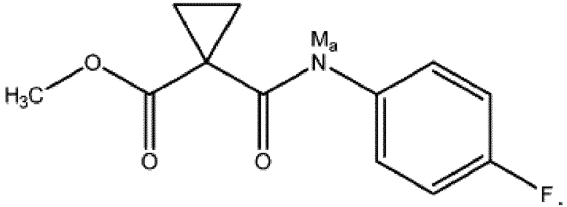

6. Procédé selon la revendication 5, le sel de Mₐ de formule (II) étant

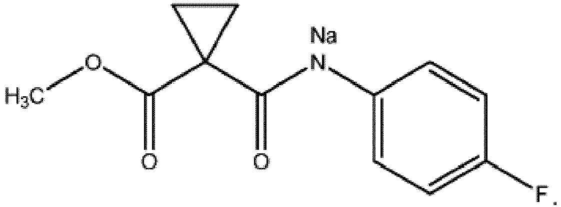

7. Procédé selon l'une quelconque des revendications 1 à 6, la conversion du sel de Mₐ de formule (II) et/ou de formule (II'), préférablement de formule (II), en composé de formule (IV) selon (ii) comprenant
(ii.a.1) la conversion du sel de Mₐ de formule (II) en un composé de formule (III) 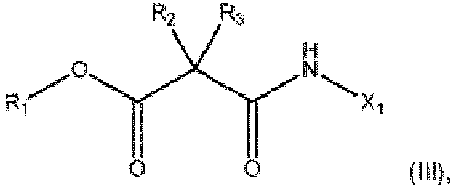 ou du sel de Mₐ de formule (II') en un composé de formule (III') 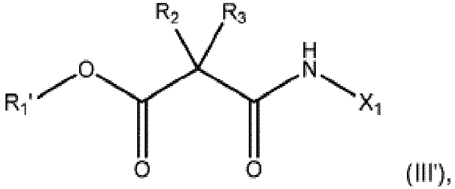 ou du mélange du sel de Mₐ de formule (II) et du sel de Mₐ de formule (II') en un mélange de composés de formule (III) et de formule (III').

8. Procédé selon la revendication 7, la liaison amide étant formée par
(ii.a.2) la mise en réaction du composé de formule (III) et/ou de formule (III'),
préférablement de formule (III), avec un sel de M_{b} du composé H₂N-X₂ ;
M_{b} étant un métal alcalin ou alcalino-terreux.

9. Procédé selon l'une quelconque des revendications 1 à 6, la liaison amide étant formée par
(ii.b.1) la mise en réaction du composé de formule (II) et/ou de formule (II'), avec un sel de M_{b} d'un composé H₂N-X₂ ;
M_{b} étant un métal alcalin ou alcalino-terreux.

10. Procédé pour la préparation d'un diamide asymétrique de l'acide malonique de formule (IV) 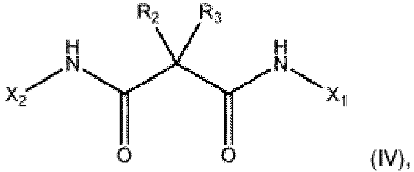 ou d'un sel pharmaceutiquement acceptable correspondant, le procédé comprenant
(i) la mise en réaction d'un composé de formule (III) 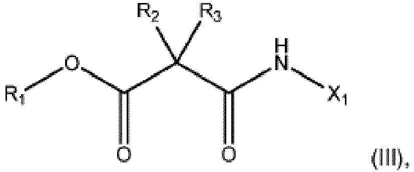 ou d'un composé de formule (III') 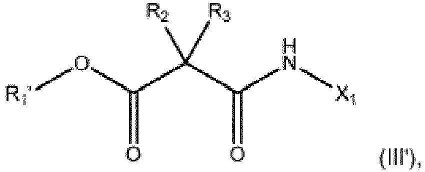 ou d'un mélange du composé de formule (III) et du composé de formule (III') avec un sel de M_{b} d'un composé H₂N-X₂, obtenant un mélange réactionnel comprenant un sel de M_{b} du composé de formule (IV) ;
(ii) la conversion du sel de M_{b} du composé de formule (IV) en composé de formule (IV) ;
R₁ et R₁' étant alkyle ou aryle, et R₁ et R₁' étant identiques ou différents ;
R₂ et R₃ étant alkyle, alcoxyle, aryle ou hétéroaryle et R₂ et R₃ étant identiques ou différents, ou R₂ et R₃, conjointement avec l'atome de C auquel R₂ et R₃ sont fixés, formant un cycle ; X₁ étant un radical substitué aromatique ou hétéroaromatique ;
X₂ étant un radical alkyle, alcényle, cycloalkyle, aromatique ou hétéroaromatique, substitué ou non substitué, X₁ étant différent de X₂ ;
M_{b} étant un métal alcalin ou alcalino-terreux.

11. Utilisation d'un sel de M_{b} d'un composé H₂N-X₂ pour la préparation d'un diamide asymétrique de l'acide malonique de formule (IV) 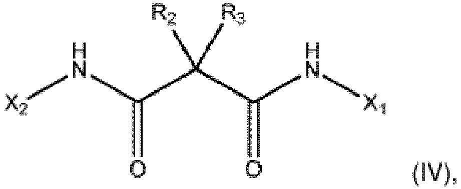
R₂ et R₃ étant alkyle, alcoxyle, aryle ou hétéroaryle et R₂ et R₃ étant identiques ou différents, ou R₂ et R₃, conjointement avec l'atome de C auquel R₂ et R₃ sont fixés, formant un cycle ;
X₁ étant un radical substitué aromatique ou hétéroaromatique ;
X₂ étant un radical alkyle, alcényle, cycloalkyle, aromatique ou hétéroaromatique, substitué ou non substitué, X₁ étant différent de X₂ ;
M_{b} étant un métal alcalin ou alcalino-terreux.

12. Utilisation selon la revendication 11, R₂ et R₃, conjointement avec l'atome de C auquel R₂ et R₃ sont fixés, formant un cycle possédant trois atomes de carbone.

13. Utilisation selon la revendication 11 ou 12, le composé de formule (IV) étant 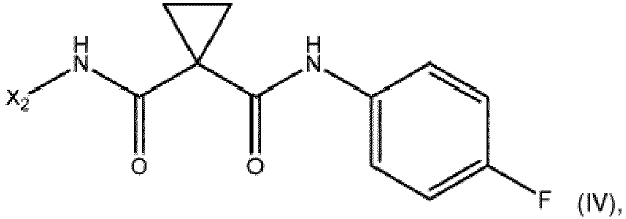 X₂ étant un radical aromatique ou hétéroaromatique, substitué, différent de para-C₆H₄-F, X₂ étant préférablement

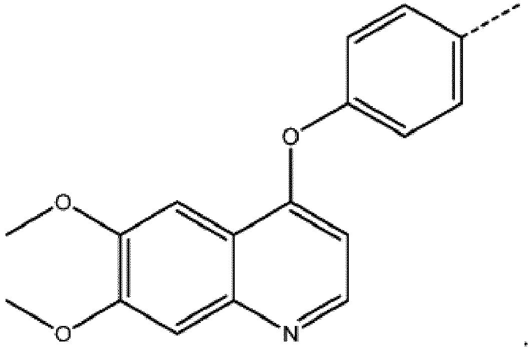

14. Utilisation selon l'une quelconque des revendications 11 à 13, le composé de formule (IV) étant le composé

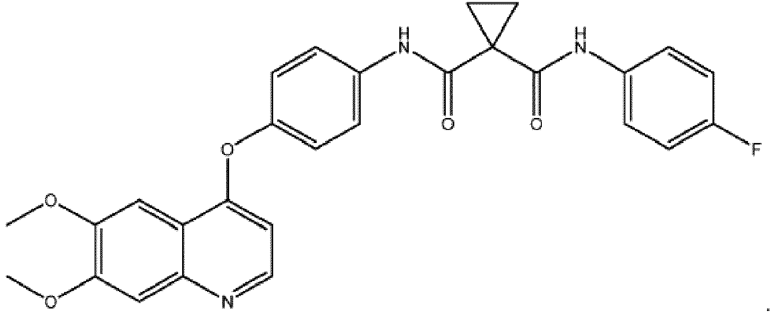

15. Sel de Mₐ de formule (II) 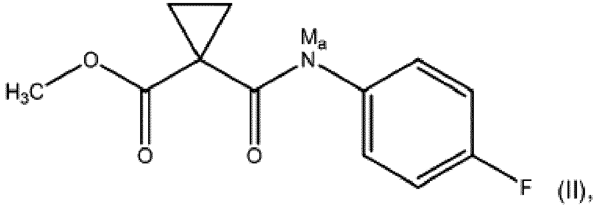 Mₐ étant un métal alcalin.
